# EUROPEAN PATENT SPECIFICATION

(11) **EP 0 628 559 B1**
(45) Date of publication and mention of the grant of the patent: **03.04.2002**
(21) Application number: 94304104.6
(22) Date of filing: 07.06.1994
(51) Int. Cl.: C07D 487/04, A61K 31/505

(54) **Pyrimidine compounds and their use as pharmaceuticals**
Pyrimidinverbindungen und ihre Verwendung als Pharmazeutika
Composés de pyrimidine et leur application comme agents pharmaceutiques

(30) Priority: 10.06.1993 EP 93304513
(43) Date of publication of application: 14.12.1994
(73) Proprietor: Beiersdorf-Lilly GmbH, 20253 Hamburg (DE)
(72) Inventor: Rühter, Gerd, D-21129 Hamburg (DE); Schotten, Theo, D-20253 Hamburg (DE); Stenzel, Wolfgang, D-21465 Reinbek (DE); Paal, Michael, D-22335 Hamburg (DE)
(74) Representative: Pritchard, Judith

(56) References cited:
- EP-A- 0 217 142
- EP-A- 0 323 841
- EP-A- 0 400 974
- EP-A- 0 412 848
- EP-A- 0 419 048
- EP-A- 0 461 040
- EP-A- 0 490 587
- EP-A- 0 500 136
- EP-A- 0 502 725
- EP-A- 0 505 111
- EP-A- 0 519 831
- EP-A- 0 533 058
- WO-A-92/18504
- FR-A- 2 448 542
- LIEBIGS ANNALEN DER CHEMIE, vol.699, 1966, WEINHEIM DE pages 127 - 132 T. PYL ET AL. 'Nucleophile Substitutionen...'

## Description

This invention relates to novel azolo-fused pyrimidine compounds and their use as pharmaceuticals.

Certain quinoline compounds of value in treating conditions such as hyper-tension and congestive heart failure are disclosed in EP-A-0 412 848. Triazolo-pyrimidine compounds for use as vasodilators are disclosed in FR-A-2 448 542.

The compounds of the invention are of the formula: 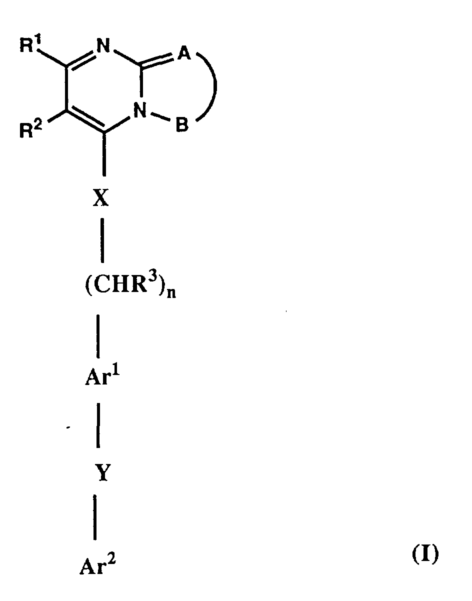 in which
R¹ is either R^{1a} selected from
   a) hydrogen,
   b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents, or by a single hydroxy, C₁₋₄-alkoxy, or C₁₋₄-alkylthio,
   c) phenyl or phenyl-C₁₋₃-alkyl, in which the phenyl group optionally may be substituted,
   d) C₂₋₈-alkenyl, C₃₋₈-cycloalkenyl, or C₂₋₈-alkynyl, which optionally may be substituted by phenyl, or
R^{1b} selected from
   a) C₁₋₆-alkylthio, C₃₋₆-cycloalkylthio, C₁₋₆-alkoxy, C₃₋₆-cycloalkoxy, mono-C₁₋₆-alkylamino, or mono-C₃₋₆-cycloalkylamino, in which an alkyl group optionally may be substituted by phenyl or by one or more fluoro substituents,
   b) phenylthio or phenoxy, in which the phenyl group optionally may be substituted,
   c) di-C₁₋₄-alkylamino, in which the alkyl groups may be the same or different or together form a polymethylene ring with three, four, five, or six carbon atoms, which optionally may be interrupted by an oxygen atom and optionally may be substituted by one or more fluoro substituents,
   d) mono-phenylamino or mono-C₁₋₄-alkyl-monophenylamino, in which the phenyl group optionally may be substituted and the alkyl groups optionally may be substituted by one or more fluoro substituents, or
   e) halo,
R² is
   a) hydrogen,
   b) C₁₋₈-alkyl, which optionally may be substituted by one or more fluoro substituents,
   c) optionally substituted phenyl,
R¹ and R² together form a polymethylene chain containing three, four or five carbon atoms, which optionally may be interrupted by an oxygen or sulfur atom,
R³ is hydrogen or C₁₋₄-alkyl, and n is 0 or 1,
X is O, S, or NR⁴, and
R⁴ is
   a) hydrogen,
   b) C₁₋₈-alkyl, C₃₋₈-cycloalkyl, C₄₋₈-cycloalkylalkyl, or C₄₋₈-alkylcycloalkyl, which optionally may be substituted by phenyl or by one or more fluoro substituents,
   c) optionally substituted phenyl,
   d) (CH₂)ₘCOOR²²,
   e) (CH₂)ₘCONR²³R²⁴,
   f) (CH₂)ₘCOOP¹, in which P¹ is a carboxy-protecting group,
   g) (CH₂)ₘCN,
   h) (CH₂)ₘ(5-tetrazolyl), and m is 1 or 2 in groups d), e), f), g) or h)
=A―B- together with the pyrimidine ring forms
   a) a pyrazolo[1,5-a]pyrimidine of formula (A), 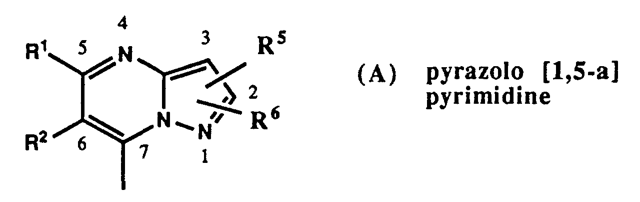
   b) a [1,2,4]triazolo[1,5-a]pyrimidine of formula (B), 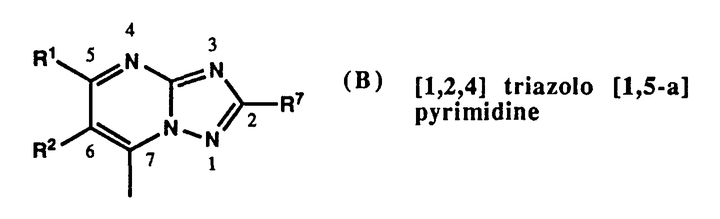
   c) an imidazo[1,5-a]pyrimidine of formula (C), 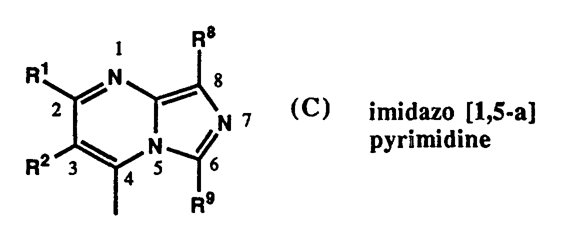
   or d) an imidazo[1,2-a]pyrimidine of formula (D), 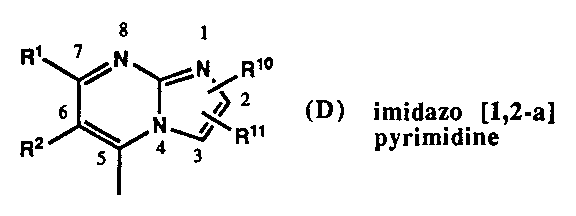
   in which
R⁵ is
   a) hydrogen,
   b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents,
   c) phenyl-C₁₋₃-alkyl,
   d) hydroxy, C₁₋₆-alkoxy, C₃₋₆-cycloalkoxy, C₁₋₃-phenylalkoxy, or phenoxy, in which the phenyl groups are optionally substituted and the alkyl and cycloalkyl groups optionally are substituted by one or more fluorine atoms,
   e) halo,
   f) mercapto,
   g) C₁₋₆-alkylthio, C₁₋₆-alkylsulfinyl, C₁₋₆-alkylsulfonyl, C₃₋₆-cycloalkylthio, C₃₋ ₆-cycloalkylsulfinyl, or C₃₋₆-cycloalkylsulfonyl, which optionally may be substituted by one or more fluorine atoms,
   h) phenylthio, phenylsulfinyl, phenylsulfonyl, phenyl-C₁₋₃-alkylthio, phenyl-C₁₋₃-alkylsulfinyl, or phenyl-C₁₋₃-sulfonyl, in which the phenyl groups optionally may be substituted,
   i) optionally substituted phenyl,
   j) cyano,
   k) COOR²²,
   l) CONR²³R²⁴,
   m) 5-tetrazolyl,
   n) COOP¹, in which P¹ is a carboxy-protecting group,
   o) SO₃H,
   p) SO₂NR²³R²⁴,
   q) nitro or nitroso, with the proviso that these groups are not connected to C-2 of the heterocycle,
   r) NR²³R²⁴,
   s) C₁₋₆-alkanoyl or 1-hydroxy-C₁₋₆-alkyl, which optionally may be substituted by one or more fluorine atoms,
   t) benzoyl or phenylhydroxymethyl, in which the phenyl group optionally may be substituted,
   u) NH(C₁₋₆-alkanoyl) or NH(C₁₋₆-alkylsulfonyl), in which the alkyl groups optionally may be substituted by one or more fluorine atoms,
   v) NH(benzoyl) or NH(benzenesulfonyl), in which the phenyl group optionally may be substituted,
R⁶ is
   a) hydrogen,
   b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents,
   c) halo,
   d) optionally substituted phenyl,
   e) C₁₋₆-alkylthio, C₁₋₆-alkylsulfinyl, C₁₋₆-alkylsulfonyl, C₃₋₆-cycloalkylthio, C₃₋₆-cycloalkylsulfinyl, or C₃₋₆-cycloalkylsulfonyl, which optionally may be substituted by one or more fluorine atoms,
R⁵ and R⁶ together may form a polymethylene chain containing three, four, or five carbon atoms,
R⁷ has the meaning as defined for R⁵ with the exception of nitro and nitroso,
R⁸ is
   a) hydrogen,
   b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents,
   c) halo,
   d) optionally substituted phenyl,
   e) nitro,
   f) cyano,
   g) 5-tetrazolyl,
   h) COOR²²,
   i) CONR²³R²⁴,
   j) COOP¹, in which P¹ is a carboxy-protecting group,
   k) NR²³R²⁴,
   l) NH(C₁₋₆-alkanoyl) or NH(C₁₋₆-alkylsulfonyl), in which the alkyl groups optionally may be substituted by one or more fluorine atoms,
   m) NH(benzoyl) or NH(benzenesulfonyl), in which the phenyl group optionally may be substituted,
R⁹ is
   a) hydrogen,
   b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents,
   c) an optionally substituted phenyl group,
   d) cyano,
   e) COOR²²,
   f) CONR²³R²⁴,
   g) 5-tetrazolyl,
   h) COOP¹, in which P¹ is a carboxy-protecting group,
   i) formyl,
   j) hydroxymethyl,
R¹⁰ has independently the same meaning as R⁵,
R¹¹ has independently the same meaning as R⁶,
Ar¹ is a group selected from
   a) 1,4-phenylene of formula (E), 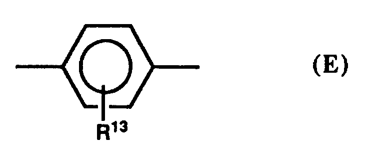
   b) 1,4-substituted pyridine of formula (F) or formula (G), 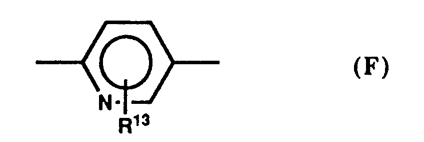 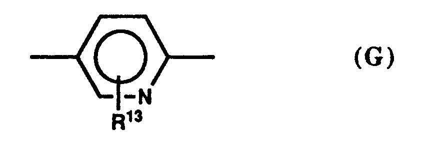
   or c) benzofuran, benzothiophene, or indole of formula (H), 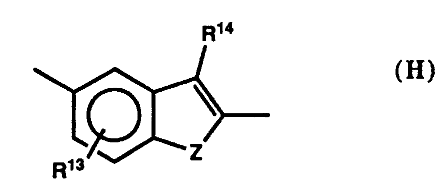
   in which the group Z is O, S, or NR¹², and R¹² is hydrogen or C₁₋₄-alkyl,
   and in each of the groups Ar¹ the substituent
R¹³ is
   a) hydrogen,
   b) halo,
   c) C₁₋₄-alkyl,
   d) C₁₋₄-alkoxy,
   e) trifluoromethyl,
   f) nitro,
R¹⁴ is
   a) hydrogen,
   b) C₁₋₆-alkyl, C₃₋₆-cycloalkyl, C₄₋₆-cycloalkylalkyl, or C₄₋₆-alkylcycloalkyl, which optionally may be substituted by one or more fluoro or chloro substituents,
   c) C₂₋₆-alkenyl or C₃₋₆-cycloalkenyl,
   d) halo,
   e) cyano,
   f) nitro,
   g) C₁₋₆-alkanoyl, in which the alkyl group optionally may be substituted by one or more fluorine atoms,
   h) C₁₋₆-alkoxy,
   i) COOR²²,
   j) CONR²³R²⁴,
Ar² is a group selected from
   a) phenyl of formula (I), 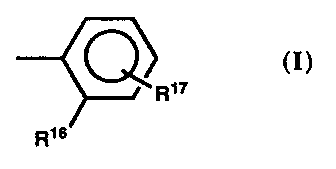
   b) pyridine of formula (J), 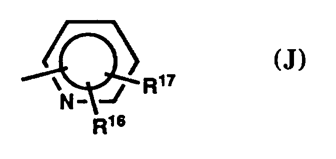
   c) 1-pyrrolyl of formula (K), 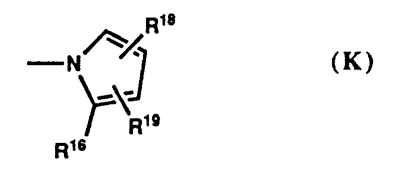
   or d) a five-membered heterocycle of formula (L), 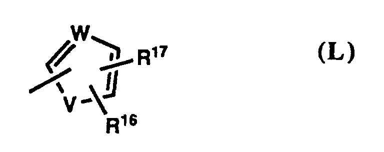
   in which the group V is O, S, SO, SO₂, or NR¹⁵, the group W is CH or N, and R¹⁵ is hydrogen or C₁₋₄-alkyl,
   with the proviso that in groups Ar² of formula (J) and (L) the substituent R¹⁶ and the group Y are in ortho positions, and in each of the groups Ar² the substituent R¹⁶ is hydrogen, an acidic group,
   COOP¹, in which P¹ is a carboxy-protecting group, or
   a group selected from
   a) cyano,
   b) a protected 5-tetrazolyl of formula (M), 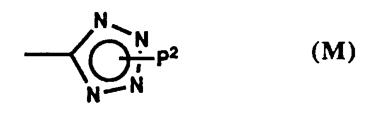 in which the group P² is a protecting group,
   c) COO(C₁₋₄-alkyl),
   d) nitro,
   e) amino,
   f) mercapto,
   g) SO₂Cl,
   h) SO₂(OC₁₋₄-alkyl),
   i) PO(OC₁₋₄-alkyl)₂,
R¹⁷ has independently the same meaning as R¹³,
R¹⁸ and R¹⁹ are independently selected from
   a) hydrogen,
   b) C₁₋₆-alkyl, C₃₋₆-cycloalkyl, C₄₋₆-cycloalkylalkyl, or C₄₋₆-alkylcycloalkyl, which optionally may be substituted by one or more fluoro or chloro substituents,
   c) C₂₋₆-alkenyl or C₃₋₆-cycloalkenyl,
   d) halo,
   e) nitro,
   f) cyano,
   g) C₁₋₄-alkylthio,
Y is a group selected from
   a) C-C single bond, CHR²⁰, CHR²⁰CH₂, OCHR²⁰, OCHR²⁰CH₂, SCHR²⁰, SCHR²⁰CH₂, NR²¹CHR²⁰, NR²¹CHR²⁰CH₂, CH₂CHR²⁰, CH₂CHR²⁰CH₂,
   b) O, S, SO₂, NR²¹, CO, CONH, NHCO, CH₂O, CH₂S, CH₂NR²¹, with the proviso that when Y is (b) Ar¹ is 1,4-phenylene of formula (E) and Ar² is phenyl of formula (I),
R²⁰ is hydrogen or
   a) COOH,
   b) COOP¹, in which P¹ is a carboxy-protecting group,
   c) COO(C₁₋₄-alkyl),
   d) 5-tetrazolyl,
   e) cyano,
   f) a protected 5-tetrazolyl of formula (M),
   with the proviso that one of the substituents R¹⁶ and R²⁰ is hydrogen and the other is a substituent other than hydrogen,
R²¹ and R²² are independently selected from hydrogen or C₁₋₆-alkyl, and
R²³ and R²⁴ are independently selected from hydrogen or C₁₋₄-alkyl, or together may form a polymethylene chain containing three, four or five carbon atoms, which optionally may be interrupted by an oxygen atom;
   or a salt thereof.

Compounds of the above formula I, in which R¹⁶ or R²⁰ is an acidic substituent or a group COOP¹, and pharmaceutical salts thereof, are useful as pharmaceuticals.

They are antagonists of angiotensin II receptors in mammals, and are indicated for use in the treatment and prophylaxis of, for example, hypertension, congestive heart failure, ocular hypertension, renal failure, and CNS disorders. Compounds of formula I in which R¹⁶ or R²⁰ is other than an acidic substituent or COOP¹, that is, the remaining compounds of formula I, are intermediates in the synthesis of the pharmaceutically active compounds.

The renin-angiotensin system (RAS) is important in regulating blood pressure in mammals (M.J. Antonaccio, J.J. Wright, in "Cardiovascular Pharmacology", 3rd edition, Raven Press, New York, 1990, p. 201). Within this system the octapeptide angiotensin II is a potent vasoconstrictor producing hypertension by acting via receptors in the cardiovascular tissue. This hormone is produced from human angiotensinogen, which first is cleaved by the enzyme renin to angiotensin I and further degraded by the angiotensin converting enzyme (ACE). Inhibitors for both enzymes have been developed and clinically tested during the last few years (Ann. Rep. Med. Chem. **1991**, 26, 63; J. Hypertension **1990**, 8, S 149). ACE inhibitors, e.g. captopril or enalapril, have become important drugs for treating hypertension and congestive heart failure. However, their major reported side effect is dry cough (Br. Medical J. **1987,** 294, 1521; J. Hypertension **1989,** 7, S 308). Therefore, it was envisaged that the development of drugs blocking receptors of angiotensin II, which are divided into several subtypes, mainly into AT₁ - and AT₂ - receptors (the former are supposed to be responsible for its cardiovascular effects), might bring benefits. The first reported angiotensin II antagonists were peptides, namely *saralasine*, but these showed low receptor subtype selectivity, lack of oral activity and partial agonist properties. However, today many orally active, subtype specific angiotensin II antagonists are known.

In the above formula I, the term "C₁₋₈-alkyl" means a straight or branched saturated alkyl group and includes methyl, ethyl, n-propyl, isopropyl, n-butyl, sec-butyl, isobutyl, tert-butyl, n-pentyl, isopentyl, n-hexyl, n-heptyl, n-octyl. The term "C₃₋₈-cycloalkyl" includes cyclopropyl, cyclobutyl, cyclopentyl, cyclohexyl.
The term "C₄₋₈-cycloalkylalkyl" means a straight or branched saturated alkyl group, which is substituted by a saturated cycloalkyl, and includes cyclopropylmethyl, cyclopentylmethyl, cyclohexylmethyl, 2-cyclohexylethyl. The term "C₄₋₈-alkylcycloalkyl" means a saturated cycloalkyl group, which is substituted by a straight or branched saturated alkyl, and includes 1-methylcyclopropyl, 2-methylcyclopropyl, 1-methylcyclopentyl, 1-methylcyclohexyl, 4,4-dimethylcyclohexyl.
The term "phenyl-C₁₋₃-alkyl" means a straight or branched saturated alkyl, which is substituted by phenyl, and includes benzyl, 2-phenylethyl, 1-phenylethyl.
The term "C₂₋₈-alkenyl" means a straight or branched alkenyl group with at least one C=C double bond and includes 1-propenyl, 2-propenyl, 3-propenyl, 2-butene-1-yl, 1-butene-1-yl, 2-methyl-1-propenyl, 2-methyl-3-propenyl.
The term "C₃₋₈-cycloalkenyl" includes 1-cyclopentenyl, 2-cyclopentenyl, 1-cyclohexenyl, 2-cyclohexenyl.
The term "C₂₋₈-alkynyl" includes 1-propynyl, 3-propynyl, 1-butynyl, 2-butyne-1-yl, 1-hexynyl.
The term "C₁₋₆-alkoxy" includes methoxy, ethoxy, propyloxy, isopropyloxy, n-butyloxy, isobutyloxy, sec-butyloxy, tert-butyloxy, n-pentyloxy, iso-pentyloxy. The term "C₃₋₆-cycloalkoxy" includes cyclopropyloxy, cyclobutyloxy, cyclopentyloxy, cyclohexyloxy.
The term "phenyl-C₁₋₃-alkoxy" means a C₁₋₃-alkoxy group, which is substituted by phenyl, and includes phenylmethoxy, 2-phenylethoxy, 1-phenylethoxy.
The term "C₁₋₆-alkanoyl" includes formyl, acetyl, propionyl, butyryl, 2-methylpropionyl, pentanoyl, 2-methylbutyryl, 3-methylbutyryl, pivaloyl.
The term "1-hydroxy-C₁₋₆-alkyl" means a C₁₋₆-alkyl group, which is substituted by hydroxy at C-1, and includes hydroxymethyl, 1-hydroxyethyl, 1-hydroxypropyl, 1-hydroxybutyl, 1-hydroxy-2-methylpropyl, 1-hydroxypentyl. The term "phenylhydroxymethyl" means a methyl group, which is substituted by hydroxy and by phenyl.
The term "C₁₋₆-alkylthio" includes methylthio, ethylthio, propylthio, isopropylthio, n-butylthio.
The term "C₃₋₆-cycloalkylthio" includes cyclopentylthio, cyclohexylthio.
The term "phenyl-C₁₋₃-alkylthio" means a C₁₋₃-alkylthio group, which is substituted by phenyl, and includes phenylmethylthio.
The term "C₁₋₆-alkylsulfinyl" includes methylsulfinyl, ethylsulfinyl, propylsulfinyl, isopropylsulfinyl, n-butylsulfinyl.
The term "C₁₋₆-alkylsulfonyl" includes methylsulfonyl, ethylsulfonyl, propylsulfonyl, isopropylsulfonyl, n-butylsulfonyl.
The term "mono-C₁₋₄-alkylamino" means an amino group, which is substituted by one straight or branched alkyl, and includes methylamino, ethylamino, propylamino, isopropylamino, n-butylamino, sec-butylamino, isobutylamino, tert-butylamino.
The term "di-C₁₋₄-alkylamino" means an amino group, which is substituted by two straight or branched alkyl groups, which are the same or different, and includes dimethylamino, N-methyl-N-ethylamino, diethylamino, dipropylamino, diisopropylamino, N-methyl-N-butylamino, pyrrolidino, piperidino.
The term "mono-C₁₋₄-alkyl-monophenylamino" includes N-phenyl-N-methylamino, N-phenyl-N-ethylamino, N-phenyl-N-butylamino.
The term "halo" means fluoro, chloro, bromo, or iodo.
The term "optionally substituted phenyl" means a phenyl group, which is unsubstituted or may be preferably substituted by one or more halo such as fluoro, chloro, bromo or iodo, C₁₋₄-alkyl, C₁₋₄-alkoxy, C₁₋₄-alkylthio, C₁₋₄-alkylsulfinyl, C ₁₋₄-alkylsulfonyl, cyano, nitro, trifluoromethyl, or hydroxy, and includes for example, phenyl, 2-methylphenyl, 3-methylphenyl, 4-methylphenyl, 2,4-dimethylphenyl, 4-ethylphenyl, 2-methoxyphenyl, 3-methoxyphenyl, 4-methoxyphenyl, 2,4-dimethoxyphenyl, 2-chlorophenyl, 3-chlorophenyl, 4-chlorophenyl, 2,4-dichlorophenyl, 3,5-dichlorophenyl, 4-fluorophenyl, pentafluorophenyl, 4-bromophenyl, 4-methylthiophenyl, 4-trifluoromethylphenyl, 4-cyanophenyl, 4-nitrophenyl, 4-hydroxyphenyl.
The term "optionally by one or more fluoro substituents substituted C₁₋₈-alkyl" includes the term "C₁₋₈-polyfluoroalkyl", which includes trifluoromethyl, pentafluoroethyl, heptafluoropropyl, nonafluoro-n-butyl.

The term "acidic group" means a group, which is capable forming an anion by donating a proton. Preferred acidic groups are those, which are known to those skilled in the art as bioisosteres of a carboxylic acid (A. Burger, Prog. Drug Res. **1991,** 37, 287), and which give compounds of formula I having pKₐ values between one and eleven, preferred between two and eight, more preferred between four and seven. Examples of acidic groups include 5-tetrazolyl, carboxylic acid, sulfonic acid, phosphonic acid, or trifluoromethaneamide.

The term "carboxy-protecting group" means a group, which is commonly used for the protection of a carboxylic acid (T.W. Greene, P.G.M. Wuts, "Protective Groups in Organic Synthesis", 2nd Ed., p. 227 ff.). In particular, it means a protecting group P¹, which is readily cleaved *in vivo*. Therefore, compounds of formula I containing those protected carboxylic acids COOP¹ may be useful as prodrugs. Substituents P¹, which are useful for forming of a prodrug, are known ("Bioreversible Carriers in Drug Design: Theory and Application", E.B. Roche, Ed., Pergamon Press, 1987, p. 14 ff.). Preferred groups COOP¹ are alkylesters, in which the alkyl group P¹ is substituted by an activating group, which preferably is connected to the C-1 of the alkyl group.

Preferred P¹ groups include
C₁₋₆-alkyl, which is substituted by halogen atoms, such as trifluoromethyl or 2,2,2-trichloroethyl;
C₁₋₆-alkyl, which is substituted by hydroxy groups, such as 2-hydroxyethyl or 2,3-dihydroxypropyl;
C₁₋₆-alkyl, which is substituted by an alkoxy or an alkoxyalkoxy group both containing between one and six carbon atoms, such as methoxymethyl, 2-methoxyethyl, or (2-methoxyethoxy)methyl;
phenacyl, in which the phenyl group optionally may be substituted, preferably the unsubstituted phenacyl group;
(C₁₋₆-alkyl)OCO(C₁₋₆-alkyl) or (C₃₋₆-cycloalkyl)OCO(C₁₋₆-alkyl) such as methoxycarbonylmethyl, ethoxycarbonylmethyl, isopropoxycarbonylmethyl, or cyclohexyloxycarbonylmethyl;
C₁₋₆-alkyl, which is substituted by C₁₋₆-alkylthio, C₁₋₆-alkylsulfonyl, or an optionally substituted phenylthio or phenylsulfonyl, such as (methylthio)methyl, (phenylthio)methyl, (methylsulfonyl)methyl, 2-(methylsulfonyl)ethyl, 2-(phenylsulfonyl)ethyl, or 2-(4-methylphenylsulfonyl)ethyl;
(C₁₋₆-alkyl)COO(C₁₋₆-alkyl) or (C₃₋₆-cycloalkyl)COO(C₁₋₆-alkyl) such as acetoxymethyl, propionyloxymethyl, butyryloxymethyl, pivaloyloxymethyl, cyclopentanoyloxymethyl, cyclohexanoyloxymethyl, 1-(acetoxy)ethyl, 1-(propionyloxy)ethyl, 1-(butyryloxy)ethyl, 1-(pivaloyloxy)ethyl, 1-(cyclopentanoyloxy)ethyl, 1-(cyclohexanoyloxy)ethyl, 2-(acetoxy)ethyl, 2-(propionyloxy)ethyl, 2-(butyryloxy)ethyl, 2-(pivaloyloxy)ethyl, 2-(cyclopentanoyloxy)ethyl, 2-(cyclohexanoyloxy)ethyl, 1-(acetoxy)propyl, 1-(propionyloxy)propyl, 1-(butyryloxy)propyl, 1-(pivaloyloxy)propyl, 1-(cyclopentanoyloxy)propyl, 1-(cyclohexanoyloxy)propyl, 1-(acetoxy)butyl, 1-(propionyloxy)butyl, 1-(butyryloxy)butyl, 1-(pivaloyloxy)butyl, 1-(cyclopentanoyloxy)butyl, or 1 -(cyclohexanoyloxy)butyl;
(C₁₋₆-alkyl)OCOO(C₁₋₆-alkyl) or (C₃₋₆-cycloalkyl)OCOO(C₁₋₆-alkyl) such as methoxycarbonyloxymethyl, ethoxycarbonyloxymethyl, propoxycarbonyloxymethyl, isopropoxycarbonyloxymethyl, butoxycarbonyloxymethyl, pentoxycarbonyloxymethyl, cyclopentoxycarbonyloxymethyl, cyclohexyloxycarbonyloxymethyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl, 1-(cyclohexyloxycarbonyloxy)ethyl, 2-(methoxycarbonyloxy)ethyl, 2-(ethoxycarbonyloxy)ethyl, 2-(propoxycarbonyloxy)ethyl, 2-(isopropoxycarbonyloxy)ethyl, 2-(butoxycarbonyloxy)ethyl, 2-(cyclohexyloxycarbonyloxy)ethyl, 1-(methoxycarbonyloxy)propyl, 1-(ethoxycarbonyloxy)propyl, 1-(propoxycarbonyloxy)propyl, 1-(isopropoxycarbonyloxy)propyl, 1-(butoxycarbonyloxy)propyl, 1-(cyclohexyloxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, or 1-(cyclohexyloxycarbonyloxy)butyl;
(5-(C₁₋₆-alkyl)-2-oxo-1,3-dioxolene-4-yl)methyl or (5-phenyl-2-oxo-1,3-dioxolene-4-yl)methyl, in which the phenyl optionally may be substituted, such as (5-phenyl-2-oxo-1,3-dioxolene-4-yl)methyl, (5-(4-methoxyphenyl)-2-oxo-1,3-dioxolene-4-yl)methyl, (5-methyl-2-oxo-1,3-dioxolene-4-yl)methyl, (5-ethyl-2-oxo-1,3-dioxolene-4-yl)methyl, (5-propyl-2-oxo-1,3-dioxolene-4-yl)methyl, or (5-butyl-2-oxo-1,3-dioxolene-4-yl)methyl;
3-phthalidyl, in which the aromatic ring optionally may be substituted.

Particularly preferred P¹ groups include
methoxymethyl, methoxycarbonylmethyl, ethoxycarbonylmethyl, acetoxymethyl, pivaloyloxymethyl, 1-(pivaloyloxy)ethyl, methoxycarbonyloxymethyl, ethoxycarbonyloxymethyl, 1-(methoxycarbonyloxy)ethyl, 1-(ethoxycarbonyloxy)ethyl, isopropoxycarbonyloxymethyl, 1-(isopropoxycarbonyloxy)ethyl, (5-methyl-2-oxo-1,3-dioxolene-4-yl)methyl, 3-phthalidyl, most particularly preferred pivaloyloxymethyl or ethoxycarbonyloxymethyl.

Protective P² groups for the protection of a tetrazole of formula (M) include triphenylmethyl, tert.-butyl, C₁₋₄-alkoxymethyl, methylthiomethyl, 4-nitrophenyl, tri(C₁₋₄-alkyl)stannyl, triphenylstannyl, 2-(trimethylsilyl)ethyl, benzenesulfonyl, 4-methylbenzenesulfonyl, 2-cyanoethyl, benzyl, 4-methoxybenzyl, 2,4,6-trimethylbenzyl, 4-nitrobenzyl. Preferred protective groups P² are triphenylmethyl, trimethylstannyl, or tri(n-butyl)stannyl.

Preferred R¹ substituents are
hydrogen, C₁₋₆-alkyl or C₃₋₆-cycloalkyl, which optionally may be substituted by one or more fluorine atoms, or C₁₋₅-alkylthio. Most preferred values of R¹ are methyl, ethyl, propyl, cyclopropyl, n-butyl, trifluoromethyl, pentafluoroethyl, 2,2,2-trifluoroethyl, methylthio, or ethylthio, and especially methyl or ethyl.

Preferred R² substituents are
hydrogen or C₁₋₄-alkyl, and mostly preferred are hydrogen, methyl and ethyl, especially hydrogen.

Preferred R³ substituents are
hydrogen and methyl, and hydrogen is particularly preferred.

Preferred X groups are
S or NR⁴ and most preferred are S, NH, NMe, NEt, N(n-Pr), N(n-Bu), NCH₂COOH, NCH₂COOMe, NCH₂COOEt, NCH₂CONH₂, NCH₂CONHMe, NCH₂CONMe₂, NCH₂CN and NCH₂(5-tetrazolyl). Particularly preferred are NH, NMe, NCH₂COOH, NCH₂COOMe, NCH₂COOEt and NCH₂CONH₂, and especially NH and NCH₂COOH.

Preferred substitution patterns of the R⁵ and R⁶ substituents in the five-membered ring of pyrazolo[1,5-a]pyrimidines of formula (A) are 2,3-dihydrogen, 2-methyl, 2-ethyl, 2,3-dimethyl, 2-ethyl-3-methyl, 3-methyl, 3-ethyl, 3-propyl, 3-butyl, 3-trifluoromethyl, 3-nitro, 3-cyano, 3-methoxycarbonyl, 3-ethoxycarbonyl, 3-chloro, 3-bromo, 3-methylthio, 3-ethylthio, 3-methylsulfonyl, 3-ethylsulfonyl, 3-trifluoromethylsulfonyl, 3-chloro-2-methyl, 3-bromo-2-methyl, 2-methyl-3-nitro, 2-amino, 2-cyano, 2-methylthio, 2-hydroxymethyl, 2-hydroxycarbonyl, 2-methoxycarbonyl, 2-ethoxycarbonyl, 2-trifluoromethanesulfonylamino, mostly preferred 2,3-dihydrogen, 2-methyl, 3-methyl, 3-ethyl, 3-propyl, 3-cyano, 3-nitro, 3-chloro, 2-methylthio, particularly preferred both substituents are hydrogen.

Preferred R⁷ substituents are
hydrogen, methyl, ethyl, methylthio, dimethylamino, and especially hydrogen.

Preferred R⁸ substituents are
hydrogen, C₁₋₄-alkyl, halo, cyano, nitro, COOMe, or COOEt, and especially hydrogen, methyl, ethyl, propyl, cyano, chloro, bromo, or nitro, and particularly hydrogen.

Preferred R⁹ substituents are
hydrogen, methyl, cyano, COOMe, COOEt, CONH₂, (pivaloyloxymethoxy)carbonyl, 5-tetrazolyl, formyl, or hydroxymethyl, and especially hydrogen, cyano, hydroxymethyl, formyl, COOEt, or COOH, particularly hydrogen or COOH.

Preferred substitution patterns of the R¹⁰ and R¹¹ substituents in the five-membered ring of imidazo[1,2-a]pyrimidines of formula (D) are 2,3-dihydrogen, 2-methyl, 2-ethyl, 2-methoxycarbonyl, 2-ethoxycarbonyl, 2-hydroxycarbonyl, 2-hydroxymethyl, 3-methoxycarbonyl, 3-ethoxycarbonyl, 3-hydroxycarbonyl, 3-hydroxymethyl, 3-formyl, 3-cyano, 3-nitro, 3-chloro, 3-bromo, 3-((pivaloyloxymethoxy)carbonyl), 3-(5-tetrazolyl), 3-aminocarbonyl, 3-SO₃H, 3-aminosulfonyl, 3-(trifluoromethanesulfonylamino), 3-methoxycarbonyl-2-methyl, 3-ethoxycarbonyl-2-methyl, 3-hydroxycarbonyl-2-methyl, 3-hydroxymethyl-2-methyl, 3-formyl-2-methyl, 2-methyl-3-nitro, 2-methyl-3-((pivaloyloxymethoxy)carbonyl), 2-methyl-3-(5-tetrazolyl), 3-aminocarbonyl-2-methyl, or 2-methyl-3-(trifluoromethanesulfonylamino), mostly preferred 2,3-dihydrogen, 2-methyl, 2-hydroxycarbonyl, 2-hydroxymethyl, 3-ethoxycarbonyl, 3-hydroxycarbonyl, 3-hydroxymethyl, 3-formyl, 3-SO₃H, 3-(trifluoromethanesulfonylamino), or 3-aminosulfonyl, particularly preferred 2,3-dihydrogen or 3-hydroxycarbonyl.

Preferred fused pyrimidines are
pyrazolo[1,5-a]pyrimidine of formula (A), imidazo[1,5-a]pyrimidine of formula (C), or imidazo[1,2-a]pyrimidine of formula (D), and mostly preferable pyrazolo[1,5-a]pyrimidine of formula (A).

Preferred R¹² and R¹³ substituents are
hydrogen or methyl, mostly preferable hydrogen

Preferred R¹⁴ substituents are
hydrogen, C₁₋₃-alkyl, cyano, halo, trifluoromethyl, or C₁₋₃-alkoxy, most preferably methyl, ethyl, cyano, trifluoromethyl, chloro, bromo, or methoxy, and especially chloro, bromo, particularly bromo.

Preferred Ar¹ groups are
1,4-phenylene of formula (E), 1,4-substituted pyridine of formula (F) or (G), or benzothiophene or benzofuran of formula (H), most preferably an unsubstituted 1,4-phenylene of formula (E), an unsubstituted pyridine of formula (F) or (G), 3-chlorobenzofuran or 3-bromobenzofuran of formula (H), and especially 1,4-phenylene or 3-bromobenzofuran of formula (E) or (H), respectively.

Preferred R¹⁵ substituents are
hydrogen or methyl, most preferably hydrogen.

Preferred acidic substituents R¹⁶ are
COOH, (pivaloyloxymethoxy)carbonyl, 5-tetrazolyl, NHSO₂CF₃, SO₃H, or PO(OH)₂, and especially 5-tetrazolyl.

Preferred R¹⁷ substituents are
hydrogen, methyl, fluoro, chloro, or bromo, and especially preferred hydrogen.

Preferred R¹⁸ and R¹⁹ substituents are
hydrogen, C₁₋₃-alkyl, halo, or cyano, most preferably hydrogen, methyl, fluoro, chloro, bromo, or trifluoromethyl, and hydrogen is particularly preferred. In preferred pyrroles of formula (K) the substituents R¹⁸ or R¹⁹ are 5-fluoro, 5-chloro, 5-bromo, 4-chloro, 4-bromo, 5-methyl, 5-trifluoromethyl, 3,5-dichloro, or 3,5-dibromo, most preferably 4-bromo, or 5-bromo. A particularly preferred value is that in which R¹⁸ and R¹⁹ are both hydrogen.

Preferred Ar² groups are
a phenyl group of formula (I), most preferably one in which R¹⁷ is hydrogen, a pyridine group of formula (J), most preferably one in which R¹⁷ is hydrogen, a pyrrole of formula (K), most preferably one in which R¹⁷ is hydrogen, or 5-halo, 5-methyl, 5-trifluoromethyl, 4-bromo, 4-chloro, 3,5-dichloro, or 3,5-dibromo, a furan of formula (L), most preferably furan-3-yl, which bears R¹⁶ at C-4 and R¹⁷ is hydrogen, or a thiophene of formula (L), mosty preferably 3-thienyl, which bears R¹⁶ at C-2 or at C-4 and R¹⁷ is hydrogen, or is 2-bromo or 2-methyl. Particularly preferred groups Ar² are those in which R¹⁷ is hydrogen, and which are phenyl, pyrrol-1-yl, or 3-thienyl of formula (I), (K), or (L), respectively.

Preferred Y groups are
C-C single bond, CH(5-tetrazolyl), CH(COOH), CH(5-tetrazolyl)CH₂, CH(COOH)CH₂, OCH(5-tetrazolyl), OCH(COOH), OCH(5-tetrazolyl)CH₂, OCH(COOH)CH₂, NHCH(5-tetrazolyl), NHCH(COOH), NHCH(5-tetrazolyl)CH₂, NHCH(COOH)CH₂, CH₂CH(5-tetrazolyl), CH₂CH(COOH), O, CO, or NHCO, mostly preferable Y groups are C-C single bond, CH(5-tetrazolyl)CH₂, CH(COOH)CH₂, OCH(5-tetrazolyl), OCH(COOH), or O, especially C-C single bond.

Preferred Ar¹-Y-Ar² groups are
2'-(tetrazol-5-yl)biphenyl-4-yl, biphenyl-4-yl-2'-carboxylic acid, biphenyl-4-yl-2'-sulfonic acid, biphenyl-4-yl-2'-phosphonic acid, 2'-(trifluoromethanesulfonylamino)biphenyl-4-yl, 4-(3-(tetrazol-5-yl)pyridine-2-yl)phenyl, 4-(4-(tetrazol-5-yl)pyridine-3-yl)phenyl, 4-(3-(tetrazol-5-yl)pyridine-4-yl)phenyl, 4-(2-(tetrazol-5-yl)pyridine-3-yl)phenyl, 2-(2-(tetrazol-5-yl)phenyl)pyridine-5-yl, 5-(2-(tetrazol-5-yl)phenyl)pyridine-2-yl, 4-(2-(tetrazol-5-yl)phenoxy)phenyl, 4-(2-(tetrazol-5-yl)benzoyl)phenyl, 4-(phthalamido)phenyl, 2-(N-(phenyl-4-yl)aminocarbonyl)benzenesulfonic acid, 4-(2-(tetrazol-5-yl)-3-thienyl)phenyl, 4-(4-(tetrazol-5-yl)-3-thienyl)phenyl, 4-(2-bromo-4-(tetrazol-5-yl)-3-thienyl)phenyl, 4-(2-methyl-4-(tetrazol-5-yl)-3-thienyl)phenyl, 4-(4-(tetrazol-5-yl)furan-3-yl)phenyl, 4-(2-(tetrazol-5-yl)pyrrol-1-yl)phenyl, 4-(5-bromo-2-(tetrazol-5-yl)pyrrol-1-yl)phenyl, 4-(5-chloro-2-(tetrazol-5-yl)pyrrol-1-yl)phenyl, 4-(5-methyl-2-(tetrazol-5-yl)pyrrol-1-yl)phenyl, 4-(5-fluoro-2-(tetrazol-5-yl)pyrrol-1-yl)phenyl, 4-(2-(tetrazol-5-yl)-5-trifluoromethylpyrrol-1-yl)phenyl, 4-(4-bromo-2-(tetrazol-5-yl)pyrrol-1-yl)phenyl, 4-(4-chloro-2-(tetrazol-5-yl)pyrrol-1-yl)phenyl, 4-(3,5-dichloro-2-(tetrazol-5-yl)pyrrol-1-yl)phenyl, 4-(3,5-dibromo-2-(tetrazol-5-yl)pyrrol-1-yl)phenyl, 3-bromo-2-(2-(tetrazol-5-yl)phenyl)benzofuran-5-yl, 3-chloro-2-(2-(tetrazol-5-yl)phenyl)benzofuran-5-yl, 3-methyl-2-(2-(tetrazol-5-yl)phenyl)benzofuran-5-yl, 3-bromo-2-(2-(tetrazol-5-yl)phenyl)benzothien-5-yl, α-(phenoxy-4-yl)benzeneacetic acid, 4-((phenyl)(tetrazol-5-yl)methoxy)phenyl, 2-(phenyl-4-yl)-3-phenylpropionic acid, 4-(2-phenyl-1-(tetrazol-5-yl)ethyl)phenyl, 4-(2-phenyl-2-(tetrazol-5-yl)ethyl)phenyl, 2-phenyl-3-(phenyl-4-yl)propionic acid, α-(N-(phenyl-4-yl)amino)benzeneacetic acid, or 4-(N-(phenyl)(tetrazol-5-yl)methyl)aminophenyl,
mostly preferred 2'-(tetrazol-5-yl)biphenyl-4-yl, biphenyl-4-yl-2'-carboxylic acid, biphenyl-4-yl-2'-sulfonic acid, biphenyl-4-yl-2'-phosphonic acid, 2'-(trifluoromethanesulfonylamino)biphenyl-4-yl, 4-(3-(tetrazol-5-yl)pyridine-2-yl)phenyl, 4-(4-(tetrazol-5-yl)pyridine-3-yl))phenyl, 4-(3-(tetrazol-5-yl)pyridine-4-yl)phenyl, 4-(2-(tetrazol-5-yl)pyridine-3-yl)phenyl, 2-(2-(tetrazol-5-yl)phenyl)pyridine-5-yl, 5-(2-(tetrazol-5-yl)phenyl)pyridine-2-yl, 4-(2-(tetrazol-5-yl)-3-thienyl)phenyl, 4-(4-(tetrazol-5-yl)-3-thienyl)phenyl, 4-(2-(tetrazol-5-yl)pyrrol-1-yl)phenyl, or 3-bromo-2-(2-(tetrazol-5-yl)phenyl)benzofuran-5-yl,
particularly preferred 2'-(tetrazol-5-yl)biphenyl-4-yl, 4-(2-(tetrazol-5-yl)pyrrol-1-yl)phenyl, or 3-bromo-2-(2-(tetrazol-5-yl)phenyl)benzofuran-5-yl, most particularly preferred 2'-(tetrazol-5-yl)biphenyl-4-yl.

It will be appreciated that the compounds of the invention can contain an asymmetric carbon atom which gives rise to enantiomers. The compounds are normally prepared as racemates and can conveniently be used as such, but individual enantiomers can be isolated by conventional techniques if so desired. Such racemates and individual enantiomers form part of the present invention.

It will also be understood that salts of the compounds of the invention can be prepared, and such salts are included in the invention. They can be any of the well known base or acid addition salts. Examples of base salts are those derived from ammonium hydroxide and alkali and alkaline earth metal hydroxides, carbonates and bicarbonates, as well as salts derived from aliphatic and aromatic amines, aliphatic diamines and hydroxy alkylamines. Bases especially useful in the preparation of such salts include ammonium hydroxide, potassium carbonate, sodium bicarbonate, lithium hydroxide, calcium hydroxide, methylamine, diethylamine, ethylene diamine, cyclohexylamine and ethanolamine. The potassium, sodium and lithium salt forms are particularly preferred.

Acid addition salts are preferably the pharmaceutically acceptable, non-toxic addition salts with suitable acids, such as those with inorganic acids, for example hydrochloric, hydrobromic, nitric, sulphuric or phosphoric acids, or with organic acids, such as organic carboxylic acids, for example glycollic, maleic, fumaric, malic, tartaric, citric, salicylic or o-acetoxybenzoic acids, or organic sulphonic acids, methane sulphonic, 2-hydroxyethane sulphonic, toluene-p-sulphonic or naphthalene-2-sulphonic acids.

In addition to pharmaceutically-acceptable salts, other salts are included in the invention. They may serve as intermediates in the purification of compounds or in the preparation of other, for example, pharmaceutically-acceptable salts, or are useful for identification, characterisation or purification.

Particularly preferred compounds of formula I of the present invention include:
1. 5-Methyl-7-[(2'-(tetrazol-5-yl)biphenyl-4-yl)methylamino]pyrazolo[1,5-a]pyrimidine
2. 5-Methyl-7-[N-methyl-N-((2'-(tetrazol-5-yl)biphenyl-4-yl)methyl)amino]pyrazolo[1,5-a]pyrimidine
3. 5-Ethyl-7-[(2'-(tetrazol-5-yl)biphenyl-4-yl)methylamino]pyrazolo[1,5-a]pyrimidine
4. 5-Ethyl-7-[N-methyl-N-((2'-(tetrazol-5-yl)biphenyl-4-yl)methyl)amino]pyrazolo[1,5-a]pyrimidine
5. 5-Propyl-7-[(2'-(tetrazol-5-yl)biphenyl-4-yl)methylamino]pyrazolo[1,5-a]pyrimidine
6. 7-[N-Methyl-N-((2'-(tetrazol-5-yl)biphenyl-4-yl)methyl)amino]-5-propylpyrazolo[1,5-a]pyrimidine
7. 5-Butyl-7-[(2'-(tetrazol-5-yl)biphenyl-4-yl)methylamino]pyrazolo[1,5-a]pyrimidine
8. 5-Butyl-7-[(N-methyl-N-((2'-(tetrazol-5-yl)biphenyl-4-yl)methyl)amino]pyrazolo[1,5-a]pyrimidine
9. 5-Cyclopropyl-7-[(2'-(tetrazol-5-yl)biphenyl-4-yl)methylamino]pyrazolo[1,5-a]pyrimidine
10. 5-Cyclopropyl-7-[(N-methyl-N-((2'-(tetrazol-5-yl)biphenyl-4-yl)methyl)amino]pyrazolo[1,5-a]pyrimidine
11. 5-Isopropyl-7-[(2'-(tetrazol-5-yl)biphenyl-4-yl)methylamino]pyrazolo[1,5-a]pyrimidine
12. 5-Isopropyl-7-[(N-methyl-N-((2'-(tetrazol-5-yl)biphenyl-4-yl)methyl)amino]pyrazolo[1,5-a]pyrimidine
13. 7-[(N-Ethyl-N-((2'-(tetrazol-5-yl)biphenyl-4-yl)methyl)amino]-5-methylpyrazolo[1,5-a]pyrimidine
14. 5-Methyl-7-[(N-propyl-N-((2'-(tetrazol-5-yl)biphenyl-4-yl)methyl)amino]pyrazolo[1,5-a]pyrimidine
15. 7-[(N-Butyl-N-((2'-(tetrazol-5-yl)biphenyl-4-yl)methyl)amino]-5-methylpyrazolo[1,5-a]pyrimidine
16. 5-Ethyl-7-[N-ethyl-N-((2'-(tetrazol-5-yl)biphenyl-4-yl)methyl)amino]pyrazolo[1,5-a]pyrimidine
17. 5-Ethyl-7-[N-propyl-N-((2'-(tetrazol-5-yl)biphenyl-4-yl)methyl)amino]pyrazolo[1,5-a]pyrimidine
18. 7-[N-Butyl-N-((2'-(tetrazol-5-yl)biphenyl-4-yl)methyl)amino]-5-ethylpyrazolo[1,5-a]pyrimidine
19. 7-[(2'-(Tetrazol-5-yl)biphenyl-4-yl)methylamino]-5-trifluoromethylpyrazolo[1,5-a]pyrimidine
20. 7-[N-Methyl-N-((2'-(tetrazol-5-yl)biphenyl-4-yl)methyl)amino]-5-trifluoromethylpyrazolo[1,5-a]pyrimidine
21. 5-Methylthio-7-[(2'-(tetrazol-5-yl)biphenyl-4-yl)methylamino]pyrazolo[1,5-a]pyrimidine
22. 5-Ethylthio-7-[(2'-(tetrazol-5-yl)biphenyl-4-yl)methylamino]pyrazolo[1,5-a]pyrimidine
23. 5-Methyl-7-[(2'-(tetrazol-5-yl)biphenyl-4-yl)methoxy]pyrazolo[1,5-a]pyrimidine
24. 5-Ethyl-7-[(2'-(tetrazol-5-yl)biphenyl-4-yl)methoxy]pyrazolo[1,5-a]pyrimidine
25. 5-Methyl-7-[(2'-(tetrazol-5-yl)biphenyl-4-yl)methylthio]pyrazolo[1,5-a]pyrimidine
26. 5-Ethyl-7-[(2'-(tetrazol-5-yl)biphenyl-4-yl)methylthio]pyrazolo[1,5-a]pyrimidine
27. 5-Propyl-7-[(2'-(tetrazol-5-yl)biphenyl-4-yl)methylthio]pyrazolo[1,5-a]pyrimidine
28. 5-Butyl-7-[(2'-(tetrazol-5-yl)biphenyl-4-yl)methylthio]pyrazolo[1,5-a]pyrimidine
29. 5-Cyclopropyl-7-[(2'-(tetrazol-5-yl)biphenyl-4-yl)methylthio]pyrazolo[1,5-a]pyrimidine
30. 5-Isopropyl-7-[(2'-(tetrazol-5-yl)biphenyl-4-yl)methylthio]pyrazolo[1,5-a]pyrimidine
31. 7-[(2'-(Tetrazol-5-yl)biphenyl-4-yl)methylthio]-5-trifluoromethylpyrazolo[1,5-a]pyrimidine
32. 5-Methyl-7-[(2'-(tetrazol-5-yl)biphenyl-4-yl)amino]pyrazolo[1,5-a]pyrimidine
33. 5-Ethyl-7-[(2'-(tetrazol-5-yl)biphenyl-4-yl)amino]pyrazolo[1,5-a]pyrimidine
34. 5-Cyclopropyl-7-[(2'-(tetrazol-5-yl)biphenyl-4-yl)amino]pyrazolo[1,5-a]pyrimidine
35. 7-[(2'-(Tetrazol-5-yl)biphenyl-4-yl)amino]-5-trifluoromethylpyrazolo[1,5-a]pyrimidine
36. 5-Ethyl-7-[N-methyl-N-(2'-(tetrazol-5-yl)biphenyl-4-yl)amino]pyrazolo[1,5-a]pyrimidine
37. 4'-[(5-Methylpyrazolo[1,5-a]pyrimidine-7-yl)amino]biphenyl-2-carboxylic acid
38. 4'-[(5-Ethylpyrazolo[1,5-a]pyrimidine-7-yl)amino]biphenyl-2-carboxylic acid
39. 4'-[(5-Methylpyrazolo[1,5-a]pyrimidine-7-yl)amino]biphenyl-2-sulfonic acid
40. 4'-[(5-Ethylpyrazolo[1,5-a]pyrimidine-7-yl)amino]biphenyl-2-sulfonic acid
41. 4'-[(5-Methylpyrazolo[1,5-a]pyrimidine-7-yl)amino]biphenyl-2-phosphonic acid
42. 4'-[(5-Ethylpyrazolo[1,5-a]pyrimidine-7-yl)amino]biphenyl-2-phosphonic acid
43. 5-Ethyl-7-[(2'-(trifluoromethanesulfonamido)biphenyl-4-yl)amino]pyrazolo[1,5-a]pyrimidine
44. 5-Methyl-7-[(2'-(tetrazol-5-yl)biphenyl-4-yl)methylamino]-[1,2,4]triazolo[1,5-a]pyrimidine
45. 5-Ethyl-7-[(2'-(tetrazol-5-yl)biphenyl-4-yl)methylamino]-[1,2,4]triazolo[1,5-a]pyrimidine
46. 5-Ethyl-7-[N-methyl-N-((2'-(tetrazol-5-yl)biphenyl-4-yl)methyl)amino]-[1,2,4]triazolo[1,5-a]pyrimidine
47. 5-Ethyl-2-methyl-7-[(2'-(tetrazol-5-yl)biphenyl-4-yl)methylamino]-[1,2,4]triazolo[1,5-a]pyrimidine
48. 2,5-Diethyl-7-[(2'-(tetrazol-5-yl)biphenyl-4-yl)methylamino]-[1,2,4]triazolo[1,5-a]pyrimidine
49. 5-Ethyl-2-methylthio-7-[(2'-(tetrazol-5-yl)biphenyl-4-yl)methylamino]-[1,2,4]triazolo[1,5-a]pyrimidine
50. 2-Amino-5-ethyl-7-[(2'-(tetrazol-5-yl)biphenyl-4-yl)methylamino]-[1,2,4]triazolo[1,5-a]pyrimidine
51. 5-Ethyl-7-[(2'-(tetrazol-5-yl)biphenyl-4-yl)methylamino]-2-trifluoromethanesulfonamido-[1,2,4]triazolo[1,5-a]pyrimidine
52. 3-Chloro-5-methyl-7-[(2'-(tetrazol-5-yl)biphenyl-4-yl)methylamino]pyrazolo[1,5-a]pyrimidine
53. 3-Chloro-5-ethyl-7-[(2'-(tetrazol-5-yl)biphenyl-4-yl)methylamino]pyrazolo[1,5-a]pyrimidine
54. 3-Chloro-7-[(2'-(tetrazol-5-yl)biphenyl-4-yl)methylamino]-5-trifluoromethylpyrazolo[1,5-a]pyrimidine
55. 3-Bromo-5-methyl-7-[(2'-(tetrazol-5-yl)biphenyl-4-yl)methylamino]pyrazolo[1,5-a]pyrimidine
56. 3-Bromo-5-ethyl-7-[(2'-(tetrazol-5-yl)biphenyl-4-yl)methylamino]pyrazolo[1,5-a]pyrimidine
57. 5-Methyl-3-nitro-7-[(2'-(tetrazol-5-yl)biphenyl-4-yl)methylamino]pyrazolo[1,5-a]pyrimidine
58. 5-Ethyl-3-nitro-7-[(2'-(tetrazol-5-yl)biphenyl-4-yl)methylamino]pyrazolo[1,5-a]pyrimidine
59. 3-Cyano-5-methyl-7-[(2'-(tetrazol-5-yl)biphenyl-4-yl)methylamino]pyrazolo[1,5-a]pyrimidine
60. 3-Cyano-5-ethyl-7-[(2'-(tetrazol-5-yl)biphenyl-4-yl)methylamino]pyrazolo[1,5-a]pyrimidine
61. 3,5-Dimethyl-7-[(2'-(tetrazol-5-yl)biphenyl-4-yl)methylamino]pyrazolo[1,5-a]pyrimidine
62. 3-Methyl-5-ethyl-7-[(2'-(tetrazol-5-yl)biphenyl-4-yl)methylamino]pyrazolo[1,5-a]pyrimidine
63. 5-Methyl-3-ethyl-7-[(2'-(tetrazol-5-yl)biphenyl-4-yl)methylamino]pyrazolo[1,5-a]pyrimidine
64. 3,5-Diethyl-7-[(2'-(tetrazol-5-yl)biphenyl-4-yl)methylamino]pyrazolo[1,5-a]pyrimidine
65. 5-Methyl-3-propyl-7-[(2'-(tetrazol-5-yl)biphenyl-4-yl)methylamino]pyrazolo[1,5-a]pyrimidine
66. 5-Ethyl-3-propyl-7-[(2'-(tetrazol-5-yl)biphenyl-4-yl)methylamino]pyrazolo[1,5-a]pyrimidine
67. 5-Ethyl-7-[(2'-(tetrazol-5-yl)biphenyl-4-yl)methylamino]-3-trifluoromethylpyrazolo[1,5-a]pyrimidine
68. 2-Methylthio-5-ethyl-7-[(2'-(tetrazol-5-yl)biphenyl-4-yl)methylamino]pyrazolo[1,5-a]pyrimidine
69. 2-Methyl-5-ethyl-7-[(2'-(tetrazol-5-yl)biphenyl-4-yl)methylamino]pyrazolo[1,5-a]pyrimidine
70. 5-Ethyl-7-[(2'-(tetrazol-5-yl)biphenyl-4-yl)methylamino]-2-trifluoromethanesulfonamidopyrazolo[1,5-a]pyrimidine
71. 5-Ethyl-5-methylsulfonyl-7-[(2'-(tetrazol-5-yl)biphenyl-4-yl)methylamino]pyrazolo[1,5-a]pyrimidine
72. 2-Cyano-5-ethyl-7-[(2'-(tetrazol-5-yl)biphenyl-4-yl)methylamino]pyrazolo[1,5-a]pyrimidine
73. 5-Ethyl-7-[(2'-(tetrazol-5-yl)biphenyl-4-yl)methylamino]pyrazolo[1,5-a]pyrimidine-2-carboxylic acid
74. 2,3-Dimethyl-5-ethyl-7-[(2'-(tetrazol-5-yl)biphenyl-4-yl)methylamino]pyrazolo[1,5-a]pyrimidine
75. 2,5-Diethyl-3-methyl-7-[(2'-(tetrazol-5-yl)biphenyl-4-yl)methylamino]pyrazolo[1,5-a]pyrimidine
76. 2-Aminocarbonyl-5-ethyl-7-[(2'-(tetrazol-5-yl)biphenyl-4-yl)methylamino]pyrazolo[1,5-a]pyrimidine
77. 2-Aminosulfonyl-5-ethyl-7-[(2'-(tetrazol-5-yl)biphenyl-4-yl)methylamino]pyrazolo[1,5-a]pyrimidine
78. 5-Ethyl-7-[(2'-(tetrazol-5-yl)biphenyl-4-yl)methylamino]-3-(trifluoromethylsulfonyl)pyrazolo[1,5-a]pyrimidine
79. 2-Amino-5-ethyl-7-[(2'-(tetrazol-5-yl)biphenyl-4-yl)methylamino]pyrazolo[1,5-a]pyrimidine
80. 3,5-Dimethyl-7-[N-methyl-N-((2'-(tetrazol-5-yl)biphenyl-4-yl)methyl)amino]pyrazolo[1,5-a]pyrimidine
81. 5-Ethyl-3-methyl-7-[N-methyl-N-((2'-(tetrazol-5-yl)biphenyl-4-yl)methyl)amino]pyrazolo[1,5-a]pyrimidine
82. 3,5-Diethyl-7-[N-methyl-N-((2'-(tetrazol-5-yl)biphenyl-4-yl)methyl)amino]pyrazolo[1,5-a]pyrimidine
83. 3-Cyano-5-methyl-7-[N-methyl-N-((2'-(tetrazol-5-yl)biphenyl-4-yl)methyl)amino]pyrazolo[1,5-a]pyrimidine
84. 3-Cyano-5-ethyl-7-[N-methyl-N-((2'-(tetrazol-5-yl)biphenyl-4-yl)methyl)amino]pyrazolo[1,5-a]pyrimidine
85. 5-Methyl-3-nitro-7-[N-methyl-N-((2'-(tetrazol-5-yl)biphenyl-4-yl)methyl)amino]pyrazolo[1,5-a]pyrimidine
86. 5-Ethyl-3-nitro-7-[N-methyl-N-((2'-(tetrazol-5-yl)biphenyl-4-yl)methyl)amino]pyrazolo[1,5-a]pyrimidine
87. 3-Chloro-5-ethyl-7-[N-methyl-N-((2'-(tetrazol-5-yl)biphenyl-4-yl)methyl)amino]pyrazolo[1,5-a]pyrimidine
88. 3-Bromo-5-ethyl-7-[N-methyl-N-((2'-(tetrazol-5-yl)biphenyl-4-yl)methyl)amino]pyrazolo[1,5-a]pyrimidine
89. 4'-[N-((5-Methylpyrazolo(1,5-a]pyrimidine-7-yl)methyl)amino]biphenyl-2-carboxylic acid
90. 4'-[N-((5-Ethylpyrazolo[1,5-a]pyrimidine-7-yl)methyl)amino]biphenyl-2-carboxylic acid
91. 4'-[N-((5-Methylpyrazolo[1,5-a]pyrimidine-7-yl)methyl)amino]biphenyl-2-sulfonic acid
92. 4'-[N-((5-Ethylpyrazolo[1,5-a]pyrimidine-7-yl)methyl)amino]biphenyl-2-sulfonic acid
93. 4'-[N-((5-Ethylpyrazolo[1,5-a]pyrimidine-7-yl)methyl)amino]biphenyl-2-phosphonic acid
94. 5-Ethyl-7-[(2'-(trifluoromethanesulfonylamido)biphenyl-4-yl)methylamino]pyrazolo[1,5-a]pyrimidine
95. Pivaloyloxymethyl 4'-[N-((5-Ethylpyrazolo[1,5-a]pyrimidine-7-yl)methyl)amino]biphenyl-2-carboxylate
96. 5-Ethyl-7-[((4-phthalamido)phenyl)amino]pyrazolo[1,5-a]pyrimidine
97. 2-[(N-(4-(5-Ethylpyrazolo[1,5-a]pyrimidine-7-yl)amino)phenyl)aminocarbony]benzene sulfonic acid
98. 2-Methyl-4-[(2'-(tetrazol-5-yl)biphenyl-4-yl)methylamino]imidazo[1,5-a]pyrimidine
99. 2-Ethyl-4-[(2'-(tetrazol-5-yl)biphenyl-4-yl)methylamino]imidazo[1,5-a]pyrimidine
100. 2-Ethyl-4-[(2'-(tetrazol-5-yl)biphenyl-4-yl)methylamino]imidazo[1,5-a]pyrimidine-6-carboxylic acid
101. 2-Methyl-4-[(2'-(tetrazol-5-yl)biphenyl-4-yl)methylamino]imidazo[1,5-a]pyrimidine-6-carboxylic acid
102. 2-Ethyl-8-methyl-4-[(2'-(tetrazol-5-yl)biphenyl-4-yl)methylamino]imidazo[1,5-a]pyrimidine-6-carboxylic acid
103. 6-Aminocarbonyl-2-ethyl-4-[(2'-(tetrazol-5-yl)biphenyl-4-yl)methylamino]imidazo[1,5-a]pyrimidine
104. 2-Ethyl-8-nitro-4-[(2'-(tetrazol-5-yl)biphenyl-4-yl)methylamino]imidazo[1,5-a]pyrimidine
105. 8-Cyano-2-ethyl-4-[(2'-(tetrazol-5-yl)biphenyl-4-yl)methylamino]imidazo[1,5-a]pyrimidine
106. 2-Ethyl-8-methyl-4-[(2'-(tetrazol-5-yl)biphenyl-4-yl)methylamino]imidazo[1,5-a]pyrimidine
107. 2,8-Diethyl-4-[(2'-(tetrazol-5-yl)biphenyl-4-yl)methylamino]imidazo[1,5-a]pyrimidine
108. 7-Methyl-5-[(2'-(tetrazol-5-yl)biphenyl-4-yl)methylamino]imidazo[1,2-a]pyrimidine-2-carboxylic acid
109. 7-Ethyl-5-[(2'-(tetrazol-5-yl)biphenyl-4-yl)methylamino]imidazo[1,2-a]pyrimidine-2-carboxylic acid
110. 7-Methyl-5-[(2'-(tetrazol-5-yl)biphenyl-4-yl)methylamino]imidazo[1,2-a]pyrimidine-3-carboxylic acid
111. 7-Ethyl-5-[(2'-(tetrazol-5-yl)biphenyl-4-yl)methylamino]imidazo[1,2-a]pyrimidine-3-carboxylic acid
112. 7-Ethyl-3-formyl-5-[(2'-(tetrazol-5-yl)biphenyl-4-yl)methylamino]imidazo[1,2-a]pyrimidine
113. 7-Ethyl-3-hydroxymethyl-5-[(2'-(tetrazol-5-yl)biphenyl-4-yl)methylamino]imidazo[1,2-a]pyrimidine
114. 3-Cyano-7-ethyl-5-[(2'-(tetrazol-5-yl)biphenyl-4-yl)methylamino]imidazo[1,2-a]pyrimidine
115. 3-Aminocarbonyl-7-ethyl-5-[(2'-(tetrazol-5-yl)biphenyl-4-yl)methylamino]imidazo[1,2-a]pyrimidine
116. Methyl 7-Ethyl-5-[(2'-(tetrazol-5-yl)biphenyl-4-yl)methylamino]imidazo[1,2-a]pyrimidine-3-carboxylate
117. Ethyl 7-Ethyl-5-[(2'-(tetrazol-5-yl)biphenyl-4-yl)methylamino]imidazo[1,2-a]pyrimidine-3-carboxylate
118. 5-Methyl-7-[(5-(2-(tetrazol-5-yl)phenyl)pyridine-2-yl)methylamino]pyrazolo[1,5-a]pyrimidine
119. 5-Ethyl-7-[(5-(2-(tetrazol-5-yl)phenyl)pyridine-2-yl)methylamino]pyrazolo[1,5-a]pyrimidine
120. 5-Methyl-7-[(2-(2-(tetrazol-5-yl)phenyl)pyridine-5-yl)methylamino]pyrazolo[1,5-a]pyrimidine
121. 5-Ethyl-7-[(2-(2-(tetrazol-5-yl)phenyl)pyridine-5-yl)methylamino]pyrazolo[1,5-a]pyrimidine
122. 5-Methyl-7-[(4-(2-(tetrazol-5-yl)pyridine-3-yl)phenyl)methylamino]pyrazolo[1,5-a]pyrimidine
123. 5-Ethyl-7-[(4-(2-(tetrazol-5-yl)pyridine-3-yl)phenyl)methylamino]pyrazolo[1,5-a]pyrimidine
124. 5-Methyl-7-[(4-(3-(tetrazol-5-yl)pyridine-4-yl)phenyl)methylamino]pyrazolo[1,5-a]pyrimidine
125. 5-Ethyl-7-[(4-(3-(tetrazol-5-yl)pyridine-4-yl)phenyl)methylamino]pyrazolo[1,5-a]pyrimidine
126. 5-Methyl-7-[(4-(4-(tetrazol-5-yl)pyridine-3-yl)phenyl)methylamino]pyrazolo[1,5-a]pyrimidine
127. 5-Ethyl-7-[(4-(4-(tetrazol-5-yl)pyridine-3-yl)phenyl)methylamino]pyrazolo[1,5-a]pyrimidine
128. 5-Methyl-7-[(4-(3-(tetrazol-5-yl)pyridine-2-yl)phenyl)methylamino]pyrazolo[1,5-a]pyrimidine
129. 5-Ethyl-7-[(4-(3-(tetrazol-5-yl)pyridine-2-yl)phenyl)methylamino]pyrazolo[1,5-a]pyrimidine
130. 5-Methyl-7-[(4-(2-(tetrazol-5-yl)phenoxy)phenyl)methylamino]pyrazolo[1,5-a]pyrimidine
131. 5-Ethyl-7-[(4-(2-(tetrazol-5-yl)phenoxy)phenyl)methylamino]pyrazolo[1,5-a]pyrimidine
132. 5-Methyl-7-[(4-(2-(tetrazol-5-yl)benzoyl)phenyl)methylamino]pyrazolo[1,5-a]pyrimidine
133. 5-Ethyl-7-[(4-(2-(tetrazol-5-yl)benzoyl)phenyl)methylamino]pyrazolo[1,5-a]pyrimidine
134. 5-Methyl-7-[(4-(2-(tetrazol-5-yl)-3-thienyl)phenyl)methylamino]pyrazolo[1,5-a]pyrimidine
135. 5-Ethyl-7-[(4-(2-(tetrazol-5-yl)-3-thienyl)phenyl)methylamino]pyrazolo[1,5-a]pyrimidine
136. 5-Methyl-7-[(4-(4-(tetrazol-5-yl)-3-thienyl)phenyl)methylamino]pyrazolo[1,5-a]pyrimidine
137. 5-Ethyl-7-[(4-(4-(tetrazol-5-yl)-3-thienyl)phenyl)methylamino]pyrazolo[1,5-a]pyrimidine
138. 5-Methyl-7-[(4-(4-(tetrazol-5-yl)furan-3-yl)phenyl)methylamino]pyrazolo[1,5-a]pyrimidine
139. 5-Ethyl-7-[(4-(4-(tetrazol-5-yl)furan-3-yl)phenyl)methylamino]pyrazolo[1,5-a]pyrimidine
140. 5-Ethyl-7-[(4-(2-methyl-4-(tetrazol-5-yl)-3-thienyl)phenyl)methylamino]pyrazolo[1,5-a]pyrimidine
141. 7-[(4-(2-Bromo-4-(tetrazol-5-yl)-3-thienyl)phenyl)methylamino]-5-ethylpyrazolo[1,5-a]pyrimidine
142. α-[4-(N-(5-Methylpyrazolo[1,5-a]pyrimidine-7-yl)methylamino)phenoxy]benzeneacetic acid
143. α-[4-(N-(5-Ethylpyrazolo[1,5-a]pyrimidine-7-yl)methylamino)phenoxy]benzeneacetic acid
144. 5-Methyl-7-[(4-((phenyl)(tetrazol-5-yl)methoxy)phenyl)methylamino]pyrazolo[1,5-a]pyrimidine
145. 5-Ethyl-7-[(4-((phenyl)(tetrazol-5-yl)methoxy)phenyl)methylamino]pyrazolo[1,5-a]pyrimidine
146. 5-Methyl-7-[(4-(N-((phenyl)(tetrazol-5-yl)methyl)amino)phenyl)methylamino]pyrazolo[1,5-a]pyrimidine
147. 5-Ethyl-7-[(4-(N-((phenyl)(tetrazol-5-yl)methyl)amino)phenyl)methylamino]pyrazolo[1,5-a]pyrimidine
148. 5-Methyl-7-[(4-(2-(phenyl)-2-(tetrazol-5-yl)ethyl)phenyl)methylamino]pyrazolo[1,5-a]pyrimidine
149. 5-Ethyl-7-[(4-(2-(phenyl)-2-(tetrazol-5-yl)ethyl)phenyl)methylamino]pyrazolo[1,5-a]pyrimidine
150. 3-[4-(N-(5-Ethylpyrazolo[1,5-a]pyrimidine-7-yl)methylamino)phenyl]-2-phenylpropionic acid
151. α-Amino-N-[4-(N'-(5-Ethylpyrazolo[1,5-a]pyrimidine-7-yl)methylamino)phenyl]benzeneacetic acid
152. 2-[4-(N-(5-Methylpyrazolo[1,5-a]pyrimidine-7-yl)methylamino)phenyl]-3-phenylpropionic acid
153. 2-[4-(N-(5-Ethylpyrazolo[1,5-a]pyrimidine-7-yl)methylamino)phenyl]-3-phenylpropionic acid
154. 5-Methyl-7-[(4-(2-(phenyl)-1-(tetrazol-5-yl)ethyl)phenyl)methylamino]pyrazolo[1,5-a]pyrimidine
155. 5-Ethyl-7-[(4-(2-(phenyl)-1-(tetrazol-5-yl)ethyl)phenyl)methylamino]pyrazolo[1,5-a]pyrimidine
156. α-[4-(N-(5-Ethylpyrazolo[1,5-a]pyrimidine-7-yl)amino)phenoxy]benzeneacetic acid
157. 5-Ethyl-7-[N-(4-((phenyl)(tetrazol-5-yl)methoxy)phenyl)amino]pyrazolo[1,5-a]pyrimidine
158. 2-[4-(N-(5-Ethylpyrazolo[1,5-a]pyrimidine-7-yl)amino)phenoxy]-3-phenylpropionic acid
159. 5-Ethyl-7-[N-(4-(2-(phenyl)-1-(tetrazol-5-yl)ethoxy)phenyl)amino]pyrazolo[1,5-a]pyrimidine
160. 5-Methyl-7-[(4-(2-(tetrazol-5-yl)pyrrol-1-yl)phenyl)methylamino]pyrazolo[1,5-a]pyrimidine
161. 5-Ethyl-7-[(4-(2-(tetrazol-5-yl)pyrrol-1-yl)phenyl)methylamino]pyrazolo[1,5-a]pyrimidine
162. 7-[(4-(5-Bromo-2-(tetrazol-5-yl)pyrrol-1-yl)phenyl)methylamino]-5-methylpyrazolo[1,5-a]pyrimidine
163. 7-[(4-(5-Bromo-2-(tetrazol-5-yl)pyrrol-1-yl)phenyl)methylamino]-5-ethylpyrazolo[1,5-a]pyrimidine
164. 7-[(4-(5-Chloro-2-(tetrazol-5-yl)pyrrol-1-yl)phenyl)methylamino]-5-ethylpyrazolo[1,5-a]pyrimidine
165. 5-Ethyl-7-[(4-(5-methyl-2-(tetrazol-5-yl)pyrrol-1-yl)phenyl)methylamino]pyrazolo[1,5-a]pyrimidine
166. 5-Ethyl-7-[(4-(5-fluoro-2-(tetrazol-5-yl)pyrrol-1-yl)phenyl)methylamino]pyrazolo[1,5-a]pyrimidine
167. 5-Ethyl-7-[(4-(2-(tetrazol-5-yl)-5-trifluoromethylpyrrol-1-yl)phenyl)methylamino]pyrazolo[1,5-a]pyrimidine
168. 7-[(4-(4-Bromo-2-(tetrazol-5-yl)pyrrol-1-yl)phenyl)methylamino]-5-ethylpyrazolo[1,5-a]pyrimidine
169. 7-[(4-(4-Chloro-2-(tetrazol-5-yl)pyrrol-1-yl)phenyl)methylamino]-5-ethylpyrazolo[1,5-a]pyrimidine
170. 7-[(4-(3,5-Dichloro-2-(tetrazol-5-yl)pyrrol-1-yl)phenyl)methylamino]-5-ethylpyrazolo[1,5-a]pyrimidine
171. 7-[(4-(3,5-Dibromo-2-(tetrazol-5-yl)pyrrol-1-yl)phenyl)methylamino]-5-ethylpyrazolo[1,5-a]pyrimidine
172. 5-Methyl-7-[N-methyl-N-((4-(2-(tetrazol-5-yl)pyrrol-1-yl)phenyl)methyl)amino]pyrazolo[1,5-a]pyrimidine
173. 5-Ethyl-7-[N-methyl-N-((4-(2-(tetrazol-5-yl)pyrrol-1-yl)phenyl)methyl)amino]pyrazolo[1,5-a]pyrimidine
174. 7-[(3-Bromo-2-(2-(tetrazol-5-yl)phenyl)benzofuran-5-yl)methylamino]-5-methylpyrazolo[1,5-a]pyrimidine
175. 7-[(3-Bromo-2-(2-(tetrazol-5-yl)phenyl)benzofuran-5-yl)methylamino]-5-ethylpyrazolo[1,5-a]pyrimidine
176. 7-[(3-Bromo-2-(2-(tetrazol-5-yl)phenyl)benzofuran-5-yl)methylamino]-5-propylpyrazolo[1,5-a]pyrimidine
177. 7-[(3-Bromo-2-(2-(tetrazol-5-yl)phenyl)benzofuran-5-yl)methylamino]-5-butylpyrazolo[1,5-a]pyrimidine
178. 7-[(3-Bromo-2-(2-(tetrazol-5-yl)phenyl)benzofuran-5-yl)methylamino]-5-cyclopropylpyrazolo[1,5-a]pyrimidine
179. 7-[(3-Bromo-2-(2-(tetrazol-5-yl)phenyl)benzofuran-5-yl)methylamino]-3-chloro-5-methylpyrazolo[1,5-a]pyrimidine
180. 7-[(3-Bromo-2-(2-(tetrazol-5-yl)phenyl)benzofuran-5-yl)methylamino]-3-chloro-5-ethylpyrazolo[1,5-a]pyrimidine
181. 7-[(3-Bromo-2-(2-(tetrazol-5-yl)phenyl)benzofuran-5-yl)methylamino]-5-methyl-3-nitropyrazolo[1,5-a]pyrimidine
182. 7-[(3-Bromo-2-(2-(tetrazol-5-yl)phenyl)benzofuran-5-yl)methylamino]-5-ethyl-3-nitropyrazolo[1,5-a]pyrimidine
183. 7-[(3-Bromo-2-(2-(tetrazol-5-yl)phenyl)benzofuran-5-yl)methylamino]-3-cyano-5-ethylpyrazolo[1,5-a]pyrimidine
184. 7-[(3-Bromo-2-(2-(tetrazol-5-yl)phenyl)benzofuran-5-yl)methylamino]-5-ethyl-3-methylpyrazolo[1,5-a]pyrimidine
185. 3-Bromo-7-[(3-Bromo-2-(2-(tetrazol-5-yl)phenyl)benzofuran-5-yl)methylamino]-5-ethylpyrazolo[1,5-a]pyrimidine
186. 7-[(3-Bromo-2-(2-(tetrazol-5-yl)phenyl)benzofuran-5-yl)methylamino]-3,5-diethylpyrazolo[1,5-a]pyrimidine
187. 3-Cyano-5-ethyl-7-[(4-(2-(tetrazol-5-yl)pyrrol-1-yl)phenyl)methylamino]pyrazolo[1,5-a]pyrimidine
188. 3-Bromo-5-ethyl-7-[(4-(2-(tetrazol-5-yl)pyrrol-1-yl)phenyl)methylamino]pyrazolo[1,5-a]pyrimidine
189. 3-Chloro-5-methyl-7-[(4-(2-(tetrazol-5-yl)pyrrol-1-yl)phenyl)methylamino]pyrazolo[1,5-a]pyrimidine
190. 3-Cyano-5-ethyl-7-[(4-(2-(tetrazol-5-yl)pyrrol-1-yl)phenyl)methylamino]pyrazolo[1,5-a]pyrimidine
191. 5-Ethyl-7-[(4-(2-(tetrazol-5-yl)pyrrol-1-yl)phenyl)methylamino]-3-nitropyrazolo[1,5-a]pyrimidine
192. 5-Ethyl-7-[(4-(2-(tetrazol-5-yl)pyrrol-1-yl)phenyl)methylamino]-3-methylpyrazolo[1,5-a]pyrimidine
193. 4-[(3-Bromo-2-(2-(tetrazol-5-yl)phenyl)benzofuran-5-yl)methylamino]-2-methylimidazo[1,5-a]pyrimidine
194. 4-[(3-Bromo-2-(2-(tetrazol-5-yl)phenyl)benzofuran-5-yl)methylamino]-2-ethylimidazo[1,5-a]pyrimidine
195. 4-[(3-Bromo-2-(2-(tetrazol-5-yl)phenyl)benzofuran-5-yl)methylamino]-2-ethyl-8-methylimidazo[1,5-a]pyrimidine
196. 4-[(3-Bromo-2-(2-(tetrazol-5-yl)phenyl)benzofuran-5-yl)methylamino]-2-ethylimidazo[1,5-a]pyrimidine-6-carboxylic acid
197. 4-[(3-Bromo-2-(2-(tetrazol-5-yl)phenyl)benzofuran-5-yl)methylamino]-2-methylimidazo[1,5-a]pyrimidine-6-carboxylic acid
198. 2-Methyl-4-[(4-(2-(tetrazol-5-yl)pyrrol-1-yl)phenyl)methylamino]imidazo[1,5-a]pyrimidine
199. 2-Ethyl-4-[(4-(2-(tetrazol-5-yl)pyrrol-1-yl)phenyl)methylamino]imidazo[1,5-a]pyrimidine
200. 2-Methyl-4-[(4-(2-(tetrazol-5-yl)pyrrol-1-yl)phenyl)methylamino]imidazo[1,5-a]pyrimidine-6-carboxylic acid
201. 2-Ethyl-4-[(4-(2-(tetrazol-5-yl)pyrrol-1-yl)phenyl)methylamino]imidazo[1,5-a]pyrimidine-6-carboxylic acid
202. 2-Ethyl-4-[(4-(2-(tetrazol-5-yl)pyrrol-1-yl)phenyl)methylamino]-8-methylimidazo[1,5-a]pyrimidine
203. 5-[(3-Bromo-2-(2-(tetrazol-5-yl)phenyl)benzofuran-5-yl)methylamino]-7-methylimidazo[1,2-a]pyrimidine-2-carboxylic acid
204. 5-[(3-Bromo-2-(2-(tetrazol-5-yl)phenyl)benzofuran-5-yl)methylamino]-7-ethylimidazo[1,2-a]pyrimidine-2-carboxylic acid
205. 5-[(3-Bromo-2-(2-(tetrazol-5-yl)phenyl)benzofuran-5-yl)methylamino]-7-methylimidazo[1,2-a]pyrimidine-3-carboxylic acid
206. 5-[(3-Bromo-2-(2-(tetrazol-5-yl)phenyl)benzofuran-5-yl)methylamino]-7-ethylimidazo[1,2-a]pyrimidine-3-carboxylic acid
207. 5-[(3-Bromo-2-(2-(tetrazol-5-yl)phenyl)benzofuran-5-yl)methylamino]-7-ethyl-3-formylimidazo[1,2-a]pyrimidine
208. 5-[(3-Bromo-2-(2-(tetrazol-5-yl)phenyl)benzofuran-5-yl)methylamino]-7-ethyl-3-hydroxymethylimidazo[1,2-a]pyrimidine
209. 5-[(3-Bromo-2-(2-(tetrazol-5-yl)phenyl)benzofuran-5-yl)methylamino]-7-ethylimidazo[1,2-a]pyrimidine
210. 7-Methyl-5-[(4-(2-(tetrazol-5-yl)pyrrol-1-yl)phenyl)methylamino]imidazo[1,5-a]pyrimidine-3-carboxylic acid
211. 7-Ethyl-5-[(4-(2-(tetrazol-5-yl)pyrrol-1-yl)phenyl)methylamino]imidazo[1,5-a]pyrimidine-3-carboxylic acid
212. 7-Ethyl-5-[(4-(2-(tetrazol-5-yl)pyrrol-1-yl)phenyl)methylamino]imidazo[1,5-a]pyrimidine-2-carboxylic acid
213. 3-Cyano-7-ethyl-5-[(4-(2-(tetrazol-5-yl)pyrrol-1-yl)phenyl)methylamino]imidazo[1,5-a]pyrimidine
214. 7-Ethyl-5-[(4-(2-(tetrazol-5-yl)pyrrol-1-yl)phenyl)methylamino]-3-formylimidazo[1,5-a]pyrimidine
215. 7-Ethyl-5-[(4-(2-(tetrazol-5-yl)pyrrol-1-yl)phenyl)methylamino]-3-hydroxymethylimidazo[1,5-a]pyrimidine
216. 2-[N-(5-Methylpyrazolo[1,5-a]pyrimidine-7-yl)-N-((2'-(tetrazol-5-yl)biphenyl-4-yl)methyl)amino]acetic acid
217. 2-[N-(5-Ethylpyrazolo[1,5-a]pyrimidine-7-yl)-N-((2'-(tetrazol-5-yl)biphenyl-4-yl)methyl)amino]acetic acid
218. Ethyl 2-[N-(5-Methylpyrazolo[1,5-a]pyrimidine-7-yl)-N-((2'-(tetrazol-5-yl)biphenyl-4-yl)methyl)amino]acetate
219. Ethyl 2-[N-(5-Ethylpyrazolo[1,5-a]pyrimidine-7-yl)-N-((2'-(tetrazol-5-yl)biphenyl-4-yl)methyl)amino]acetate
220. Methyl 2-[N-(5-Methylpyrazolo[1,5-a]pyrimidine-7-yl)-N-((2'-(tetrazol-5-yl)biphenyl-4-yl)methyl)amino]acetate
221. Methyl 2-[N-(5-Ethylpyrazolo[1,5-a]pyrimidine-7-yl)-N-((2'-(tetrazol-5-yl)biphenyl-4-yl)methyl)amino]acetate
222. 2-[N-(5-Methylpyrazolo[1,5-a]pyrimidine-7-yl)-N-((2'-(tetrazol-5-yl)biphenyl-4-yl)methyl)amino]acetamide
223. 2-[N-(5-Ethylpyrazolo[1,5-a]pyrimidine-7-yl)-N-((2'-(tetrazol-5-yl)biphenyl-4-yl)methyl)amino]acetamide
224. N,N-Dimethyl-2-[N-(5-Ethylpyrazolo[1,5-a]pyrimidine-7-yl)-N-((2'-(tetrazol-5-yl)biphenyl-4-yl)methyl)amino]acetamide
225. 2-[N-(5-Ethylpyrazolo[1,5-a]pyrimidine-7-yl)-N-((2'-(tetrazol-5-yl)biphenyl-4-yl)methyl)amino]-N-methylacetamide
226. 2-[N-(5-Ethylpyrazolo[1,5-a]pyrimidine-7-yl)-N-((2'-(tetrazol-5-yl)biphenyl-4-yl)methyl)amino]acetonitrile
227. 5-[(N-(5-Ethylpyrazolo[1,5-a]pyrimidine-7-yl)-N-((2'-(tetrazol-5-yl)biphenyl-4-yl)methyl)amino)methyl]tetrazole
228. Pivaloyloxymethyl 2-[N-(5-Ethylpyrazolo[1,5-a]pyrimidine-7-yl)-N-((2'-(tetrazol-5-yl)biphenyl-4-yl)methyl)amino]acetate
229. 2-[N-((3-Bromo-2-(2-(tetrazol-5-yl)phenyl)benzofuran-5-yl)methyl)-N-(5-methylpyrazolo[1,5-a]pyrimidine-7-yl)amino]acetic acid
230. 2-[N-((3-Bromo-2-(2-(tetrazol-5-yl)phenyl)benzofuran-5-yl)methyl)-N-(5-ethylpyrazolo[1,5-a]pyrimidine-7-yl)amino]acetic acid
231. Ethyl 2-[N-((3-Bromo-2-(2-(tetrazol-5-yl)phenyl)benzofuran-5-yl)methyl)-N-(5-ethylpyrazolo[1,5-a]pyrimidine-7-yl)amino]acetate
232. Methyl 2-[N-((3-Bromo-2-(2-(tetrazol-5-yl)phenyl)benzofuran-5-yl)methyl)-N-(5-ethylpyrazolo[1,5-a]pyrimidine-7-yl)amino]acetate
233. 2-[N-(5-Methylpyrazolo[1,5-a]pyrimidine-7-yl)-N-((4-(2-(tetrazol-5-yl)pyrrol-1-yl)phenyl)methyl)amino]acetic acid
234. 2-[N-(5-Ethylpyrazolo[1,5-a]pyrimidine-7-yl)-N-((4-(2-(tetrazol-5-yl)pyrrol-1-yl)phenyl)methyl)amino]acetic acid
235. Methyl 2-[N-(5-Ethylpyrazolo[1,5-a]pyrimidine-7-yl)-N-((4-(2-(tetrazol-5-yl)pyrrol-1-yl)phenyl)methyl)amino]acetate
236. Ethyl 2-[N-(5-Ethylpyrazolo[1,5-a]pyrimidine-7-yl)-N-((4-(2-(tetrazol-5-yl)pyrrol-1-yl)phenyl)methyl)amino]acetate
237. 2-[N-(5-Ethylpyrazolo[1,5-a]pyrimidine-7-yl)-N-((4-(2-(tetrazol-5-yl)pyrrol-1-yl)phenyl)methyl)amino]acetamide
238. 2-[N-(3-Chloro-5-methylpyrazolo[1,5-a]pyrimidine-7-yl)-N-((2'-(tetrazol-5-yl)biphenyl-4-yl)methyl)amino]acetic acid
239. 2-[N-(3-Chloro-5-ethylpyrazolo[1,5-a]pyrimidine-7-yl)-N-((2'-(tetrazol-5-yl)biphenyl-4-yl)methyl)amino]acetic acid
240. Ethyl 2-[N-(3-Chloro-5-ethylpyrazolo[1,5-a]pyrimidine-7-yl)-N-((2'-(tetrazol-5-yl)biphenyl-4-yl)methyl)amino]acetate
241. Ethyl 2-[N-(3-Chloro-5-methylpyrazolo[1,5-a]pyrimidine-7-yl)-N-((2'-(tetrazol-5-yl)biphenyl-4-yl)methyl)amino]acetate
242. 2-[N-(5-Ethyl-3-methylpyrazolo[1,5-a]pyrimidine-7-yl)-N-((2'-(tetrazol-5-yl)biphenyl-4-yl)methyl)amino]acetic acid
243. 2-[N-(3,5-Diethylpyrazolo[1,5-a]pyrimidine-7-yl)-N-((2'-(tetrazol-5-yl)biphenyl-4-yl)methyl)amino]acetic acid
244. 2-[N-(5-Ethyl-3-propylpyrazolo[1,5-a]pyrimidine-7-yl)-N-((2'-(tetrazol-5-yl)biphenyl-4-yl)methyl)amino]acetic acid
245. 2-[N-(5-Ethyl-3-nitropyrazolo[1,5-a]pyrimidine-7-yl)-N-((2'-(tetrazol-5-yl)biphenyl-4-yl)methyl)amino]acetic acid
246. 2-[N-(3-Cyano-5-ethylpyrazolo[1,5-a]pyrimidine-7-yl)-N-((2'-(tetrazol-5-yl)biphenyl-4-yl)methyl)amino]acetic acid
247. 2-[N-(3-Bromo-5-ethylpyrazolo[1,5-a]pyrimidine-7-yl)-N-((2'-(tetrazol-5-yl)biphenyl-4-yl)methyl)amino]acetic acid
248. 2-[N-(2,5-Diethyl-3-methylpyrazolo[1,5-a]pyrimidine-7-yl)-N-((2'-(tetrazol-5-yl)biphenyl-4-yl)methyl)amino]acetic acid
249. 2-[N-(5-Ethylpyrazolo[1,5-a]pyrimidine-7-yl)-N-(2'-(tetrazol-5-yl)biphenyl-4-yl)amino]acetic acid
250. Methyl 2-[N-(5-Ethylpyrazolo[1,5-a]pyrimidine-7-yl)-N-(2'-(tetrazol-5-yl)biphenyl-4-yl)amino]acetate
251. Ethyl 2-[N-(5-Ethylpyrazolo[1,5-a]pyrimidine-7-yl)-N-(2'-(tetrazol-5-yl)biphenyl-4-yl)amino]acetate
252. 2-[N-(5-Ethylpyrazolo[1,5-a]pyrimidine-7-yl)-N-(2'-(tetrazol-5-yl)biphenyl-4-yl)amino]acetamide

The most preferred compounds are as follows:
5-Methyl-7-[(2'-(tetrazol-5-yl)biphenyl-4-yl)methylamino]pyrazolo[1,5-a]pyrimidine
5-Ethyl-7-[2'-(tetrazol-5-yl)biphenyl-4-yl)methylamino]pyrazolo[1,5-a]pyramidine
3-Chloro-5-ethyl-7-[(2'-(tetrazol-5-yl)biphenyl-4-yl)methylamino]pyrazolo[1,5-a]pyrmidine
7-[(3-Bromo-2-(2-tetrazol-5-yl)phenyl)benzofuran-5-yl)methylamino]-5-ethylpyrazolo[1,5-a]pyrimidine
2-[N-(5-Methylpyrazolo[1,5-a]pyrimidine-7-yl)-N-((2'-(tetrazol-5-yl)biphenyl-4-yl)methyl)amino]acetic acid
2-[N-(5-Ethylpyrazolo[1,5-a]pyrmidine-7-yl)-N-((2'-(tetrazol-5-yl)biphenyl-4-yl)methyl)amino]acetic acid.

This invention also includes processes for preparing compounds of the formula (I) above. The principal processes are as follows:
1) reacting a compound of the formula (II) 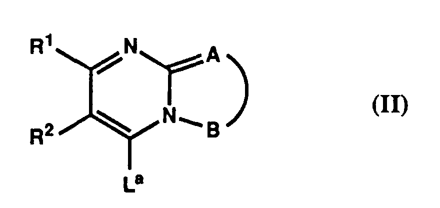 in which L^{a} is a leaving group,
   with a compound of the formula (III)

   HX(CHR³)ₙ - Ar¹ - Y - Ar² (III)
2) reacting a compound of the formula (IV) 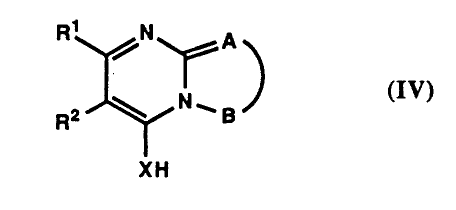 with a compound of the formula (V)

   L^{b}CHR³ - Ar¹ - Y - Ar² (V)

   in which L^{b} is a leaving group.
3) reacting a compound of the formula (IX) 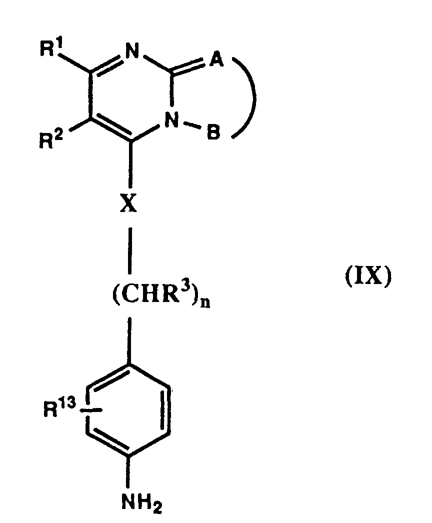 with a compound of the formula X, XI or XII (Scheme 2, below).
4) converting a compound of the formula (I) in which R¹⁶ or R²⁰ is cyano to give a compound of the formula (I) in which R¹⁶ or R²⁰ is COOH.
5) reacting a compound of the formula (I) in which R¹⁶ or R²⁰ is cyano with an azide to give a compound of the formula (I) in which R¹⁶ or R²⁰ is tetrazolyl.
6) oxidising a compound of the formula (I) in which R¹⁶ is mercapto to give a compound in which R¹⁶ is SO₃H.
7) reacting a compound of the formula (I) in which R¹⁶ is amino with trifluoromethane sulfonyl chloride or with trifluorosulfonic acid anhydride to give a compound in which R¹⁶ is NHSO₂CF₃.
8) removing a protecting group, or hydrolysing an ester to give the unprotected free acid.

The reactions of process steps (1) and (2) are summarised in the following scheme:

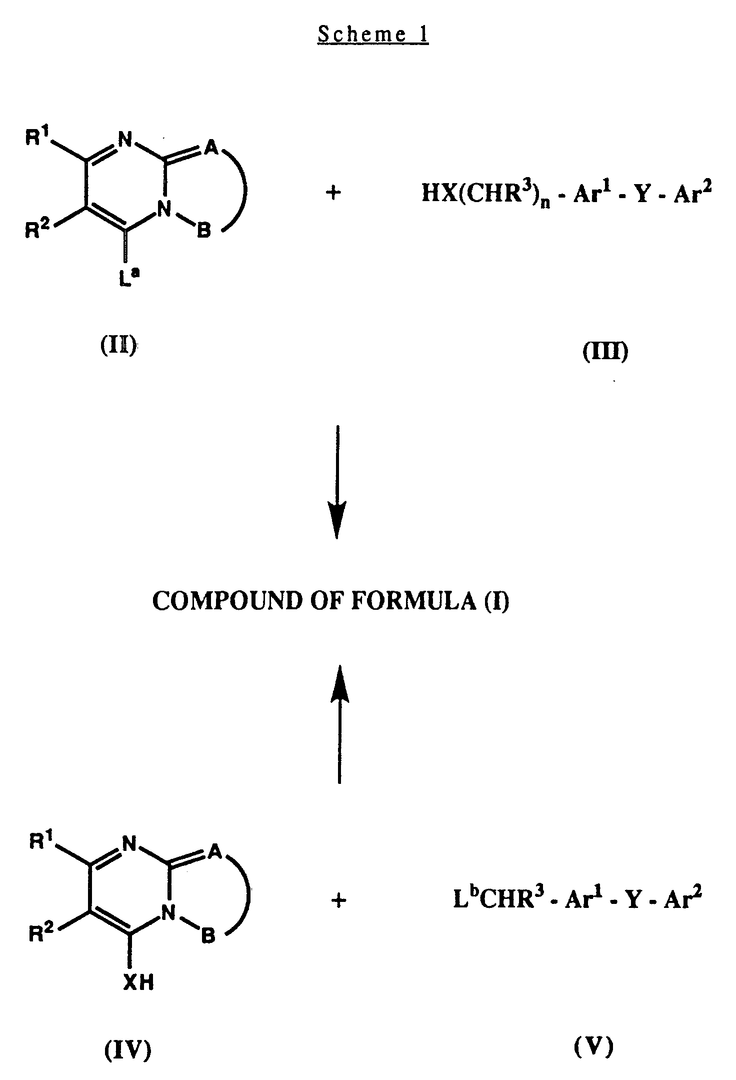

Compounds of formula I, in which R¹⁶ or R²⁰ are acidic substituents according to their general meaning, are prepared from appropriate precursors of formula I by standard methods. In particular, carboxylic acids (R¹⁶, R²⁰ = COOH) are prepared from their corresponding esters or other derivatives, in which the carboxylic acid is protected, or from the corresponding nitriles (R¹⁶, R²⁰ = COO(C₁₋₄-alkyl), COOP¹, or cyano). Tetrazoles (R¹⁶, R²⁰ = 5-tetrazolyl) are prepared from corresponding nitriles or protected tetrazoles, in which R¹⁶ or R²⁰ is cyano or a group of formula (M), sulfonic acids (R¹⁶ =SO₃H) from their esters (R¹⁶ = SO₂(OC₁₋₄-alkyl)), their chlorides (R¹⁶ = SO₂Cl), or their thiols (R¹⁶ = mercapto), phosphonic acids (R¹⁶ = PO(OH)₂) from their esters (R¹⁶ = PO(OC₁₋₄-alkyl)₂), trifluoromethanesulfonamides (R¹⁶ = NHSO₂CF₃) from the corresponding nitro via the amino derivatives (R¹⁶ = NO₂ or NH₂). These processes may also be carried out in an earlier stage of the synthesis of compounds of formula I during the preparation of compounds of formula III or formula V (Scheme 1), however, tetrazoles (R¹⁶, R²⁰ = 5-tetrazolyl) or carboxylic acids (R¹⁶, R²⁰ = COOH) of formula I are preferably prepared in this stage of the sequence. It is obvious to those skilled in the art, that substituents R², R⁵, R⁷, R⁹, R¹⁰, or R¹⁴ in compounds of formula I having the same meaning are or may also be transferred to acidic substituents of the meaning of R¹⁶ or R²⁰ during the described processes.

Esters of formula I (R¹⁶, R²⁰ = COO(C₁₋₄-alkyl)) are cleaved to their carboxylic acids (R¹⁶, R²⁰ = COOH) using standard methods (R. Sustmann, H.G. Korth, in Houben-Weyl, "Methoden der Organischen Chemie", Vol. E 5, p. 223 ff.; T.W. Greene, P.G.M. Wuts, "Protective Groups in Organic Synthesis", 2nd Ed., p. 227 ff.). Preferred methods use alkali hydroxides, particularly sodium or potassium hydroxide, in water or mixtures of water and alcohols at room temperature or at elevated temperatures up to the boiling point of the mixture. Preferred alcohols contain the same C₁₋₄-alkyl as the ester substituent R¹⁶ or R²⁰. tert.-Butyl esters (R¹⁶, R²⁰ = COOC(CH₃)₃) may be cleaved by using acidic conditions of the reaction, preferred by using trifluoroacetic acid in solvents such as dichloromethane or chloroform. The conversion of nitriles of formula I (R¹⁶, R²⁰ = cyano) to carboxylic acids I (R¹⁶, R²⁰ = COOH) requires heating of the nitriles in alkali hydroxide solutions.

Compounds of formula I, in which one of the substituents, in particular the substituents R¹⁶ or R²⁰, is a protected carboxylic acid group COOP¹, are prepared from the corresponding acids by known methods. In preferred methods the acid is alkylated in the presence of a base by an appropriate alkylating agent P¹ Hal, in which the halogen preferably is chlorine, bromine, or iodine. Examples for such alkylating agents are (chloromethyl)methylether, (chloromethyl)(2-methoxyethyl)ether, phenacyl bromide, cyclohexyl 2-chloropropionate, ethyl 2-chloropropionate, 1-chloro-1-(ethoxycarbonyloxy)ethane, 1-chloro-1-(isopropoxycarbonyloxy)ethane, 1-chloro-1-(cyclohexyloxycarbonyloxy)ethane, chloro(ethoxycarbonyloxy) methane, chloro(isopropoxycarbonyloxy)methane, chloro(cyclohexyloxycarbonyloxy)methane, chloromethyl pivaloate, chloromethyl propionate, chloromethyl acetate, 3-phthalidyl bromide, 4-bromomethyl-5-methyl-2-oxo-1,3-dioxolene. The reaction is carried out in solvents such as acetone, butanone, acetonitrile, DMSO, DMF, or N,N-dimethyl acetamide, preferably at room temperature, in the presence of bases such as potassium carbonate, sodium carbonate, sodium hydride, sodium hydroxide, potassium t-butoxide. In a particular preferred method potassium carbonate in DMF or N,N-dimethyl acetamide is used.

Tetrazoles of the formula I, in which R¹⁶ or R²⁰ is 5-tetrazolyl, may be prepared from nitriles of the formula I (R¹⁶, R²⁰ = CN) or carboxylic acids of the formula I (R¹⁶, R²⁰ = COOH). The conversion of the nitriles may be carried out directly using inorganic azides, or indirectly by preparation of an intermediate compound I, in which R¹⁶ or R²⁰ is a protected tetrazole of the formula (M), and subsequent removal of the protecting group P². Those intermediates may be isolated during the process or transferred to the unprotected tetrazole without their isolation. It may be advantageous to convert compounds containing a tetrazole protecting group to compounds with different protecting group P² during the process.

Preferred methods for the direct conversion to tetrazoles of the formula I use sodium azide in polar solvents such as DMF, N-methylpyrrolidinone, 1,3-dimethylimidazolidin-2-one, DMSO, diethylsulfoxide at temperatures between 100 °C and 160 °C and are usually catalyzed by ammonium chlorides. Particularly preferred methods are heating of the nitriles with NaN₃ in the presents of NH₄Cl or LiCI at 120 °C to 140 °C (W.G. Finnegan, R.A. Henry, R. Lofquist, J. Am. Chem. Soc. **1958**, 80, 3908; G.F. Holland, J.N. Pereira, J. Med. Chem. **1967,** 10, 149), heating in the presence of Et₃NHCl in N-methylpyrrolidinone at 150 °C (P.R. Bernstein, E.P. Vacek, Synthesis **1987,** 1133), or heating of the nitrile with aluminum azide in boiling tetrahydrofuran (E.R. Wagner, J. Org. Chem. **1973**, 38, 2976).

Methods for the preparation of tetrazoles of the formula I, which run through an intermediate I containing a protected tetrazole of the formula (M), are known from the literature: J.V. Duncia, M.E. Pierce, J.B. Santella III, J. Org. Chem. **1991**, 56, 2395; Eur. Patent Appl. EP 291,969). Preferred methods are heating of a nitrile I (R¹⁶, R²⁰ = cyano) with a tri(C₁₋₄-alkyl)stannyl azide or triphenylstannyl azide, particularly with trimethylstannyl azide or tri(n-butyl)stannyl azide, in toluene, xylene, mesitylene, or in DMF. The stannyl protecting group is removed in an additional step using basic or acidic reaction conditions. Preferred are acidic conditions and include stirring in the presence of mineral acids such as hydrochloric acid in inert solvents such as methanol, ethanol, ether, or THF at room temperature or slight warming of the mixture. They include also stirring with saturated aqueous ammonium chloride solution or stirring with silicagel using the above mentioned solvents. Reactions leading to compounds containing the protective group P² = trimethylstannyl may be preferably carried out with preformed trimethylstannyl azide. Tri(n-butyl)stannyl azide preferably is prepared from tri(n-butyl)stannyl chloride and sodium azide, and used *in situ* for the conversion (International Patent Appl. WO 92/02508). These products are preferably not isolated and converted to the unprotected tetrazole or to compounds of the formula I containing the protective group P² = CPh₃, which is removed using basic or acidic conditions. Preferred conditions for this reaction use mineral acids such as hydrochloric acid in solvents such as methanol, ethanol, THF, or dioxane, or aqueous acetic acid, which may contain additional amounts of the above mentioned organic solvents. The reaction is carried out at room temperature, however, heating of the mixture may be necessary in some cases. The protective group P² = CPh₃ is preferably introduced in an earlier stage of the sequence leading to corresponding compounds of the formula III or V, and finally is removed from the corresponding compounds of the formula I. Preferred methods for its introduction use triphenylmethyl chloride and amines as base such as triethylamine or pyridine in solvents such as dichloromethane or chloroform.

The other mentioned protective groups P² of the present invention are also preferably introduced into an earlier intermediate of the sequence. Corresponding compounds, in which P² is tert.-butyl, benzyl, 4-methylbenzyl, 4-methoxybenzyl, 4-nitrobenzyl, 2,4,6-trimethylbenzyl, 2-cyanoethyl, or 4-nitrophenyl are prepared from corresponding carboxylic acids by known methods. The sequence starts with the preparation of corresponding primary amides by standard methods, which are chlorinated to iminoyl chlorides, preferred by using PCl₅, POCl₃, or SOCl₂. These chlorides react with azides, preferred with sodium, trimethylsilyl, or trimethylstannyl azide, in the presence of a base such as triethylamine to the desired protected tetrazoles. Benzyl protective groups P² are preferably removed by hydrogenation using standard methods in ethanol, methanol, or ethyl acetate as solvents, which may contain additional amounts of acetic acid, and Raney-Ni or Pd on charcoal as common catalysts. The group 2-cyanoethyl is preferably removed by aqueous sodium hydroxide solution, which may contain an additional amount of an organic solvent such as methanol, ethanol, THF, or dioxane. The protective group P² = 4-nitrophenyl is removed by stirring with an alkali metal alkanethiolate or an alkali metal alkanolate, preferred with sodium or potassium methoxide, sodium or potassium ethoxide, or sodium or potassium propanethiolate in polar solvents such as DMF or N-methylprrolidinone at room temperature (Eur. Patent Appl. EP 495,626). The above mentioned tetrazole protective groups P² = C₁₋₄-alkoxymethyl, methylthiomethyl, 2-(trimethylsilyl)ethyl, benzenesulfonyl, and 4-methylbenzenesulfonyl are introduced and removed by standard procedures (T.W. Greene, P.G.M. Wuts, "Protective Groups in Organic Synthesis", 2nd Edition, John Wiley & Sons).

Preferred methods for the preparation of compounds, in which R¹⁶ or R²⁰ is 5-tetrazolyl, are
a) heating of nitriles with sodium azide in the presence of triethylammonium chloride in N-methylpyrrolidinone,
b) heating of nitriles with trimethylstannyl azide in toluene and acidic work-up of the mixture with hydrochloric acid, ammonium chloride, or silica gel,
c) heating of nitriles with tri(n-butyl)stannyl azide, which is prepared *in situ* from tri(n-butyl)stannyl chloride and sodium azide, in toluene, and acidic work-up with hydrochloric acid,
d) removal of the protecting group P² = CPh₃ by aqueous acetic acid, mixtures of aqueous HCl and THF or methanol, or solutions of sodium hydroxide in aqueous methanol at room temperature,
e) removal of the protecting group P² = 4-nitrophenyl by stirring with sodium propanethiolate, sodium methoxide, or sodium ethoxide in DMF or N-methylpyrrolidinone at room temperature.

Compounds of the formula I, in which R¹⁶ is NHSO₂CF₃, may be prepared from compounds of the formula I, in which R¹⁶ is nitro, by reduction to the corresponding primary aromatic amine and its subsequent conversion to the trifluoromethyl sulfonamide. The reduction is carried out by standard procedures (R. Hemmer, W. Lürken, in Houben-Weyl, "Methoden der Organischen Chemie", Vol. E 16 d, p. 815 ff.). Preferred methods use metals such as Zn, Sn, or Fe in acids such as aqueous HCl oder aqueous acetic acid, optionally with addition of an organic solvent such as methanol, ethanol or THF. Other preferred methods use metals in lower states of oxidation, e.g. Sn(II) dichloride in HCl or Ti(III) trichloride in acetone. Particularly preferred is the reduction by catalytic hydrogenation, which uses metal catalysts from metals such as Pd, Pt, or Ni, preferably Pd on charcoal or Raney-Ni, or their oxides such as PtO₂ in solvents such as methanol, ethanol, THF, or ethyl acetate, optionally with addition of an acid such as acetic acid. Those catalytic reductions may also be carried out with formic acid in the presence of an trialkylamine such as triethylamine or with ammonium formiate instead of elemental hydrogen (S. Ram, R.E. Ehrenkaufer, Synthesis **1988**, 91). The reaction of the primary amine I (R¹⁶ = NH₂) to the sulfonamide I (R¹⁶ = NHSO₂CF₃) is carried out with trifluoromethylsulfonyl chloride or preferably with trifluorosulfonic acid anhydride in a suitable inert solvent such as toluene, dichloromethane, or chloroform at temperatures between -78 °C and room temperature, preferably by mixing the reagents with cooling and warming to room temperature. The addition of an amine such as triethylamine, diisopropylethylamine, pyridine, 2,6-di-tert.butyl-4-methylpyridine, or 4-dimethylaminopyridine as above is preferred.

Sulfonic acids of the formula I (R¹⁶ = SO₃H) may be prepared from appropriate intermediates of the formula I by standard methods for the preparation of aromatic sulfonic acids, preferred from the corresponding sulfonyl chlorides I (R¹⁶ = SO₂Cl) (S. Pawlenko, in Houben-Weyl, "Methoden der Organischen Chemie", Vol. E 11/2, p. 1055 ff.; ibid., p. 1067 ff.). These are preferably prepared from aromatic amines I (R¹⁶ = NH₂), which in a first step are converted to their diazonium salts by standard methods and in a second step are treated with sulfur dioxide in the presence of Cu(II) chloride and acetic acid or with NaHSO₃ in the presence of Cu(II) sulfate and HCl (H. Meerwein, G. Dittmar, R. Göllner, K. Hafner, F. Mensch, O. Steinfort, Chem. Ber. **1957**, 90, 841; R.V. Hoffman, Org. Synth. **1981,** 60, 121). The sulfonyl chlorides are hydrolyzed to the sulfonic acids I (R¹⁶ = SO₃H) by heating in an aqueous solution, preferably in an alkaline aqueous solution, which contains alkali carbonates such as sodium carbonate or potassium carbonate, or alkali hydroxides such as sodium hydroxide or potassium hydroxide. The hydrolysis may be supported by forming of an intermediate ester I (R¹⁶ = SO₂(OC₁₋₄-alkyl)) of the sulfonic acid, preferably of a methyl or ethyl ester, which may also be prepared from corresponding intermediates of formula III or formula V. The conversion from the sulfonyl chlorides I is carried out by treating with an C₁₋₄-alkoxide or with the corresponding alcohol in pyridine, and the subsequent hydrolysis by the above mentioned conditions. Another preferred method for the preparation of a sulfonic acid of the formula I (R¹⁶ =SO₃H) is the oxidation of a thiol of the formula I (R¹⁶ = SH). This is preferably carried out by using elemental chlorine or sodium hypochlorite in an aqueous solution, by using potassium permanganate, or by using hydrogen peroxide in the presence of acids such as sulfuric acid or methylsulfonic acid, or in the presence of trifluoroacetic acid anhydride.

Phosphonic acids of formula I (R¹⁶ = PO(OH)₂), may be prepared by standard methods for the synthesis of aromatic phosphonic acids from appropriate intermediates (B. Gallenkamp, W. Hofer, B.-W. Krüger, F. Maurer, T. Pfister, in Houben-Weyl, "Methoden der Organischen Chemie", Vol. E 2, p. 300 ff.), preferably by hydrolysis of their esters I (R¹⁶ = PO(OC₁₋₄-alkyl)₂), particularly preferred by hydrolysis of their ethylesters I (R¹⁶ = PO(OEt)₂) (ibid., p. 310 ff.). The hydrolysis is preferably carried out using acidic conditions. Preferred acids, which may be used, are inorganic acids such as sulfuric acid, phosphoric acid, or dry hydrochloric acid, carboxylic acids such as formic acid or acetic acid, organic sulfonic acids such as methanesulfonic acid, trifluoromethanesulfonic acid, or 4-toluenesulfonic acid. The reaction is carried out in an inert solvent such as acetonitrile, optionally with addition of water, at temperatures between room temperature and warming of the mixture.

The synthesis of compounds of formula I is shown in Scheme 1. It starts from intermediates of formula III or formula V, respectively, which preferably contain non-acidic substituents R¹⁶ or R²⁰, in particular R¹⁶, R²⁰ = cyano or COO(C₁₋₄-alkyl), which finally are converted to the acidic substituents by the above described procedures. The alkylation starting from intermediates of formula V is preferred, if X is S. In the other cases the reaction with intermediates of formula III from Scheme 1 is preferred.

Leaving groups L^{a} in compounds of formula II are halo, OSi(C₁₋₄-alkyl)₃, OSO₂(C₁₋ 4-alkyl), OSO₂Ph, in which the phenyl group optionally may be substituted by methyl, chloro, or nitro. Preferred leaving groups L^{a} are chloro, bromo, OSO₂Me, OSO₂Ph, OSO₂(4-MeC₆H₄), OSiMe₃, or OSiMe₂tBu, particularly preferred chloro, OSO₂Me, or OSO₂(4-MeC₆H₄), most particularly chloro.

Examples of the reaction of azolofused pyrimidines of formula II, particularly of those in which L^{a} is chloro, with amines, thiols, alcohols, or phenols according to Scheme 1 are known. Compounds of formula II, in which the leaving group L^{a} is halo, alkyl- or arylsulfonate, react with amines of formula III in an inert solvent, optionally in the presence of an additional base, at temperatures between room temperature and reflux of the mixture. Inert solvents for this reaction are alcohols such as methanol, ethanol, isopropanol, n-butanol, tert-butanol, or ethyleneglycol, ethers such as diethylether, THF, 1,2-dimethoxyethane, aromatic hydrocarbons such as toluene, xylene, benzene, or chlorobenzene, chlorinated hydrocarbons such as chloroform, dichloromethane, or 1,2-dichloroethane, DMF, or acetonitrile, preferred solvents methanol, ethanol, isopropanol, or acetonitrile. As additional bases there may be used tertiary amines such as triethylamine, diisopropylethylamine, dimethylaniline, diethylaniline, or pyridine, inorganic hydrides such as sodium or potassium hydride, or inorganic carbonates such as sodium, potassium, or lithium carbonate. The most preferred bases are triethylamine, potassium or sodium carbonate. Preferred methods are heating in isopropanol, ethanol, or methanol, especially ethanol, optionally in the presence of at least one equivalent of triethylamine or potassium carbonate. Compounds of formula II, in which the leaving group L^{a} is a trialkylsilylether, react with amines of formula III by heating at 150 °C to 180 °C. This may be carried out in an inert solvent, which allows heating to these temperatures, or without any solvent, optionally in the presence of a weak acid such as ammonium sulfate or toluene-4-sulfonic acid. The reaction of compounds of formula II with thiols, alcohols, or phenols of formula III requires the addition of at least one equivalent of a strong base, e.g. an alkali hydroxide such as sodium, potassium, or lithium hydroxide, an alkali alkoxide, preferably a sodium or potassium alkoxide, particularly their methoxide, ethoxide, or isopropoxide, or an alkali hydride such as sodium or potassium hydride, and is carried out in the above mentioned inert solvents including water for those cases in which hydroxides are used, and including mixtures of water and organic solvents optionally in the presence of a phase transfer catalyst for biphasic mixtures. The reaction of thiols of formula III is preferably carried out with potassium or sodium methoxide or potassium or sodium ethoxide in methanol or ethanol, respectively, particularly preferred with sodium ethoxide in ethanol, the reaction of alcohols or phenols of formula III preferably with sodium hydride in DMF, both reactions being carried out at temperatures between room temperature and 120 °C.

The alkylation of compounds of formula IV, in which X is S, O, or NR⁴, with compounds of formula V leads to specific embodiments of formula I, wherein n = 1. Leaving groups L^{b} in compounds of formula V are chloro, bromo, OSO₂(C₁₋₄-alkyl), or OSO₂Ph, in which the phenyl group optionally may be substituted by methyl, chloro, or nitro. Preferred leaving groups L^{b} are chloro, bromo, OSO₂Me, OSO₂Ph, or OSO₂(4-MeC₆H₄), and bromo is particularly preferred. The reaction is carried out in inert solvents such as DMF, DMSO, acetonitrile, benzene, toluene, xylene, ether, THF, 1,2-dimethoxyethane, acetone or butanone at temperatures between room temperature and reflux of the mixture and usually requires the presence of at least one equivalent of a base such as pyridine, triethylamine, diisopropylethylamine, 4-dimethylaminopyridine, potassium carbonate, sodium carbonate, potassium hydride, sodium hydride, potassium hydroxide, or sodium hydroxide. In the case of the alkali hydroxides water may be used as solvent and the reaction may be carried out in a biphasic mixture with an organic solvent in the presence of a phase transfer catalyst. Preferred methods for the alkylation of compounds of formula IV are
a) refluxing in acetone or butanone in the presence of potassium carbonate, and
b) stirring at room temperature in DMF in the presence of sodium hydride as base. Method b) is particularly preferred.

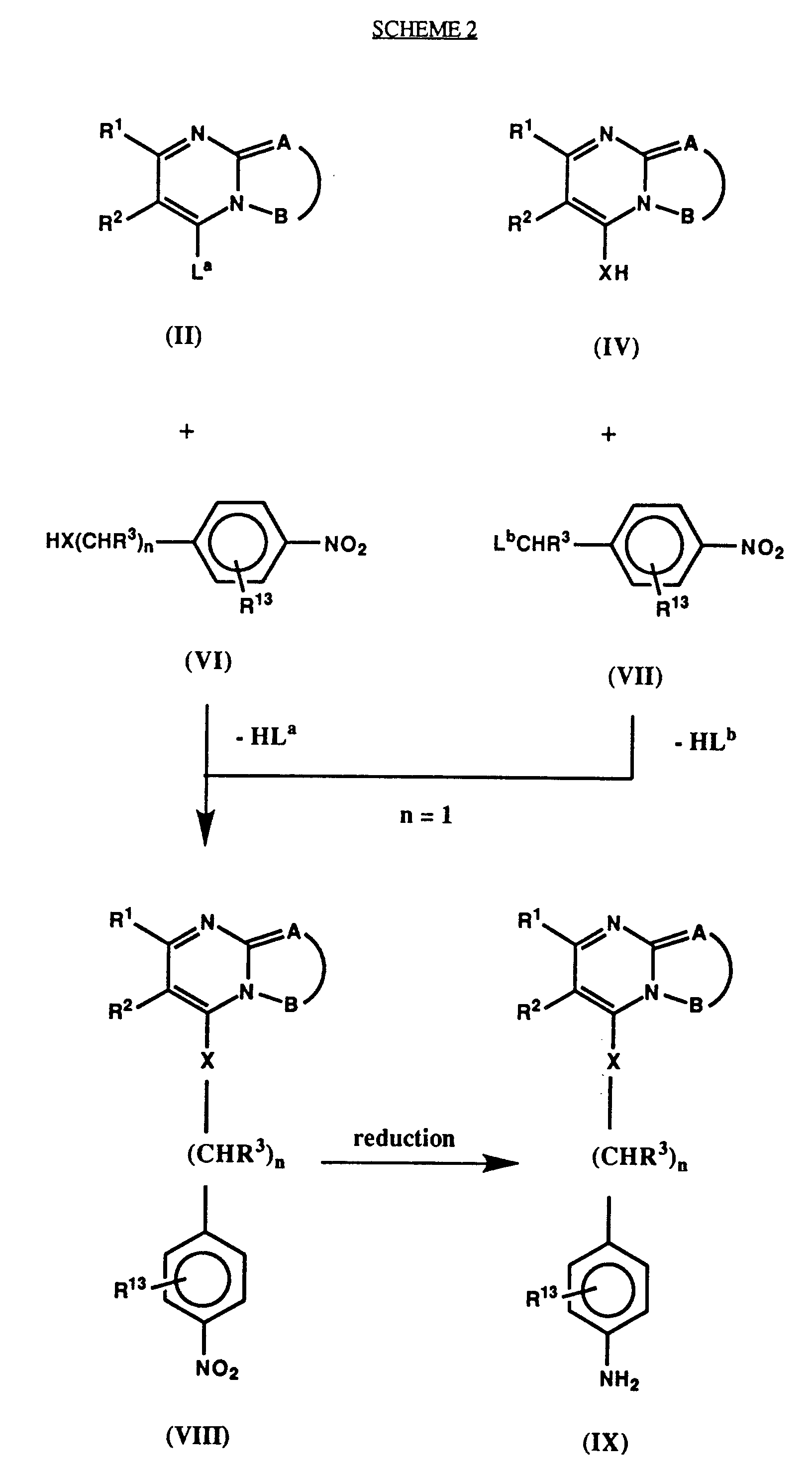

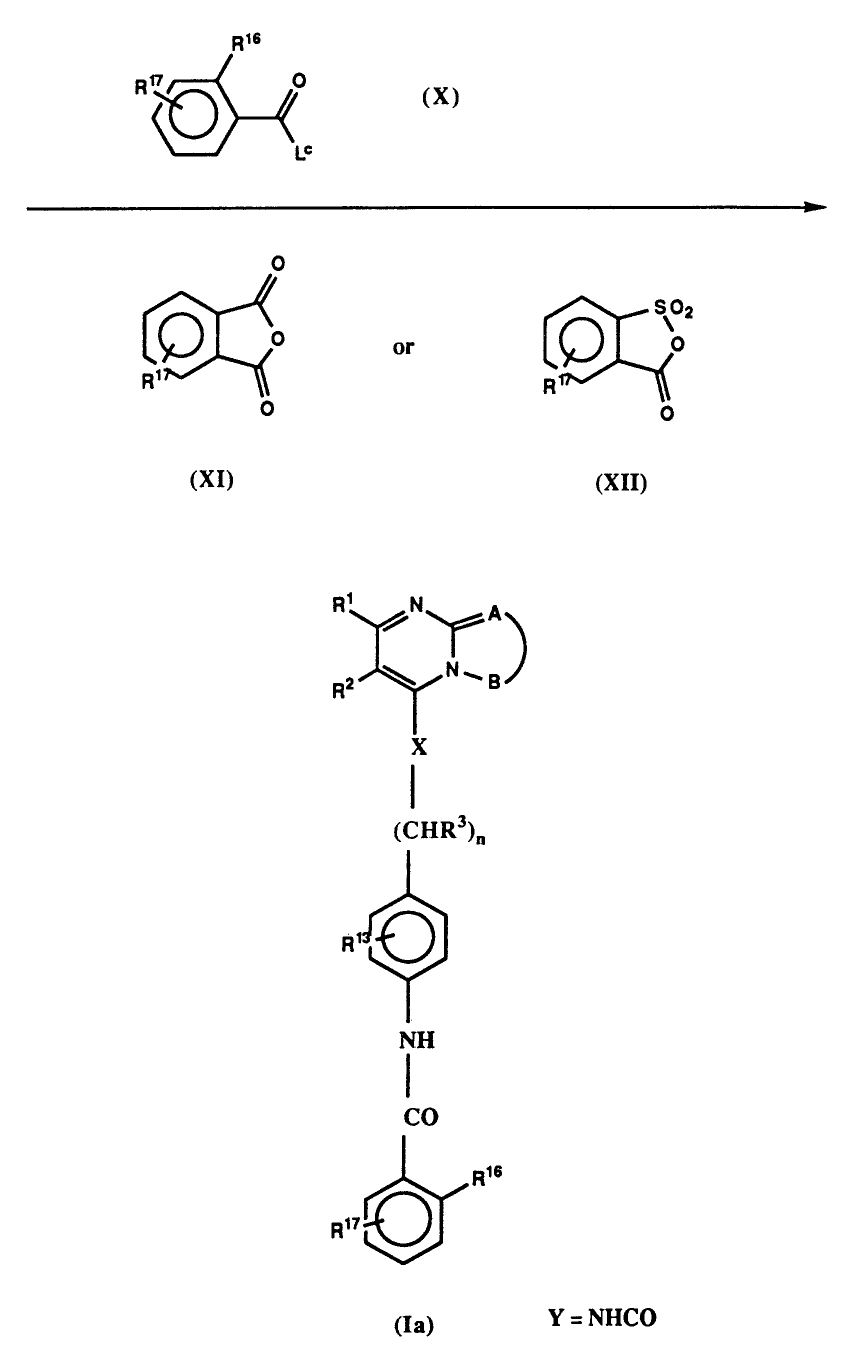

In Scheme 2 preferred methods for the synthesis of compounds of formula Ia, which is a specific embodiment of formula I, in which Y is NHCO, are shown. These methods are particularly preferred for compounds of formula Ia, in which R¹⁶ is COOH or SO₃H. The reaction of the intermediates II and IV with nitrobenzenes of formula VI or formula VII, respectively, which are known compounds or may be prepared by known methods, is carried out in the same manner as described above in Scheme 1. The nitro compounds of the formula VIII are reduced to the amino compounds of formula IX by standard methods (R. Hemmer, W. Lürken, in Houben-Weyl, "Methoden der Organischen Chemie", Vol. E 16 d, p. 815 ff.), which are also mentioned above. A preferred method is the reduction by catalytic hydrogenation, which uses Pd on charcoal as a catalyst in solvents such as methanol, ethanol, THF, or ethyl acetate, optionally with addition of an acid such as acetic acid. The amines of formula IX are converted to the amides of the formula Ia by reaction with carboxylic acids X (L^{c} = OH) or other derivatives X, in which L^{c} is a leaving group, using standard methods, preferably with carboxylic acid chlorides X (L^{c} = Cl) (D. Döpp, H. Döpp, in Houben-Weyl, "Methoden der Organischen Chemie", Vol. E 5, p. 941 ff.). The condensation of carboxylic acids X (L^{c} = OH) with amines of the formula IX is preferably carried out with a reagent for dehydration, particularly in the presence of N,N'-dicyclohexyl carbodiimide. Compounds of formula Ia, in which R¹⁶ is COOH or SO₃H, are preferably prepared by reaction of the amines of formula IX with phthalic acid anhydrides XI or with cyclic anhydrides of 2-sulfobenzoic acid XII, respectively (European Patent Appl. EP 253 310; J.V. Duncia, A.T. Chiu, D.J. Carini, G.B. Gregory, A.L. Johnson, W.A. Price, G.J. Wells, P.C. Wong, J.C. Calabrese, P.B.M.W.M. Timmermans, J. Med. Chem. **1990,** 33, 1312). The amine IX is stirred with an equimolar amount or a slight excess of the anhydrides XI or XII in an inert solvent such as diethylether, THF, ethyl acetate, DMF, benzene, toluene, methanol, ethanol, dichloromethane, chloroform, or acetonitrile, optionally in the presence of a base such as sodium hydride, sodium or potassium acetate, sodium or potassium carbonate, pyridine, triethylamine, or ethyldiisopropylamine at room temperature or warming of the mixture. If insoluble inorganic bases are used, it may be advantageous to add a quarternary ammonium salt such as tetrabutyl ammonium chloride, trioctylmethylammonium chloride, or triethylbenzylammonium chloride as a phase transfer catalyst. In preferred methods both compounds are stirred in dichloromethane at room temperature or heated in the presence of potassium carbonate / triethylbenzylammonium chloride.

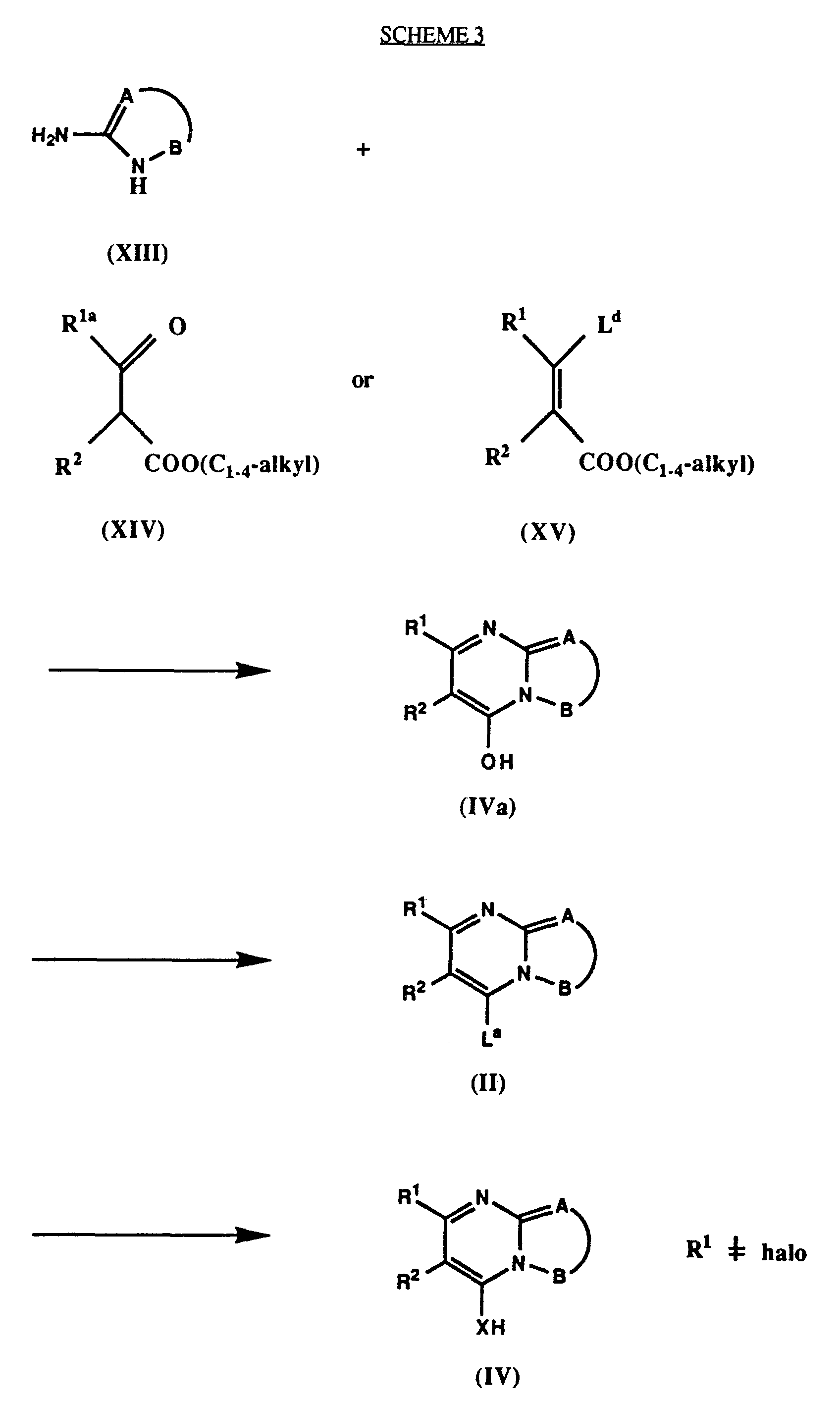

In Scheme 3 methods for the synthesis of the intermediate azolofused pyrimidines II and IV are shown. They start from aminoazoles of the formula XIII, which react with 3-oxoesters of the formula XIV or derivatives XV to fused hydroxypyrimidines of the formula IVa. 3-Oxoesters XIV are used for the synthesis of compounds of formula IVa, in which R¹ has the meaning of R^{1a}. Compounds of formula IVa, in which R¹ has the meaning of R^{1b}, may be prepared from compounds of formula XV, in which R¹ is R^{1b}, particularly in those cases, in which R^{1b} is an amino- or a thiosubstituent according to its general meaning, and if L^{d} is a thiosubstituent. In general, the leaving group L^{d} in compounds of the formula XV may have the meaning of R^{1b} including NH₂. If R¹ is R^{1a}, preferred leaving groups L^{d} are methoxy, ethoxy, amino, methylamino, dimethylamino, anilino, pyrrolidino, piperidino, morpholino, methylthio, or benzylthio, particularly preferred methoxy or ethoxy. If R¹ is a thiosubstituent R^{1b}, the leaving group L^{d} has preferably the same meaning as R^{1b}. If R¹ is an amino substituent R^{1b}, L^{d} is preferably S(C₁₋₄-alkyl) or SCH₂Ph, particularly preferred SMe or SCH₂Ph.

Many examples for the synthesis of fused hydroxypyrimidines IVa and their conversion to derivatives II and IV are known: W.L. Mosby, in "Heterocyclic Systems with Bridgehead Nitrogen Atoms", ed. by A. Weissberger, Interscience Publishers, New York, 1961; J.V. Greenhill, p. 305 ff. (pyrazolopyrimidines), S.W. Schneller, p. 890-891 (triazolopyrimidines), and J.A. Montgomery, J.A. Secrist III, p. 647 ff. (imidazopyrimidines), in "Comprehensive Heterocyclic Chemistry", Vol. 5, Pergamon Press, 1984; M.H. Elnagdi, M.R.H. Elmoghayar, G.E.H. Elgemeie, Adv. Heterocyclic Chem. **1987,** 41, 319, in particular: C.F.H. Allen, H.R. Beilfuss, D.M. Burness, G.A. Reynolds, J.F. Tinker, J.A. van Allan, J. Org. Chem. **1959,** 24, 779, 787; C.F.H. Allen, G.A. Reynolds, J.F. Tinker, L.A. Williams, J. Org. Chem. **1960**, 25, 361; Y. Makisumi, Chem. Pharm. Bull. **1962**, 10, 612, 620; **1964**, 12, 204; W. Ried, K.-P. Peuchert, Liebigs Ann. Chem. **1962**, 660, 104; W. Ried, S. Aboul-Fetouh, Chem. Ztg. **1989**, 113, 181; W.A. Kleschick, J. Bordner, J. Heterocyclic Chem. **1989**, 26, 1489; K. Esses-Reiter, J. Reiter, J. Heterocyclic Chem. **1987**, 24., 1503; J. Reiter, L. Pongo, P. Dvortsak, Tetrahedron **1987**, 43, 2497; M.H. Elnagdi, E.M. Kandeel, E.M. Zayed, Z.E. Kandil, J. Heterocyclic Chem. **1977**, 14, 155; R.K. Robins, G.R. Revankar, D.E. O'Brien, R.H. Springer, T. Novinson, A. Albert, K. Senga, J.P. Miller, D.G. Streeter, J. Heterocyclic Chem. **1985**, 22, 601; R.H. Springer, M.B. Scholten, D.E. O'Brien, T. Novinson, J.P. Miller, R.K. Robins, J. Med. Chem. **1982**, 25, 235; K. Senga, T. Novinson, R.H. Springer, R.P. Rao, D.E. O'Brien, R.K. Robins, H.R. Wilson, J. Med. Chem. **1975**, 18, 312; K. Senga, T. Novinson, H.R. Wilson, R.K. Robins, J. Med. Chem. **1981,** 24, 610; T. Novinson, R.K. Robins, T.R. Matthews, J. Med. Chem. **1977**, 20, 296; T. Novinson, B. Bhooshan, T. Okabe, G.R. Revankar, R.K. Robins, K. Senga, H.R. Wilson, J. Med. Chem. **1976**, 19, 512; T. Novinson, D.E. O'Brien, R.K. Robins, J. Heterocyclic Chem. **1974**, 11, 873; T. Pyl, W. Baufeld, Liebigs Ann. Chem. **1966**, 699, 127; Ger. Offen. DE 3 338 292; European Patent Appl. EP 500 137. In particular, the reaction of aminoazoles XIII with ketene-S,S-acetals of formula XV is known: A. Thomas, M. Chakraborty, H. Ila, H. Junjappa, Tetrahedron **1990**, 46, 577; T. Eisenächer, R. Pech, R. Böhm, Pharmazie **1992**, 47, 580.

The reaction of aminoazoles XIII with the compounds XIV or XV may be carried out under acidic or basic conditions, or, if the esters XIV or XV are liquids and these may be used as a solvent, by heating a mixture of both compounds at temparatures between 120 °C and 200 °C, optionally in the presence of an additional inert higher boiling cosolvent. Acidic conditions use heating in carboxylic acids such as acetic acid, or heating in the presence of Lewis acids such as BF₃, ZnCl₂, AlCl₃, or TiCl₄ in an inert solvent, preferably in an alcohol containing the same alkyl chain as the esters XIV or XV. It is particularly preferred to use ethanol with ethyl esters XIV or XV. In methods using basic reaction conditions both compounds may be warmed in DMF in the presence of sodium hydride or in the presence of an alkali carbonate such as sodium or potassium carbonate, or may be stirred in aqueous sodium or potassium hydroxide or sodium or potassium carbonate solution containing between 5 % and 10 % of the base at temperatures between room temperature and 100 °C. Preferred basic conditions use sodium or potassium alkoxides in alcohols, which contain the same alkyl chain. Particularly preferred is sodium ethoxide in ethanol and ethyl esters XIV or XV at reflux of the mixture. The reaction of aminoazoles XIII and esters of the formula XIV or formula XV is most preferably carried out by heating in acetic acid.

Fused hydroxypyrimidines IVa are converted to fused halopyrimidines II (L^{a} = halo) by mineral acid halides, preferably to their chloro or bromo derivatives by POCl₃, PCl₅, SOCl₂, or POBr₃, respectively, most preferably by heating in POCl₃ at temperatures between 60 °C and 120 °C, preferably at reflux, and optionally with addition of an inert solvent such as benzene, toluene, xylene, chlorobenzene, chloroform, or 1,2-dichloroethane. The chlorination may be carried out in the presence of a tertiary amine or amide such as pyridine, N,N-dimethylaniline, N,N-diethylaniline, DMF, or N,N,N',N'-tetramethyl urea. A most preferred method uses heating in POCl₃ in the presence of N,N-diethylaniline. Compounds of formula II, in which L^{a} is OSO₂(C₁₋₄-alkyl) or (substituted) OSO₂Ph are prepared from compounds of formula IVa and the corresponding sulfonyl chlorides. The reaction is carried out in the presence of a base such as pyridine, 4-(N,N-dimethylamino) pyridine, potassium carbonate, sodium or potassium hydroxide in inert organic solvents such as chloroform, dichloromethane, toluene, diethylether, or THF. In the case of insoluble inorganic bases a quarternary ammonium salt such as tetrabutylammonium chloride, trioctylmethylammonium chloride, triethylbenzylammonium chloride is added. Compounds of formula II, in which L^{a} is OSi(C₁₋₄-alkyl)₃, in particular OSiMe₃, are preferably not isolated during the course of the reaction. The silylation of the fused hydroxypyrimidines IVa is carried out by heating at temperatures between 120 °C and 160 °C with an excess of reagents such as hexamethyldisilazane, N,O-bis(trimethylsilyl) acetamide, N,O-bis(trimethylsilyl) trifluoroacetamide, N,N'-bis(trimethylsilyl) urea, or N,O'-bis(trimethylsilyl) carbamate, which may be used as a solvent. The silylation with trimethylsilyl chloride, or trimethylsilyl bromide is carried out in the presence of a base such as pyridine, triethylamine, 4-(N,N-dimethylamino)pyridine in inert solvents such as chloroform, dichloromethane, or toluene.

Compounds of formula IV, in which X is S or NR⁴, are prepared from compounds of formula II. Preferred compounds II for this conversion are fused chloropyrimidines II (L^{a} = Cl). The reaction with amines R⁴NH₂ is carried out according to the methods described in Scheme 1. Fused aminopyrimidines IV (X = NH) are prepared with ammonia in an inert solvent, preferably in an alcohol such as methanol, ethanol, or isopropanol. In a preferred modification this reaction is carried out in ethanol by heating in an autoclave. Fused mercaptopyrimidines IV (X = S) are preferably prepared by reaction of compounds II (L^{a} = Cl) with thiourea. Preferred solvents for this reaction are alcohols such as methanol, ethanol, propanol, isopropanol, or butanol, particularly preferred ethanol, and the reaction is preferably carried out by heating at reflux. For this conversion sodium sulfide in water, alcohols, or mixtures thereof, or hydrogen sulfide in basic alcoholic solution, e.g. in methanol or ethanol containing sodium, potassium, or ammonium carbonate may also be used.

The aminoazoles XIII are known compounds or may be prepared by known methods: J. Elguero, p. 273 ff. (aminopyrazoles), M.R. Grimmett, p. 457 ff. (aminoimidazoles), and J.B. Polya, p. 761 ff. (aminotriazoles), in "Comprehensive Heterocyclic Chemistry", Vol. 5, Pergamon Press, 1984; M.H. Elnagdi, F.M. Abdel-Galil, B.Y. Riad, G.E.H. Elgemeie, Heterocycles **1983**, 20, 2437; M.H. Elnagdi, M.R.H. Elmoghayar, G.E.H. Elgemeie, Synthesis **1984,** 1; M.R. Grimmett, Adv. Heterocyclic Chem. **1980**, 27, 241; C. Temple, J.A. Montgomery, in "The Chemistry of Heterocyclic Compounds", ed. by A. Weissberger, E.C. Taylor, Vol. 37 (1,2,4-Triazoles), Wiley-Interscience, New York, 1981.

The compounds of formula XIV and formula XV are also known, or they may be prepared by known procedures, e.g. 3-oxoesters XIV according to W. Wierenga, H.I. Skulnick, J. Org. Chem. **1979**, 44, 310; ketene-S,S- or ketene-S,N-acetals of formula XV according to methods from E. Schaumann, in Houben-Weyl, "Methoden der Organischen Chemie", Vol. E 11, p. 260 ff., p. 325 ff., or M. Kolb, Synthesis **1990**, 171.

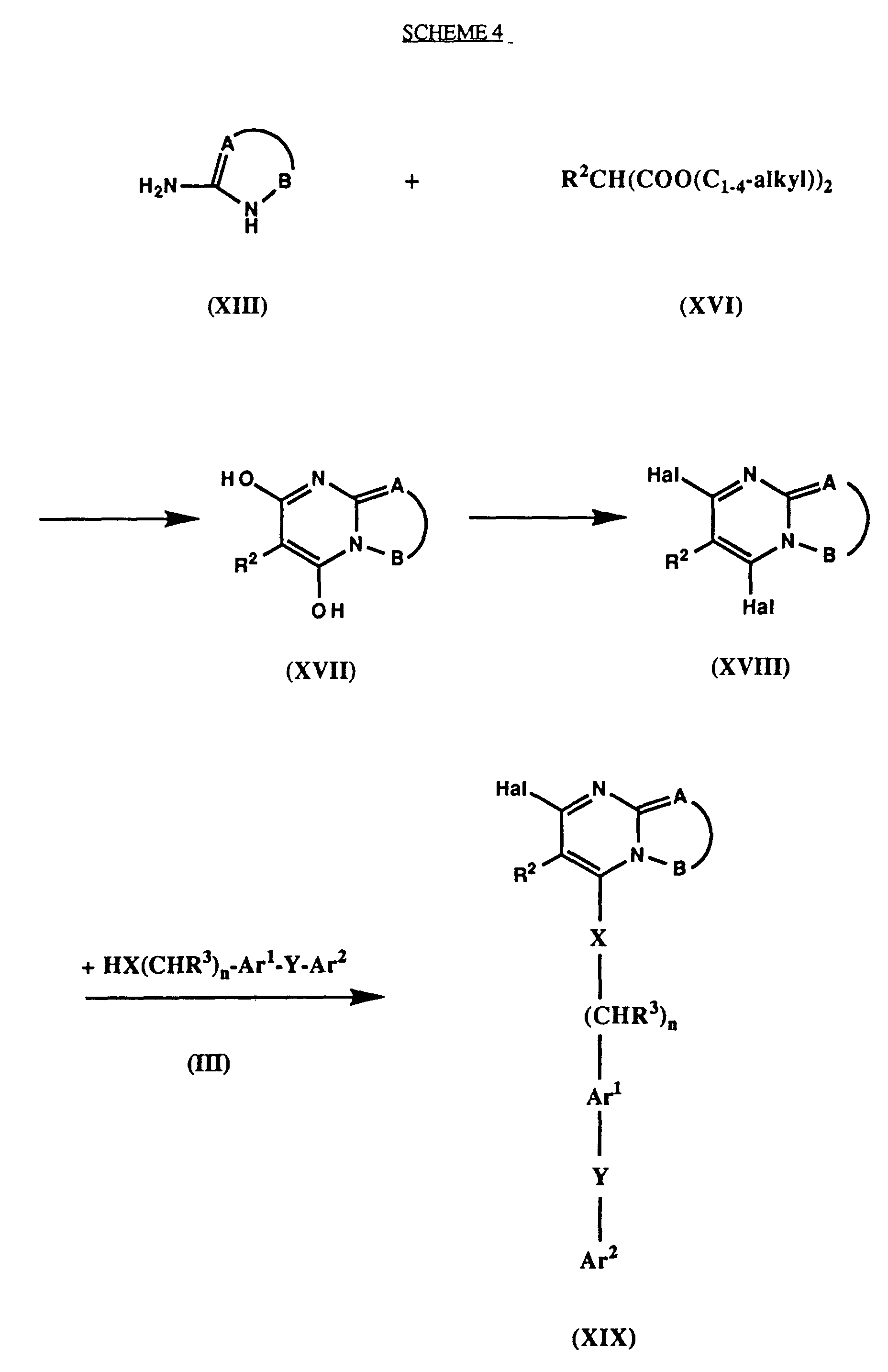

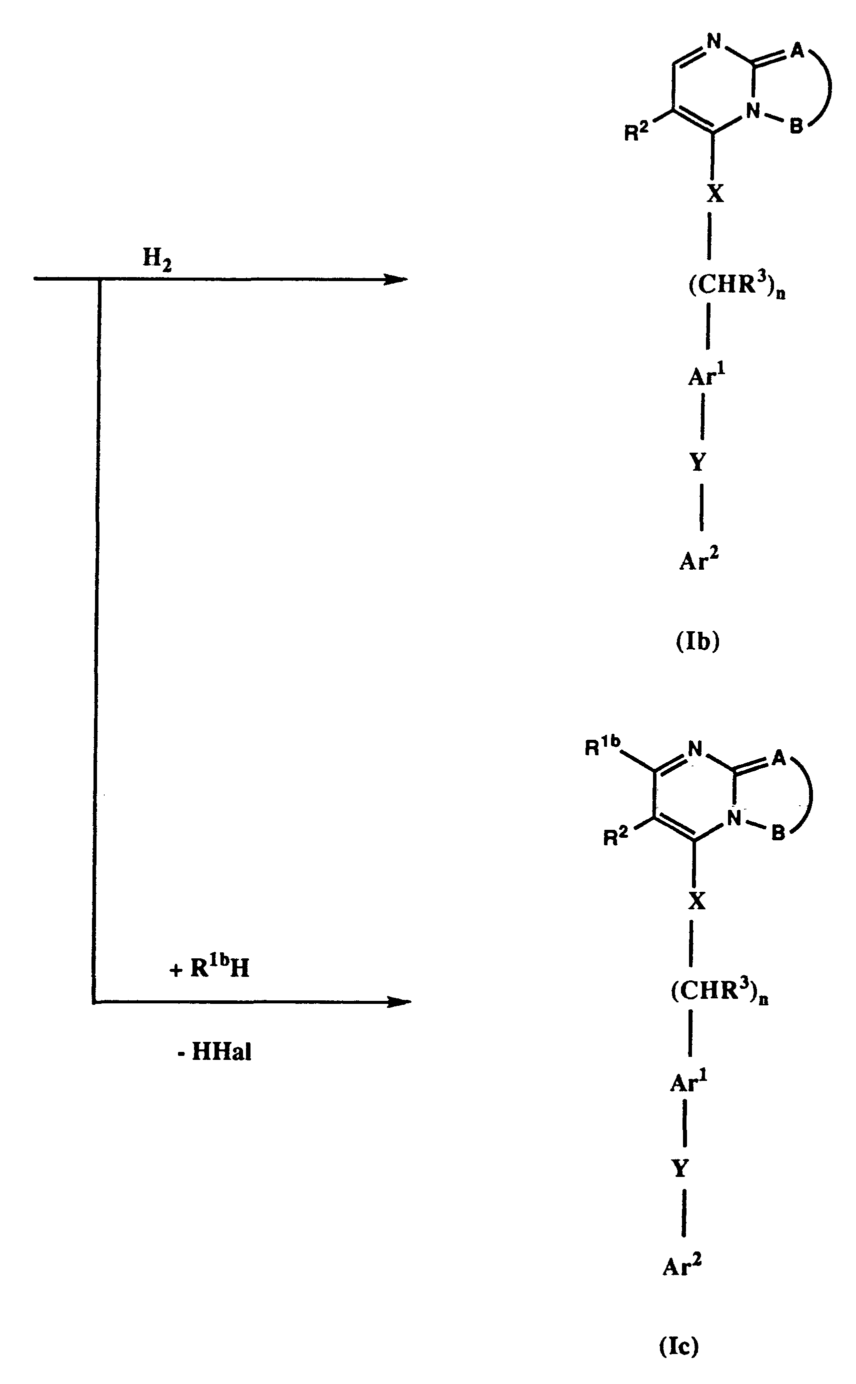

Preferred methods for the synthesis of compounds of formula Ib or formula Ic, which are special embodiments of formula I, in which R¹ is hydrogen or the substituent R^{1b}, respectively, are shown in Scheme 4. Aminoazoles of formula XIII react with malonic esters XVI to give fused dihydroxypyrimidines XVII, which are halogenated to fused dihalogenopyrimidines XVIII, preferably to fused dichloropyrimidines XVIII (Hal = Cl). Reaction with compounds of formula III leads to compounds of formula XIX, whose remaining halogen atom may be removed by hydrogenation leading to compounds of formula Ib or may be substituted by alcohols, phenols, amines, or thiols R^{1b}H leading to compounds of formula Ic. Examples for the reactions of Scheme 4 are known from the above cited general literature on fused hydroxypyrimidines, in particular: R.H. Springer, M.K. Dimmitt, T. Novinson, D.E. O'Brien, R.K. Robins, L.N. Simon, J.P. Miller, J. Med. Chem. **1976,** 19, 291; G.R. Revankar, R.K. Robins, R.L. Tolman, J. Org. Chem. **1974,** 39, 1256; T. Novinson, R.K. Robins, T.R. Matthews, J. Med. Chem. **1977,** 20, 296; R.K. Robins, G.R. Revankar, D.E. O'Brien, R.H. Springer, T. Novinson, A. Albert, K. Senga, J.P. Miller, D.G. Streeter, J. Heterocyclic Chem. **1985**, 22, 601; Y. Makisumi, Chem. Pharm. Bull. **1962**, 10, 612; E. Tenor, R. Ludwig, Pharmazie **1971**, 26, 534; E. Lippmann, P. Strauch, E. Tenor, Pharmazie **1991**, 46, 184.

The reaction of aminoazoles XIII with malonic esters XVI, which are known compounds or may be prepared by known methods, is preferably carried out by using basic conditions. These are described for the similar conversion of 3-oxoesters of formula XIV in Scheme 3. Preferred basic conditions use sodium or potassium alkoxides in alcohols, which contain the same alkyl chain. Particularly preferred is the use of sodium ethoxide in ethanol and ethylesters XVI at reflux of the mixture. The subsequent halogenation to dihalogenides of formula XVIII is also carried out by the methods from Scheme 3, preferably by using POCl₃, optionally in the presence of tertiary amines or amides, most particularly preferred by refluxing in POCl₃ in the presence of N,N-diethylaniline, leading to dichlorides of formula XVIII (Hal = Cl). The substitution of one halo in compounds of formula XVIII with amines, thiols, alcohols, or phenols of formula III is carried out by methods described in Scheme 1, e.g. by heating of amines of formula III with dichlorides XVIII in alcohols such as methanol, ethanol, propanol, isopropanol, n-butanol, or tert-butanol, preferably in ethanol. It may be advantageous to carry out this reaction at lower temperatures than reflux, e.g. at room temperature or by slight warming of the mixture. The remaining halo in compounds of formula XIX is removed by catalytic hydrogenation, using metal catalysts derived from metals such as Pd, Pt, or Ni, preferably Pd on charcoal or Raney-Ni, or their oxides such as PtO₂ in solvents such as methanol, ethanol, THF, or ethyl acetate, optionally with addition of acetic acid and / or sodium acetate. In preferred methods 5 % or 10 % Pd on charcoal in a mixture of acetic acid and methanol or ethanol at room temperature is used. The reaction of compounds of formula XIX with amines, alcohols, phenols, or thiols R^{1b}H is carried out by methods, which are similar to the substitution of the first halogen atom, however, requires more drastic conditions such as heating in higher boiling solvents and / or under elevated pressure. A preferred method for the substitution with amines R^{1b}H is heating of a chloro derivative XIX (Hal =Cl) with an excess of the amine in solvents such as methanol, ethanol, isopropanol, DMF, acetonitrile, or toluene in an autoclave. Alcohols R^{1b}H react preferably with derivatives of formula XIX by heating with their sodium or potassium alkoxides, which are dissolved in these alcohols, and the reaction is preferably carried out in an autoclave. A preferred process for the reaction with phenols or thiols R^{1b}H involves in a first step the generation of phenolates or thiolates with bases such as sodium hydride in solvents such as DMF and in a second step heating of these reagents with compounds of formula XIX, optionally carried out in an autoclave. The introduction of an amino, alkoxy, or phenoxy substituent R^{1b} may also be carried out using the described conditions by replacement of a substituent R^{1b} = alkylthio, preferably by replacement of methylthio, optionally after oxidation to the corresponding sulfone.

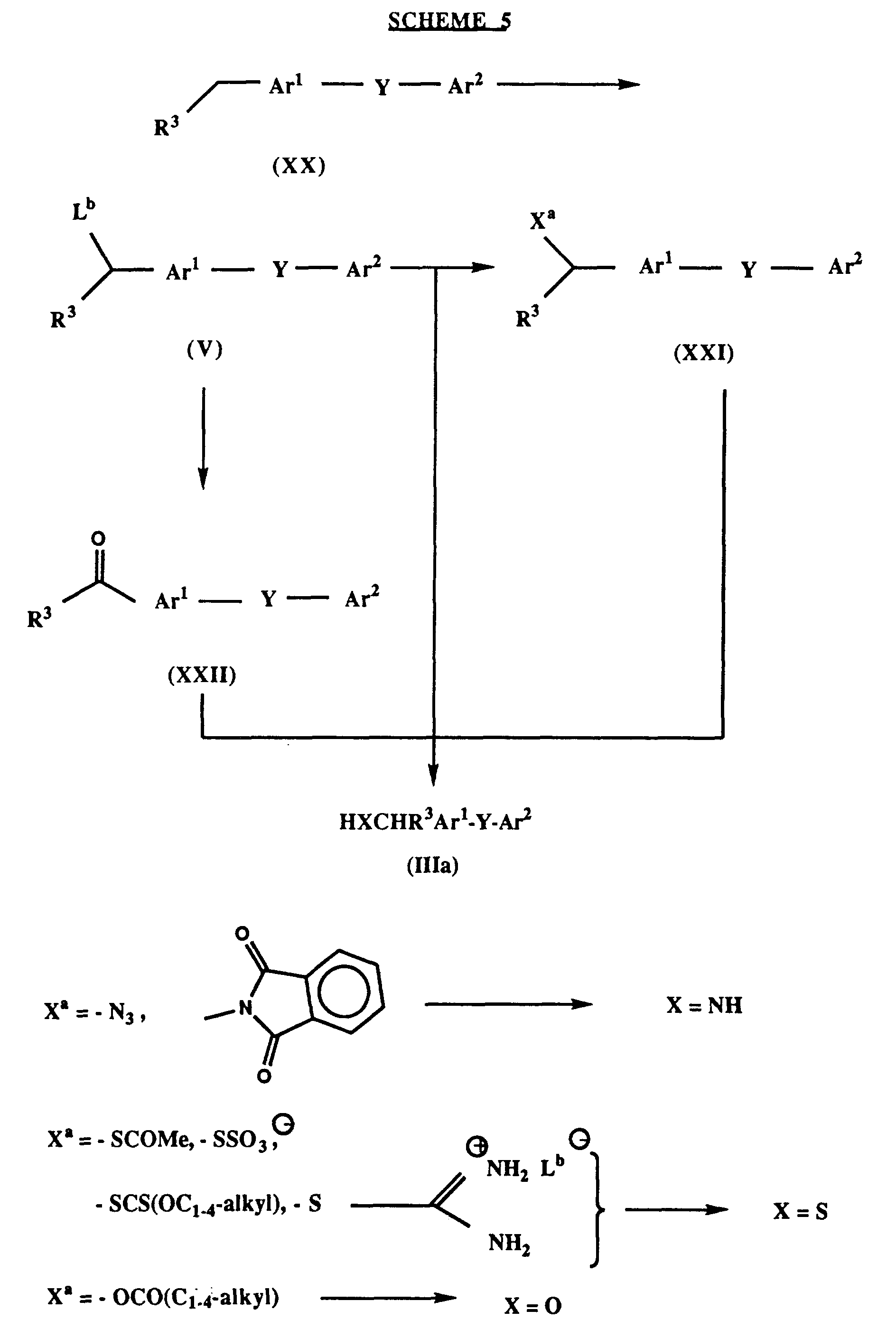

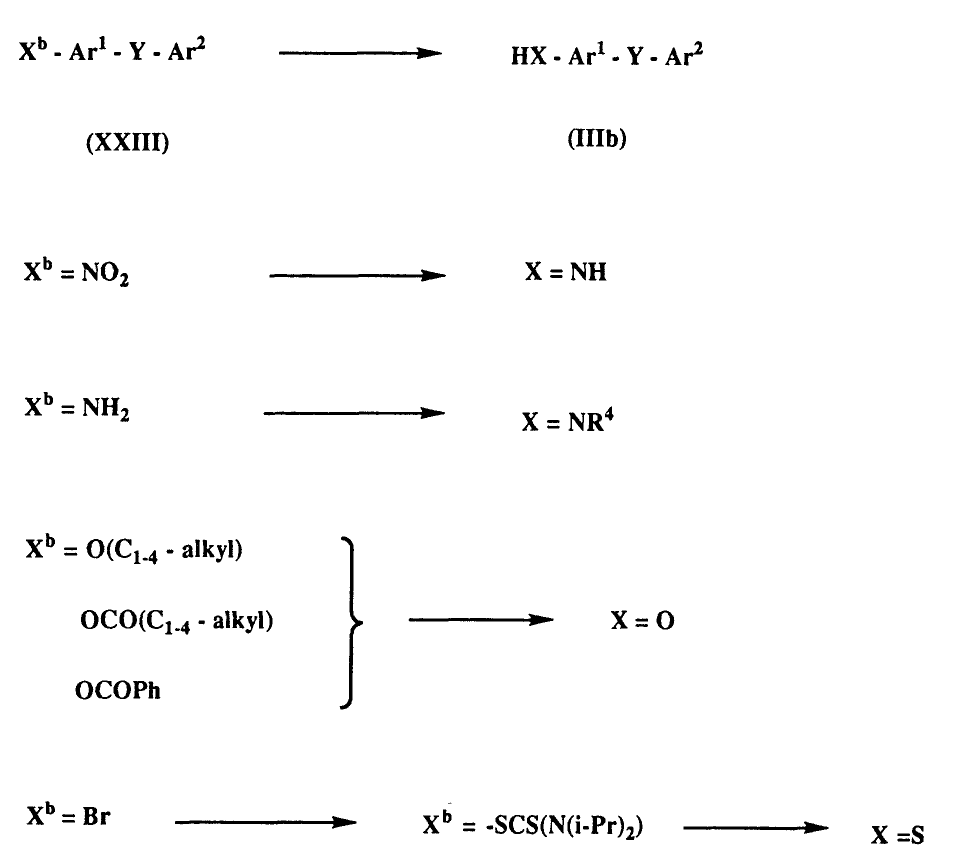

Preferred methods for the synthesis of compounds of formula III or formula V from Scheme 1 and from Scheme 4 are summerized in Scheme 5. These compounds are known or may be prepared by known methods. In particular, compounds of formula XX and of formula V (L^{b} = Br) are known, e.g. those, in which Ar¹ and Ar² are phenyl rings of formula (E) or formula (I) (European Patent Appl. EP 253 310; EP 291 969; EP 323 841; EP 324 377; EP 400 835; EP 400 974; EP 401 030; EP 419 048), in which R³ is other than hydrogen and Ar¹ and Ar² are phenyl rings (European Patent Appl. EP 456 442), in which Ar¹ and / or Ar² are pyridinyl rings of formula (F), (G), or (J) (European Patent Appl. EP 504 888; EP 510 813; US Patent 5 149 699), in which Ar² is a furan or a thiophene of formula (L) (European Patent Appl. EP 510 812), in which Ar² is a pyrrol of formula (L) or formula (M) (European Patent Appl. EP 480 204, Int. Patent Appl. WO 92/11255, WO 92/15577), in which Ar¹ is an indole of formula (H) (European Patent Appl. EP 429 257), a benzothiophene of formula (H) (European Patent Appl. EP 430 709), or a benzofuran of formula (H) (European Patent Appl. EP 434 249; International Patent Appl. WO 92/09600), or in which Y is a group containing an acidic substituent R²⁰ (International Patent Appl. WO 91/11909; European Patent Appl. EP 519 831).

Compounds of formula V, in which the leaving group L^{b} is chloro or bromo, may be prepared by halogenation of compounds of formula XX, in particular, bromides by bromination with N-bromo succinimide in refluxing tetrachloromethane in the presence of a catalytic amount of dibenzoyl peroxide or azobis isobutyronitrile (AIBN). Compounds of formula V, in which L^{b} is a sulfonyloxy group, are prepared from benzylalcohols III (X = O) and the corresponding aliphatic or aromatic sulfonyl chlorides by standard procedures.

Compounds of formula IIIa, the special embodiment of formula III, in which n is 1, are prepared from compounds of formula V via suitable intermediates of formula XXI, in which the group X^{a} is a suitable precursor of the group X, or intermediates of formula XXII. For the preparation of benzylamines IIIa (X = NH) compounds of formula V are treated with an inorganic azide such as lithium or sodium azide in an inert polar solvent such as DMF, N,N-dimethyl acetamide, or DMSO, optionally by heating of the mixture. The resulting azide XXI (X^{a} = N₃) is reduced to the benzylamine IIIa by known methods, e.g. by catalytic hydrogenation, preferably with Pd on charcoal as catalyst, by reduction with LiAlH₄, or by treating with triphenylphosphine in solvents such as THF, ether, or 1,2-dimethoxyethane. In another preferred method compounds of formula V are reacted with potassium phthalamide in the above mentioned polar solvents, optionally by heating the mixture. The intermediates XXI (X^{a} = 1,3-dihydro-1,3-dioxo-2H-isoindol-2-yl) are converted to benzylamines IIIa by standard procedures, preferably by heating with hydrazine in methanol or in ethanol. Secondary benzylamines IIIa (X = NR⁴) are prepared by heating compounds of formula V with an excess of the amine R⁴NH₂ in solvents such as methanol, ethanol, or THF, or by reaction of the amines with aldehydes or ketones XXII by standard conditions and subsequent reduction with sodium borohydride or sodium cyanoborohydride. The carbonyl compounds XXII may be prepared by oxidation of compounds of formula V, preferably by heating in DMSO in the presence of a base such as sodium bicarbonate or by heating with 2-nitropropane in the presence of a base such as sodium ethoxide in ethanol. Benzylalcohols of formula IIIa (X = O) may be prepared by reduction of carbonyl compounds XXII, e.g. with sodium borohydride in methanol or ethanol, or by addition of Grignard reagents R³ MgBr or alkyllithium reagents R³ Li to aldehydes XXII (R³ = H). In another preferred method compounds of formula V are treated with inorganic salts of lower aliphatic carboxylic acids to intermediates XXI (X^{a} = OOC(C₁₋₄-alkyl)), preferably with sodium or potassium acetate to acetates XXI (X^{a} = OOCMe), which are hydrolized by standard procedures, preferably with aqueous alcoholic alkali hydroxide solution or by reduction with sodium or lithium borohydride. Benzylthiols IIIa (X =S) are prepared from compounds of formula V by standard methods such as heating with alkali sulfides such as sodium sulfide, or by heating with thiourea, sodium or potassium thioacetate, sodium thiosulfate, or reaction with carbon disulfide in the presence of an alkali C₁₋₄-alkoxide, preferably methoxide or ethoxide, to the corresponding intermediates XXI, which are cleaved to thiols IIIa, preferably by using alkaline standard conditions.

Compounds of formula IIIb, the special embodiment of formula III, in which n is 0, may be prepared from suitable precursors XXIII by conversion of suitable groups X^{b} to the groups XH. Thus, aromatic amines IIIb (X = NH) may be prepared by reduction of the corresponding nitro compounds XXIII (X^{b} = NO₂), preferably by catalytic hydrogenation of the nitro compounds, secondary aromatic amines IIIb (X = NR⁴) by alkylation of the primary amines or by their acylation with chlorides, anhydrides, or esters of corresponding carboxylic acids and subsequent reduction of the resulting amides with reagents such as LiAlH₄. Phenols of formula IIIb (X = O) may be prepared by cleavage of suitable protected phenols such as esters XXIII (X^{b} = OOC(C₁₋₄-alkyl), OOCPh), preferably acetates (X^{b} = OOCMe), or ethers XXIII (X^{b} = O(C₁₋₄-alkyl)), preferably methylethers (X^{b} = OMe) using standard procedures. Thiophenols of formula IIIb (X = S) may be prepared by metallation of aromatic bromides of formula XXIII (X^{b} = Br) with reagents such as butyl lithium or tert. butyl lithium at low temperatures, preferably below - 50 °C, and trapping the aryl lithium compounds with elemental sulfur or with tetraisopropylthiuram disulfide.

Substituents R⁵, R⁶, R⁷, R⁸, R⁹, R¹⁰, or R¹¹ in fused pyrimidines of formula (A), (B), (C), or (D) may be converted to other substituents. These conversions may be carried out on compounds of formula I, II, IV, XIII, or on any other compound, which contains one of the fused heterocycles and is presented in the schemes.

A substituent R⁵ in compounds containing a pyrazolo[1,5-a]pyrimidine of formula (A) or a substituent R¹⁰ in compounds containing an imidazo[1,2-a]pyrimidine of formula (D), which means hydrogen and which is connected to C-3 of the heterocycle, may be converted to halo, mercapto, nitro, nitroso, C₁₋₈-alkylsulfonyl, C₁₋₄-perfluoroalkylsulfonyl, phenylsulfonyl, SO₃H, SO₂NR²³R²⁴, formyl, C₁₋₆-alkanoyl, or benzoyl by known or according to known methods: H. Dorn, H. Dilcher, Liebigs Ann. Chem. **1967,** 707, 141; T. Novinson, B. Bhooshan, T. Okabe, G.R. Revankar, R.K. Robins, K. Senga, H.R. Wilson, J. Med. Chem. **1976**, 19, 512; W.E. Kirkpatrick, T. Okabe, I.W. Hillyard, R.K. Robins, A.T. Dren, T. Novinson, J. Med. Chem. **1977,** 20, 386; R.H. Springer, M.B. Scholten, D.E. O'Brien, T. Novinson, J.P. Miller, R.K. Robins, J. Med. Chem. **1982**, 25, 235; T. Novinson, R. Hanson, M.K. Dimmitt, L.N. Simon, R.K. Robins, D.E. O'Brien, J. Med. Chem. **1974**, 17, 645; T.Novinson, R.K. Robins, D.E. O'Brien, Tetrahedron Lett. **1973,** 3149; Y. Rival, G. Grassy, G. Michel, Chem. Pharm. Bull. **1992,** 40, 1170; T. Pyl, W. Baufeld, Liebigs Ann. Chem. **1966**, 699, 112. Thus, a chlorination is preferably carried out with N-chloro succinimide or with a mineral acid chloride such as sulfuryl chloride, a bromination preferably with N-bromo succinimide or with elemental bromine, an iodination preferably with iodine monochloride in an inert solvent such as acetonitrile, dichloromethane, chloroform, tetrachloromethane, or 1,2-dichloroethane, or in acetic acid, optionally by heating under reflux in these solvents. A nitration may be carried out with concentrated or fuming nitric acid, optionally in the presence of concentrated sulfuric acid, or with any other reagent, which is common in the art for the nitration of an aromatic ring. A nitroso compound (R⁵, R¹⁰ = nitroso) may be prepared in a similar manner using an alkali nitrite, preferably sodium nitrite, in mineral acids such as hydrochloric acid, in acetic acid, or in mixtures of both. A sulfonic acid (R⁵, R¹⁰ = SO₃H) may be prepared with fuming sulfuric acid, a sulfonamide (R⁵, R¹⁰ = SO₂NR²³R²⁴) with chlorosulfonic acid and treating of the intermediate sulfonyl chloride with an amine HNR²³R²⁴ using standard conditions. A formyl group (R⁵, R¹⁰ = CHO) is preferably introduced into the heterocycle by POCl₃ / DMF, the substituents R⁵, R¹⁰ = C₁₋₆-alkanoyl, benzoyl, C₁₋₈-alkylsulfonyl, or phenylsulfonyl by using chlorides of the corresponding carboxylic acids or sulfonic acids, respectively, optionally in the presence of a Lewis acid catalyst selected from aluminum chloride, zinc chloride, tin(IV) chloride, or titanium(IV) chloride. The substituent R⁵, R¹⁰ = C₁₋₄-perfluoroalkylsulfonyl, in particular trifluoromethylsulfonyl, may be introduced by using the corresponding sulfonic acid anhydrides (J.B. Hendrickson, K.W. Bair, J. Org. Chem **1977**, 24, 3875). Compounds, in which R⁵ or R¹⁰ is mercapto, may be prepared from the unsubstituted derivatives with inorganic isothiocyanates, preferably with potassium isothiocyanate, in the presence of elemental bromine in solvents such as methanol or ethanol or with other isothiocyanates such as Cu(II) isothiocyanate and subsequent hydrolysis of the intermediate by solutions of alkaline hydroxides such as sodium or potassium hydroxide in methanol, ethanol, or isopropanol. These thiols may also be prepared by heating of the corresponding compounds, in which R⁵ or R¹⁰ is bromo, with inorganic sulfides such as sodium sulfide in polar solvents such as DMF or N,N-dimethyl acetamide (M.R.H. Elmoghayar, M.K.A. Ibrahim, I. EI-Sakka, A.H.H. Elghandour, M.H. Elnagdi, Arch. Pharm. **1983**, 316, 697).

In compounds containing a pyrazolo[1,5-a]pyrimidine of formula (A) or a [1,2,4]triazolo[1,5-a]pyrimidine of formula (B), a substituent R⁵, which is connected to C-2 of the heterocycle, or a substituent R⁷, respectively, may be converted to other substituents. For example, a hydroxy group may be converted to a halogen, preferably to a chlorine or bromine, by heating with mineral acid halides such as POCl₃ or POBr₃ (W. Ried, K.-P. Peuchert, L. Ann. Chem. **1965**, 682, 136). A substituent R⁵ or R⁷, which is a suitable leaving group such as halo, preferably chloro or bromo, C₁₋₆-alkylthio or C₁₋₆-alkylsulfonyl, preferably methylthio or methylsulfonyl, particularly preferred methylsulfonyl, may be substituted by other substituents R⁵ or R⁷. For example, these substituents may be replaced by an C₁₋₈-alkoxy, C₁₋₃-phenylalkoxy, or a phenoxy, preferably by heating the corresponding alkali alkoxides or phenolates in alcohols of the same alkyl chain or in a polar solvent such as DMF. In a similar manner an amino group NH₂ or NR²³R²⁴ may be introduced by heating with ammonia or with the corresponding amine HNR²³R²⁴. A nitrile, in which R⁵ or R⁷ means cyano, may be prepared by heating of the corresponding chlorides, bromides, or preferably methylsulfones with inorganic cyanides, preferably with sodium cyanide, potassium cyanide, or copper(I) cyanide, in polar organic solvents such as DMF (J.R. Beck, M.P. Lynch, F.L. Wright, J. Heterocyclic Chem. **1988**, 25, 555; J.R. Beck, S.A. Ackmann, M.A. Staszak, F.L. Wright, J. Heterocyclic Chem. **1988,** 25, 955). A primary amine R⁵ or R⁷ = NH₂ may be converted to halogens selected from chlorine, bromine, or iodine, to hydrogen, C₁₋₆-alkylthio, or phenylthio by methods of diazotization of a primary amine, preferably by using alkyl nitrites such as isoamyl nitrite in inert organic solvents such as dichloromethane, chloroform, or THF (J.R. Beck, R.P. Gajewski, M.P. Lynch, F.L. Wright, J. Heterocyclic Chem. **1987**, 24, 267).

It is obvious to those skilled in the art that other conversions of substituents R⁵, R⁶, R⁷, R⁸, R⁹, R¹⁰, or R¹¹ connected to the bicyclic heterocycle may be carried out by standard procedures, e.g. esters COOR²² or amides CONR²³R²⁴ may be prepared from carboxylic acids; esters, amides, or nitriles may be hydrolyzed to carboxylic acids; a nitro or a nitroso group may be reduced to a primary amine, which may be converted to an amide or sulfonamide; a formyl group or an alkanoyl group may be reduced to a primary or secondary alcohol ; a hydroxy, mercapto, or amino group may be alkylated to an ether, thioether, or primary or secondary amino group; a cyano group may be converted to a 5-tetrazolyl; a formyl group by addition of a Grignard or an alkyl lithium reagent to a secondary alcohol ; a thioether may be oxidized to a sulfoxide, a sulfone, or to a sulfonic acid, which may be converted to a sulfonamide via its sulfonyl chloride.

The activity of the compounds of the present invention was determined by the following tests:

### Ligand-Receptor Binding Assay of the Angiotensin II Type 1 Receptor

The commercially available NED-014 kit (E.I. Du Pont de Nemours (Germany) GmbH, NEN Division) is used, and the binding assay is carried out as described by S.J. Fluharty and L.P. Reagan, J. Neurochemistry **1989**, 52, 1393. 200 µl of homogenized rat liver membrane suspension is incubated for 3-4 hours at room temperature with a buffered solution of 36 pM of the radioligand ([¹²⁵I]-Sar¹, Ile⁸ - AII, specific activity: 2200 Ci/mmol) alone and in the presence of various concentrations of the unlabeled test compound. After rinsing the membranes with wash buffer followed by vacuum filtration through a glass fiber filter the activity is determined in a scintillation counter. The affinity constant Kᵢ for binding of the compound to the receptor is determined from its IC50 value, which is the concentration blocking the binding of isotopically labeled ligand by 50 %, as decribed by Y.C. Cheng and W.H. Prusoff (Biochem. Pharmacol. **1973**, 22, 3099).

### In Vitro Test at the Isolated Rabbit Aorta

The test is carried out according to a published method (P.C. Wong, A.T. Chiu, W.A. Price, M.J.M.C. Thoolen, D.J. Carini, A.L. Johnson, R.I. Taber, P.B.M.W.M. Timmermans, J. Pharmacol. Exp. Ther. **1988**, 247, 1). Male New Zealand White rabbits (2-3 kg) are anaesthetized with carbon dioxide, debleeded, and laparotomized. A piece (3 cm) of the descending thoracic aorta is removed and conserved at 4 °C in Krebs-Ringer buffer solution (NaCI, 112; KCl, 5; CaCl₂·2H₂O, 2.5; KH₂PO₄, 1; MgSO₄·7H₂O. 1.2; NaHCO₃, 25; glucose, 11.5 (in millimolar concentrations)), which is bubbled with carbon dioxide. The aorta is cut into helical strips approximately 2 mm wide, which are transferred into an organ bath containing Krebs' bicarbonate solution at 37 °C continuously bubbled with carbon dioxide. The resting tension is set to 5 g, and the strips are allowed to equilibrate for 45 - 60 minutes. After this period a cumulative dose-response curve to angiotensin II (3·10⁻¹⁰ M to 1·10⁻⁷ M) is constructed. The bath is changed by several washing procedures until the base line is reached. 45 minutes later the angiotensin II antagonist is added in concentrations of 10⁻⁵ M, 10⁻⁶ M, 10⁻⁷ M, etc.. After incubation for 15 minutes the cumulative dose-response curve to angiotensin II is repeated in the presence of the test compound in each case. The responses are expressed as a percentage of the maximal angiotensin II response. The pA₂ values of the antagonists are determined from Schild plottings. They are in the range of pA₂ = 5.2 - 9.2 for the presented examples.

### In Vivo Test in Conscious Renal Artery-Ligated Hypertensive Sprague-Dawley Rats

Male Sprague-Dawley Rats weighing 250-300 g are randomized into a test compound-doses regime and into a control group for an appropriate vehicle control. A number of n = 8 animals is used per dose and test compound. The animals are anaesthetized with hexobarbital (Evipan-Sodium, 100 mg/kg, i.p.) and prepared accordimg to the method of J.L. Cangiano, C. Rodriguez-Sargent, and M. Martinez-Maldonado (J. Pharm. Exp. Ther. **1979,** 208, 310) by ligating the left renal artery. Six days after this surgical manipulation the activity of the test compound is determined. Again the animals are anaesthetized by injection of hexobarbital (80-100 mg/kg, i.p.), and both the right jugular vein and the carotid artery are cannulated. The catheters are passed subcutaneously to the dorsal side of the neck and exteriorized. 60 - 90 minutes after anaesthesia the animals have recovered, and their blood pressure and their heart rate are adjusted to a constant level. After 30 minutes registration of both parameters the test compound in DMSO / PEG 10 : 90 is administered orally or intraveneously through the jugular vein. The blood pressure of the animals is reduced by at least 20 % after intraveneous application of 10 mg/kg of a test compounds selected from the presented examples.

Thus, the compounds of the present invention are useful in human and veterinary medicine for the treatment and prophylaxis of cardiovascular and circulatory disorders such as hypertension, in particular essential, malignant, resistant, renal, renovascular, primary and secondary pulmonary hypertension, acute or chronic congestive heart failure, aortic or cardiac insufficiency, post-myocardial infarction, angina pectoris, peripheral ischemic disorders, cardiac and vascular hypertrophy, atherosclerosis, Raynauds's disease, for the treatment and prophylaxis of renal failure such as chronic renal failure, diabetic nephropathy, chronic glumerulonephritis, glumerular sclerosis and scleroderma, nephritis with proteinuria, impaired hyperuricemia, primary and secondary (pulmonary) hyperaldosteronism, for the treatment and prophylaxis of cerebrovascular, cognitive and learning disorders, and other disorders of the central nervous system (CNS) such as Alzheimer's disease, Parkinson's disease, depression, anxiety, (senile) dementia, schizophrenia, cerebral stroke or cerebral apoplexy, alcohol or drug dependency, for the treatment and prophylaxis of elevated intraocular pressure (glaucoma), and other diseases associated with the action of angiotensin II such as pulmonary diseases for example emphysia and edema of the lung or chronic bronchitis, Bartter's syndrome, gastrointestinal, bladder, or gynecological disorders. The compounds may also be used for the treatment of psoriasis, for perioperative organ protection, and for the prevention of postsurgical vascular restenosis.

The invention also includes a pharmaceutical composition comprising a pharmaceutically-acceptable diluent or carrier in association with a compound of formula I, or a pharmaceutically-acceptable salt thereof.

The compounds may be administered by various routes, for example, by the oral or rectal route, topically or parentally, for example by injection, being usually employed in the form of a pharmaceutical composition. Such compositions form part of the present invention and are prepared in a manner well known in the pharmaceutical art and normally comprise at least one active compound in association with a pharmaceutically-acceptable diluent or carrier. In making the compositions of the present invention, the active ingredient will usually be mixed with a carrier, or diluted by a carrier, and/or enclosed with a carrier which may, for example, be in the form of a capsule, sachet, paper or other container. Where the carrier serves as a diluent, it may be solid, semi-solid, or liquid material which acts as a vehicle, excipient or medium for the active ingredient. Thus, the composition may be in the form of tablets, lozenges, sachets, cachets, elixirs, suspensions, as a solid or in a liquid medium, ointments containing, for example, up to 10% by weight of the active compound, soft and hard gelatin capsules, suppositories, injection solutions and suspensions and sterile packaged powders.

Some examples of suitable carriers are lactose, dextrose, sucrose, sorbitol, mannitol, starches, gum acacia, calcium phosphate, alginates, tragacanth, gelatin, syrup, methyl cellulose, methyl- and propyl- hydroxybenzoate, talc magnesium stearate and mineral oil. The compositions of the injection may, as it is well known in the art, be formulated so as to provide quick, sustained or delayed release of the active ingredient after administration to the patient.

When the compositions are formulated in unit dosage form, it is preferred that each unit dosage form contains from 5 mg to 500 mg, for example, from 25 mg to 200 mg. The term 'unit dosage form' refers to physically discrete unit suitable as unit dosages for human subjects and animals, each unit containing a predetermined quantity of active material calculated to produce the desired therapeutic effect, in association with the required pharmaceutical carrier.

The active compounds are effective over a wide dosage range and, for example, dosages per day will normally fall within the range of from 0.5 to 300 mg/kg, more usually in the range of from 5 to 100 mg/kg. However, it will be understood that the amount administered will be determined by the physician in the light of the relevant circumstances, including the condition to be treated, the choice of compound to be administered and the chosen route of administration, and therefore the above dosage ranges are not intended to limit the scope of the invention in any way.

The invention is illustrated by the following Examples.

### Example 1

### 5-Ethyl-7-[N-methyl-N-((2'-(tetrazol-5-yl)biphenyl-4-yl)methyl)amino]pyrazolo[1,5-a]pyrimidine

### Step A: Preparation of 5-Ethylpyrazolo[1,5-a]pyrimidine-7-ol

119.9 g (1.443 mol) of 3(5)-aminopyrazole (prepared according to G. Ege, P. Arnold, Synthesis **1976**, 52) and 188.3 g (1.447 mol) of methyl 3-oxo-n-valerate were dissolved in 400 ml acetic acid and heated for 70 min at a bath temperature of 145 °C. After cooling to room temperature colorless crystals separated, which were removed from the mixture by suction, washed with ethanol, and dried *in vacuo,* m.p. 230 - 235°C

### Step B: Preparation of 7-Chloro-5-ethylpyrazolo[1,5-a]pyrimidine

16.1 g (98.7 mmol) of the compound of the previous step were mixed with 162 ml phosphorus oxytrichloride and 12 g (10.7 ml) of N,N-diethylaniline. The flask was dipped into a preheated oil bath and the mixture heated with reflux for 45 minutes. The POCl₃ was removed *in vacuo* and the remainig syrup poured on to crushed ice. After extraction for five times with dichloromethane the combined organic layers were washed with cold saturated sodium carbonate solution, dried over sodium sulfate, and evaporated *in vacuo.* The title compound was obtained after chromatography on silica gel with ethyl acetate / hexane 7:3 in pale yellow crystals,
m.p. 66 - 68°C

### Step C: Preparation of N-(2'-Cyanobiphenyl-4-yl)methyl-N-methyl amine

62.1 g (228 mmol) 4-bromomethyl-2'-cyanobiphenyl (prepared according to D.J. Carini et al., J. Med. Chem. **1992,** 34, 2525) were dissolved in 3 I THF. After addition of 1.5 1 of a 40 % aqueous solution of methylamine the mixture was stirred over night at room temperature, and the THF distilled off *in vacuo*. The remaining aqueous layer was extracted with ethyl acetate, the extract dried by stirring over night with 0.4 nm mole sieve, and evaporated *in vacuo*. The title compound was obtained as a viscous oil, which was pure enough according to its ¹H-NMR spectrum for further conversions,

### Step D: Preparation of 7-[N-(2'-Cyanobiphenyl-4-yl)methyl-N-methylamino]-5-ethylpyrazolo[1,5-a]pyrimidine

1.8 g (10 mmol) of the chloro compound of Step B and 2.2 g (10 mmol) of the amine of Step C were dissolved in 60 ml ethanol and heated with reflux for 4 h. After addition of 3 ml triethylamine the heating was continued for an additional hour. The mixture was diluted with water and extracted twice with dichloromethane. The combined organic layers were dried over sodium sulfate, evaporated *in vacuo*, and the title compound obtained as an oil after purification by column chromatography on silica gel using ethyl acetate / hexane 9:1 as eluent,

### Step E: Preparation of 5-Ethyl-7-[N-methyl-N-((2'-(tetrazol-5-yl)biphenyl-4-yl)methyl)amino]pyrazolo[1,5-a]pyrimidine

The compound was prepared according to a method from the patent WO 92 / 02508. 7.1 g (21.8 mmol) tributyltin chloride and 2.5 g (38.5 mmol) sodium azide were dissolved in 50 ml water. The mixture was stirred for 4 h at room temperature and extracted with 100 ml toluene. The extract was concentrated to a volume of about 30 ml and the remaining water azeotropically removed by this process. A solution of 2.0 g (5.4 mmol) of the nitrile from the previous step in 10 ml toluene was added and the whole heated with reflux for 90 h. The mixture was poured into a solution of 5.4 g sodium nitrite in 20 ml water and 20 ml 12 % hydrochloric acid with ice cooling. After stirring for some minutes a solution of 3.2 g sulphamic acid in 20 ml water was added and the whole again stirred for a while. A viscous precipitate was formed, which after decantation of the solvent was washed three times with water. The title compound separated in colorless crystals after addition of THF. These were filtered, washed with THF, and dried *in vacuo*,
m.p. 147 - 150°C (dec.)

### Example 2

### 5-Ethyl-7-[(2'-(tetrazol-5-yl)biphenyl-4-yl)methylamino]pyrazolo[1,5-a]pyrimidine

### Step A: Preparation of 7-[(2'-Cyanobiphenyl-4-yl)methylamino]-5-ethylpyrazolo[1,5-a]pyrimidine

5.5 g (30.3 mmol) of the chloride from Example 1, Step B and 6.3 g (30.3 mmol) of 4-aminomethyl-2'-cyanobiphenyl (prepared according to EP 459 136) were heated in 150 ml dry ethanol for 10 h. After removal of the solvent *in vacuo* yellow crystals formed. These were purified by suspension in acetone and dissolved in ethyl acetate. The solution was washed with 10 % sodium carbonate solution, the organic layer dried over sodium sulfate, and evaporated to dryness to give colorless crystals of the title compound,
m.p. 230 - 233°C

### Step B: Preparation of 5-Ethyl-7-[(2'-(tetrazol-5-yl)biphenyl-4-yl)methylamino]pyrazolo[1,5-a]pyrimidine

3.5 g (9.9 mmol) of the nitrile of the previous step were converted to its tetrazol with an excess of *in situ* formed tributyltin azide by the method described in Example 1, Step E. The title compound was obtained in two crops of colorless crystals from comparable purity after crystalisation from THF,
m.p. 177 - 179°C

### Example 3

### 3-Chloro-5-ethyl-7-[(2'-(tetrazol-5-yl)biphenyl-4-yl)methylamino]pyrazolo[1,5-a]pyrimidine

### Step A: Preparation of 3,7-Dichloro-5-ethylpyrazolo[1,5-a]pyrimidine

5.5 g (30 mmol) of the chloro compound (Example 1, Step B) and 4.0 g (30 mmol) of N-chlorosuccinimide were dissolved in 150 ml chloroform, stirred for 4 h at room temperature, and finally heated for some minutes on a steam bath. The mixture was poured on to ice water, the aqueous layer extracted with dichloromethane, the combined organic layers washed twice with saturated sodium carbonate solution, dried over sodium sulfate, and evaporated in *in vacuo*. The title compound was obtained as an oil after chromatography on silica gel with dichlormethane.

### Step B: Preparation of 3-Chloro-7-[(2'-cyanobiphenyl-4-yl)methylamino]-5-ethylpyrazolo[1,5-a]pyrimidine

2.2 g (10.2 mmol) of the chloride from the previous step and 2.1 g (10.1 mmol) 4-aminomethyl-2'-cyanobiphenyl were heated with reflux in 30 ml t-butanol for 15 h. The solvent was removed *in vacuo*, and the residue dissolved in ethyl acetate. After washing with 10 % sodium carbonate solution, the organic layer was dried over sodium sulfate, and evaporated *in vacuo.* The title compound was separated from remaining starting materials by column chromatography (silica gel, dichloromethane) and obtained in pale yellow crystals after evaporation to dryness,
m.p. 152 - 154°C

### Step C: Preparation of 3-Chloro-5-ethyl-7-[(2'-(tetrazol-5-yl)biphenyl-4-yl)methylamino]pyrazolo[1,5-a]pyrimidine

3.9 g (10 mmol) of the nitrile of the previous step were converted to its tetrazol with an excess of *in situ* formed tributyltin azide by the method described in Example 1, Step E. The title compound was obtained from THF in colorless crystals from the crude mixture,
m.p. 136 - 138°C

### Example 4

### 5-Ethyl-2-phenyl-7-[(2'-(tetrazol-5-yl)biphenyl-4-yl)methylamino]pyrazolo[1,5-a]pyrimidine

### Step A: Preparation of 5-Ethyl-2-phenylpyrazolo[1,5-a]pyrimidine-7-ol

4.9 g (30.8 mmol) 3-amino-5-phenylpyrazole, which was prepared from 3-oxo-3-phenylpropionitrile and hydrazine (M.H. Elnagdi, M.R.H. Elmoghayar, G.E.H. Elgemeie, Synthesis **1984**, 1), and 4.0 g (30.7 mmol) methyl 3-oxovalerate were heated for 70 min in 10 ml acetic acid. The title compound precipitated in colorless needles from the mixture upon cooling, which were filtered by suction, washed with water, and recrystallized from ethanol,
m.p. 316 - 318°C

### Step B: Preparation of 7-Chloro-5-ethyl-2-phenylpyrazolo[1,5-a]pyrimidine

3.3 g (13.8 mmol) of the hydroxy compound from the previous step were heated in 25 ml POCl₃ containing 1.5 ml N,N-diethylaniline according to Example 1, Step B. The chloride was purified by column chromatography (silica gel, dichloromethane with ascending polarity by addition of up to 2 % ethanol) and obtained in pale yellow crystals after evaporation to dryness,
m.p. 81 - 83°C

### Step C: Preparation of 7-[(2'-Cyanobiphenyl-4-yl)methylaminol]-5-ethyl-2-phenylpyrazolo[1,5-a]pyrimidine

2.6 g (10 mmol) of the chloro compound from the previous step and 2.1 g (10 mmol) of 4-aminomethyl-2'-cyanobiphenyl were heated in 30 ml dry ethanol for 10 h. The work-up procedure was described in Step B of the preceding example, and the title compound obtained as an oil after column chromatography (silica gel, dichloromethane),

### Step D: Preparation of 5-Ethyl-2-phenyl-7-[(2'-(tetrazol-5-yl)biphenyl-4-yl)methylamino]pyrazolo[1,5-a]pyrimidine

2.1 g (4.9 mmol) of the nitrile from the previous step were heated with an excess of *in situ* formed tributyltin azide according to Example 1, Step E. The tetrazole was obtained as a white powder by crystallization from THF,
m.p. 163 - 165 °C (dec.)

### Example 5

### 5-Ethyl-3-nitro-7-[(2'-(tetrazol-5-yl)biphenyl-4-yl)methylamino]pyrazolo[1,5-a]pyrimidine

### Step A: Preparation of 5-Ethyl-3-nitropyrazolo[1,5-a]pyrimidine-7-ol

6.0 g (46.8 mmol) 3(5)-amino-4-nitropyrazole (prepared according to H. Dorn, H. Dilcher, Liebigs Ann. Chem. **1967**, 707, 141) and 6.1 g (46.8 mmol) methyl 3-oxo-n-valerate were heated in 20 ml acetic acid for 70 min. After cooling of the mixture to room temperature crystals precipitated, which were filtered by suction and treated with saturated sodium bicarbonate solution. The remaining crystals were filtered, and another crop of crystals precipitated from the filtrate upon acidification with hydrochloric acid . Both crops were combined, washed with water, and dried *in vacuo* to give a pure sample of the title nitro compound in pale yellow crystals.
m.p. 220 - 222 °C

### Step B: Preparation of 7-Chloro-5-ethyl-3-nitropyrazolo[1,5-a]pyrimidine

7.3 g (35.1 mmol) of the hydroxy compound from the previous step were heated in 50 ml POCl₃ containing 3.2 ml N,N-diethylaniline according to Example 1, Step B. The title compound was purified by column chromatography (silica gel, dichloromethane) and obtained as a beige powder after evaporation to dryness, m.p. 139 - 140 °C

### Step C: Preparation of 7-[(2'-Cyanobiphenyl-4-yl)methylamino]-5-ethyl-3-nitropyrazolo[1,5-a]pyrimidine

3.4 g (15.0 mmol) of the chloride from the previous step and 3.1 g (14.9 mmol) 4-aminomethyl-2'-cyanobiphenyl were heated in 75 ml dry ethanol for 10 h. Upon cooling to room temperature crystals precipitated, which were filtered by suction and dissolved in dichloromethane. The solution was washed with 10 % sodium carbonate solution, dried over sodium sulfate, and evaporated to dryness *in vacuo* to give pale yellow crystals of the title compound,
m.p. 205 - 207 °C

### Step D: Preparation of 5-Ethyl-3-nitro-7-[(2'-(tetrazol-5-yl)biphenyl-4-yl)methylamino]pyrazolo[1,5-a]pyrimidine

2.8 g (7.0 mmol) of the nitrile from the previous step were heated with an excess of *in situ* formed tributyltin azide according to Example 1, Step E. The title tetrazole was obtained in yellow crystals from THF after the normal work-up procedure,
m.p. 138 - 139 °C (dec.)

### Example 6

### 3,5-Diethyl-7-[(2'-(tetrazol-5-yl)biphenyl-4-yl)methylamino]pyrazolo[1,5-a]pyrimidine

### Step A: Preparation of 3,5-Diethylpyrazolo[1,5-a]pyrimidine-7-ol

5.0 g (45.0 mmol) 3(5)-amino-4-ethylpyrazole (prepared by the method of R.H. Springer, M.B. Scholten, D.E. O'Brien, T. Novinson, J.P. Miller, R.K. Robins, J. Med. Chem. **1982**, 25, 235) and 5.85 g (45.0 mmol) methyl 3-oxo-n-valerate were heated in 14.5 ml acetic acid according to Example 1, Step A to give the title compound in colorless crystals, which were filtered by suction, washed with a small amout of acetic acid, stirred in 50 ml water, filtered again, and dried *in vacuo*,
m.p. > 310 °C

### Step B: Preparation of 7-Chloro-3,5-diethylpyrazolo[1,5-a]pyrimidine

2.2 g (11.5 mmol) of the compound from the previous step were heated in 19 ml POCl₃ containing 1.25 ml N,N-diethylaniline according to Example 1, Step B. After evaporation of the dichloromethane extract the crude title compound was obtained as a yellow oil, which was pure enough for the next step as detected by its ¹H-NMR spectrum.

### Step C: Preparation of 7-[(2'-Cyanobiphenyl-4-yl)methylamino]-3,5-diethylpyrazolo[1,5-a]pyrimidine

2.4 g (11.4 mmol) of the chloride from the previous step and 2.4 g (11.5 mmol) of 4-aminomethyl-2'-cyanobiphenyl were heated with reflux in 25 ml dry ethanol for 4 h. After cooling to room temperature solid precipitates were removed from the mixture by filtration, and the filtrate was evaporated *in vacuo*. The residue was stirred three times with 50 ml t-butylmethylether, while it solidified. The crystals were filtered off, and the combined filtrates were evaporated *in vacuo.* The title compound was obtained from the residue after chromatography (silica gel, dichloromethane) and evaporation of the pure fractions to dryness as a pale yellow oil, which solidified upon standing.

### Step D: Preparation of 3,5-Diethyl-7-[(2'-(tetrazol-5-yl)biphenyl-4-yl)methylamino]pyrazolo[1,5-a]pyrimidine

1.0 g (2.6 mmol) of the nitrile from the previous step were heated with an excess of *in situ* formed tributyltin azide according to Example 1, Step E. The title tetrazole was obtained in pale yellow crystals after evaporation of its solution in THF *in vacuo* and stirring of the residue with hexane,
m.p. 100 - 102 °C (dec.)

### Example 7

### 5-Methyl-7-[(2'-(tetrazol-5-yl)biphenyl-4-yl)methylamino]pyrazolo[1,5-a]pyrimidine

### Step A: Preparation of 7-[(2'-Cyanobiphenyl-4-yl)methylamino]-5-methylpyrazolo[1,5-a]pyrimidine

3.35 g (20 mmol) 7-chloro-5-methylpyrazolo[1,5-a]pyrimidine (prepared according to Y. Makisumi, Chem. Pharm. Bull. **1962**, 10, 620) and 4.17 g (20 mmol) 4-aminomethyl-2'-cyanobiphenyl were heated with reflux in 40 ml dry ethanol for 4 hours. The solvent was distilled off *in vacuo*, and the remaining oil solidified after stirring with a mixture of 20 ml hexane and 20 ml t-butylmethylether to give a reddish powder of the pure title compound,
m.p. 221 - 223 °C

### Step B: Preparation of 5-Methyl-7-[(2'-(tetrazol-5-yl)biphenyl-4-yl)methylamino]pyrazolo[1,5-a]pyrimidine

6.6 g (19.4 mmol) of the nitrile from the previous step were heated with an excess of *in situ* formed tributyltin azide according to Example 1, Step E. After the normal work-up procedure an oil was obtained, which gave colorless crystals of the title compound by stirring with THF,
m.p. 219 °C (dec.)

### Example 8

### 5-Butyl-7-[(2'-(tetrazol-5-yl)biphenyl-4-yl)methylamino]pyrazolo[1,5-a]pyrimidine

### Step A: Preparation of 5-Butylpyrazolo[1,5-a]pyrimidine-7-ol

6.5 g (37.7 mmol) ethyl 3-oxoheptanoate (prepared according to W. Wierenga, H.I. Skulnick, J. Org. Chem. **1979**, 44, 310) and 3.2 g (38.5 mmol) 3(5)-aminopyrazol were heated in 20 ml acetic acid for 70 min. The solvent was distilled off *in vacuo* and the residue dissolved in dichloromethane. After extraction with saturated sodium bicarbonate solution, drying over sodium sulfate, and evaporation *in vacuo* the title compound was obtained by crystallization from ethanol,
m.p. 177 - 178 °C, colorless crystals

### Step B: Preparation of 5-Butyl-7-chloropyrazolo[1,5-a]pyrimidine

5.0 g (26.1 mmol) of the hydroxy compound from the previous step were heated in 40 ml POCl₃ containing 2.8 ml N,N-diethylaniline according to Example 1, Step B, and the chloride was obtained after chromatography as a pale yellow oil.

### Step C: Preparation of 5-Butyl-7-[(2'-cyanobiphenyl-4-yl)methylamino]pyrazolo[1,5-a]pyrimidine

1.0 g (4.8 mmol) of the chloride from Step B and 1.0 g (4.8 mmol) of 4-aminomethyl-2'-cyanobiphenyl were heated in 40 ml ethanol for 5 h, and after removal of the solvent *in vacuo* the title compound was obtained from ethanol / hexane as a white powder,
m.p. 200 - 202 °C

### Step D: Preparation of 5-Butyl-7-[(2'-(tetrazol-5-yl)biphenyl-4-yl)methylamino]pyrazolo[1,5-a]pyrimidine

500 mg (1.3 mmol) of the nitrile from the previous step were heated with an excess of *in situ* formed tributyltin azide according to Example 1, Step E. The tetrazole was purified by chromatography (silica gel, dichloromethane containing 4 % ethanol) and obtained in crystals after evaporation to dryness,
m.p. 113 - 120 °C

### Example 9

### 5-Methyl-7-[N-propyl-N-((2'-(tetrazol-5-yl)biphenyl-4-yl)methyl)amino]pyrazolo[1,5-a]pyrimidine

### Step A: Preparation of 7-[N-(2'-Cyanobiphenyl-4-yl)methyl-N-propylamino]-5-methylpyrazolo[1,5-a]pyrimidine

2.5 g (10.0 mmol) of N-(2'-cyanobiphenyl-4-yl)methyl-N-propyl amine (prepared from n-propylamine and 4-bromomethyl-2'-cyanobiphenyl by the method described in EP 490 820) and 1.8 g (10.7 mmol) 7-chloro-5-methylpyrazolo[1,5-a]pyrimidine (prepared according to Y. Makisumi, Chem. Pharm. Bull. **1962**, 10, 620) were heated with reflux in 20 ml dry ethanol for 4 h. The mixture was concentrated to halve of the volume *in vacuo* and cooled with ice, while the remaining starting amine crystallized. It was filtered with suction, and the rest of the solvent was removed. The title compound was obtained from the residue after column chromatography (silica gel, acetone / hexane 1:9 and 2:3) as a yellow oil.

### Step B: Preparation of 5-Methyl-7-[N-propyl-N-((2'-(tetrazol-5-yl)biphenyl-4-yl)methyl)amino]pyrazolo[1,5-a]pyrimidine

920 mg (2.4 mmol) of the nitrile from the previous step were heated with an excess of *in situ* formed tributyltin azide according to Example 1, Step E. After the normal work-up procedure the tetrazole was purified by chromatography (silica gel, dichloromethane containing 5 % ethanol) and obtained as a beige powder after evaporation to dryness and stirring with hexane,
m.p. 110 - 112 °C

### Example 10

### 5-Ethyl-7-[(2'-(tetrazol-5-yl)biphenyl-4-yl)methylthio]pyrazolo[1,5-a]pyrimidine

### Step A: Preparation of 5-Ethylpyrazolo[1,5-a]pyrimidine-7-thiol

5.0 g (27.5 mmol) of the chloride from Example 1, Step B and 4.2 g (55.2 mmol) thiourea were heated in 100 ml dry ethanol for 5 h. The solvent was distilled off *in vacuo* and the residue crystallized from ethanol / water to give the title compound in yellow crystals,
m.p. 178 - 180 °C

### Step B: Preparation of 7-[(2'-Cyanobiphenyl-4-yl)methylthio]-5-ethylpyrazolo[1,5-a]pyrimidine

1.7 g (9.5 mmol) of the thiol from the previous step and 2.6 g (9.5 mmol) of 4-Bromomethyl-2'-cyanobiphenyl were dissolved in 30 ml dry DMF. 250 mg (10.4 mmol) sodium hydride were carefully added and the mixture stirred at room temperature over night. After careful addition of 100 ml water the title compound precipitated in pale yellow crystals, which were recrystallized from ethanol and dried *in vacuo,*
m.p. 153 - 155 °C

### Step C: Preparation of 5-Ethyl-7-[(2'-(tetrazol-5-yl)biphenyl-4-yl)methylthio]pyrazolo[1,5-a]pyrimidine

2.5 g (6.75 mmol) of the nitrile from the previous step were heated with an excess of *in situ* formed tributyltin azide according to Example 1, Step E. After the normal work-up procedure the residue was recrystallized from ethanol and the title compound obtained in yellow crystals,
m.p. 128 - 135 °C (dec.)

### Example 11

### 5-Ethyl-7-[(2'-(tetrazol-5-yl)biphenyl-4-yl)amino]pyrazolo[1,5-a]pyrimidine

### Step A: Preparation of 4-Amino-2'-cyanobiphenyl

6.0 g (26.8 mmol) of 2'-cyano-4-nitrobiphenyl (prepared according to B. Sain, J.S. Sandhu, J. Org. Chem. **1990**, 55, 2545) were dissolved in a mixture of 60 ml ethanol and 60 ml ethyl acetate, and the solution was filled into an autoclave. After addition of 660 mg 10 % Pd on charcoal the mixture was set under an atmosphere of hydrogen (2 - 3 bar). The reduction was carried out at room temperature, the course of the reaction monitored by thin layer chromatography, and it had completed after 2.5 h. The catalyst was filtered and the solvent distilled off *in vacuo.* The title amine was obtained by crystallization from hexane as a white powder,
m.p. 98 - 100 °C

### Step B: Preparation of 7-[(2'-Cyanobiphenyl-4-yl)amino]-5-ethylpyrazolo[1,5-a]pyrimidine

1.4 g (7.7 mmol) of the chloride from Example 1, Step B and 1.5 g (7.7 mmol) 4-Amino-2'-cyanobiphenyl were heated in 30 ml dry ethanol for 5 h. After removal of the solvent *in vacuo* the nitrile was obtained in colorless crystals from ethanol,
m.p. 247 - 248 °C

### Step C: Preparation of 5-Ethyl-7-[(2'-(tetrazol-5-yl)biphenyl-4-yl)amino]pyrazolo[1,5-a]pyrimidine

2.2 g (6.5 mmol) of the nitrile from the previous step were heated with an excess of *in situ* formed tributyltin azide according to Example 1, Step E. The title compound was purified by column chromatography (silica gel, dichloromethane containing 4 % to 10 % ethanol), and it was obtained in yellow crystals after evaporation to dryness,
m.p. 135 °C

### Example 12

### 4'-[(5-Ethylpyrazolo[1,5-a]pyrimidine-7-yl)amino]biphenyl-2-carboxylic acid

### Step A: Preparation of Ethyl 4'-[(5-Ethylpyrazolo[1,5-a]pyrimidine-7-yl)amino]biphenyl-2-carboxylate

A solution of 800 mg (4.4 mmol) 7-chloro-5-ethylpyrazolo[1,5-a]pyrimidine (Example 1, Step B) and 1.0 g (4.1 mmol) ethyl 4'-amino-biphenyl-2-carboxylate (prepared from protected 4-bromoaniline and ethyl 2-bromobenzoate by the method of J.C. Adrian, Jr., C.S. Wilcox, J. Am. Chem. Soc. **1989**, 111, 8055) in 40 ml dry ethanol was heated with reflux for 5 h. After cooling to room temperature pale yellow crystals of the title compound precipitated, which were filtered by suction and recrystallized from ethanol,
m.p. 223 - 225 °C

### Step B: Preparation of 4'-[(5-Ethylpyrazolo[1,5-a]pyrimidine-7-yl)amino]biphenyl-2-carboxylic acid

500 mg (1.3 mmol) of the ester from the previous step were dissolved in a mixture of 5 ml aqueous 2 N sodium hydroxide solution and 20 ml ethanol and kept for 12 h with stirring at a temperature between 40 °C and 50 °C. The solution was concentrated *in vacuo* to volume of about 10 ml and acidified with 2 N hydrochloric acid. The title compound precipitated in beige crystals, which were filtered by suction after cooling with ice and recrystallized from ethanol,
m.p. 251 - 256 °C (dec.)

### Example 13

### 5-Ethyl-7-[(2'-(tetrazol-5-yl)biphenyl-4-yl)methylamino]-[1,2,4]triazolo[1,5-a]pyrimidine

### Step A: Preparation of 5-Ethyl-[1,2,4]triazolo[1,5-a]pyrimidine-7-ol

33.6 g (0.4 mol) of 3-amino-1H-1,2,4-triazole and 52 g (0.4 mol) of methyl 3-oxo-n-valerate were heated in 130 ml acetic acid for 70 min. After cooling crystals precipitated, which were filtered by suction and dissolved in 1 l water. After several extractions with dichloromethane the combined organic layers were dried over sodium sulfate, and the solvent distilled off *in vacuo*. The title compound crystallized during the evaporation in colorless crystals,
m.p. 212 °C

### Step B: Preparation of 7-Chloro-5-ethyl-[1,2,4]triazolo[1,5-a]pyrimidine

7.4 g (45.1 mmol) of the compound from the previous step were heated for 45 min in 80 ml POCl₃ containing 4.1 ml N,N-diethylaniline. A red crystalline material was obtained after work-up of the mixture according to Example 1, Step B. The title chloride was purified by column chromatography (silica gel, dichloromethane with ascending polarity by addition of up to 2 % ethanol) and obtained in yellow crystals,
m.p. 155 - 157 °C

### Step C: Preparation of 7-[(2'-Cyanobiphenyl-4-yl)methylamino]-5-ethyl-[1,2,4]triazolo[1,5-a]pyrimidine

1.8 g (9.9 mmol) of the chloro compound from the previous step and 2.1 g (10.1 mmol) of 4-aminomethyl-2'-cyanobiphenyl were heated for 8 h in 30 ml t-butanol with reflux. The mixture was worked up according to Example 3, Step B, and the title compound obtained as an oil after column chromatography (silica gel, dichloromethane with ascending polarity by addition of up to 4 % ethanol).

### Step D: Preparation of 5-Ethyl-7-[(2'-(tetrazol-5-yl)biphenyl-4-yl)methylamino]-[1,2,4]triazolo[1,5-a]pyrimidine

1.2 g (3.4 mmol) of the nitrile from the previous step were heated with an excess of *in situ* formed tributyltin azide according to Example 1, Step E. The tetrazole was obtained in colorless crystals from THF after the normal work-up procedure,
m.p. 140 - 142 °C (dec.)

### Example 14

### 5-Ethyl-7-[((4-phthalamido)phenyl)amino]pyrazolo[1,5-a]pyrimidine

### Step A: Preparation of 5-Ethyl-7-[(4-nitrophenyl)amino]pyrazolo[1,5-a]pyrimidine

4.5 g (24.8 mmol) of 7-Chloro-5-ethylpyrazolo[1,5-a]pyrimidine (Example 1, Step B) and 3.5 g (25.3 mmol) 4-nitroaniline were heated in 75 ml dry ethanol for 5 h. During evaporation of the mixture *in vacuo* crystals precipitated, which were dissolved in dichloromethane. The solution was washed with saturated sodium bicarbonate solution, dried over sodium sulfate, and evaporated to dryness *in vacuo* to give the title compound as a yellow powder,
m.p. 158 - 160 °C

### Step B: Preparation of 7-[(4-Aminophenyl)amino]-5-ethylpyrazolo[1,5-a]pyrimidine

3.71 g (13.1 mmol) of the nitro compound from the previous step were dissolved in a mixture of 75 ml ethanol and 75 ml acetic acid, and 500 mg of 10 % Pd on charcoal were added. The mixture was filled into a hydrogenation vessel and stirred over night at room temperature under an atmosphere of hydrogen. The catalyst was filtered off, washed with ethanol and then washed with a mixture of equal volumes water and ethanol. The filtrate was evaporated *in vacuo*, the residue dissolved in dichloromethane, the solution extracted with saturated sodium bicarbonate solution, the organic layer dried over sodium sulfate, evaporated *in vacuo*, and the remaining crystals recrystallized from ethanol / hexane to give the title compound in pale yellow crystals,
m.p. 153 - 154 °C

### Step C: Preparation of 5-Ethyl-7-[((4-phthalamido)phenyl)amino]pyrazolo[1,5-a]pyrimidine

1.0 g (3.9 mmol) of the amine from the previous step and 580 mg (3.9 mmol) phthalic acid anhydride were dissolved in 40 ml dichloromethane and stirred for 3 days at room temperature, while a colorless precipitate was formed. In order to complete the reaction, the same amount of the anhydride, 600 mg of potassium carbonate, and a catalytic amount of triethylbenzylammonium chloride were added, and the mixture was stirred for additional 12 h at 40 °C. After addition of 5 ml of water it was stirred for another 10 min, and the title compound was filtered by suction, dried *in vacuo,* and recrystallized from ethanol to give beige crystals,
m.p.165 - 170 °C

### Example 15

### 2-[N-(4-(5-Ethylpyrazolo[1,5-a]pyrimidine-7-yl)aminophenyl)aminocarbonyl]benzene sulfonic acid

1.3 g (5.1 mmol) of the amine from Example 14, Step B and 940 mg (5.1 mmol) of 2-sulfobenzoic acid cyclic anhydride were dissolved in 20 ml dichloromethane. A solid material precipitated immediately from the clear solution, and the mixture was stirred at room temperature over night. The the pale yellow crystals of the title compound were filtered by suction, heated with ethanol, filtered again after cooling to room temperature, and dried *in vacuo,*
m.p. > 320 °C

### Example 16

### 1-(4-(N-(5-Ethylpyrazolo[1,5-a]pyrimidine-7-yl)amino)benzamido)-2-(tetrazol-5-yl)benzene Hydrochloride

### Step A: Preparation of 1-(4-Nitrobenzamido)-2-(tetrazol-5-yl)benzene

3.0 g (18.6 mmol) 2-(tetrazol-5-yl)aniline (prepared by heating of 2-aminobenzonitrile in the presence of sodium azide according to E.R. Wagner, J. Org. Chem. **1973**, 38, 2976) and 3.2 ml pyridine were dissolved in 50 ml dichloromethane. A solution of 3.7 g (19.9 mmol) 4-nitrobenzoyl chloride in 50 ml dichloromethane was added via a dropping funnel, and the mixture was heated with reflux for 1 h. The title compound precipitated from the solution, was filtered with suction, washed with hexane, and dried *in vacuo* to give a yellow powder,
m.p. 223 - 225 (dec.)

### Step B: Preparation of 1-(4-Aminobenzamido)-2-(tetrazol-5-yl)benzene

4.5 g (14.5 mmol) of the nitro compound from the previous step were dissolved in 120 ml dry THF. The solution was filled into a hydrogenation vessel, and 300 mg of 10 % Pd on charcoal were added. The mixture was stirred over night at room temperature under an atmosphere of hydrogen. The catalyst was removed by filtration, and the filtrate was evaporated *in vacuo* to give the pure title compound as a pale yellow powder,
m.p. 202 - 204 °C

### Step C: Preparation of 1-(4-(N-(5-Ethylpyrazolo[1,5-a]pyrimidine-7-yl)amino)benzamido)-2-(tetrazol-5-yl)benzene Hydrochloride

1.8 g (9.9 mmol) 7-Chloro-5-ethylpyrazolo[1,5-a]pyrimidine (Example 1, Step B) and 2.7 g (9.6 mmol) of the amine from the previous step were dissolved in 50 ml dry ethanol and heated with reflux for 6 h. A precipitate was formed upon cooling, which was isolated by filtration. To remove remaining starting materials the solid was heated in a mixture of methanol and water, and the pure title compound was obtained as a pale yellow powder after filtration with suction and drying *in vacuo*,
m.p. 300 °C (dec.)

### Example 17

### 6-Ethyl-7-[(2'-(tetrazol-5-yl)biphenyl-4-yl)methylamino]pyrazolo[1,5-a]pyrimidine

### Step A: Preparation of 6-Ethylpyrazolo[1,5-a]pyrimidine-7-ol

2.9 g (34.9 mmol) 3(5)-aminopyrazole and 5.0 g (34.7 mmol) ethyl 2-formylbutyrate (prepared from ethyl butyrate and ethyl formate by the method of S.S. Klioze, F.P. Darmory, J. Org. Chem. **1975**, 40, 1588) were dissolved in 20 ml dry ethanol and heated with reflux for 12 h. In order to complete the reaction, a solution of 800 mg (34.8 mmol) sodium in 5 ml dry ethanol was added and the mixture heated for additional 4 h. After cooling to room temperature solid precipitates were filtered off and the solution concentrated *in vacuo.* The residue was stirred with 150 ml water and adjusted to pH 4 with diluted hydrochloric acid. The title compound crystallized in colorless crystals, which were filtered with suction and dried *in vacuo*,
m.p. 247 - 248 °C

### Step B: Preparation of 7-Chloro-6-ethylpyrazolo[1,5-a]pyrimidine

1.13 g (6.9 mmol) of the compound from the previous step were heated in 11.5 ml POCl₃ containing 0.7 ml N,N-diethylaniline according to Example 1, Step B. The combined organic layers of dichloromethane were dried over sodium sulfate and evaporated *in vacuo* to give 1.3 g of the crude title compound as an oil, which was used for the next step without further purification.

### Step C: Preparation of 7-[(2'-Cyanobiphenyl-4-yl)methylamino]-6-ethylpyrazolo[1,5-a]pyrimidine

1.3 g (7 mmol) of the crude chloride from the previous step and 1.5 g (7.2 mmol) of 4-aminomethyl-2'-cyanobiphenyl were dissolved in 15 ml dry ethanol, heated with reflux for 4 h, and the solvent was removed *in vacuo*. Crystals were formed by stirring of the oily residue with hexane / t-butylmethylether, which were filtered by suction. These were suspended in water, the suspension was adjusted to pH 7 by diluted NaOH and extracted with dichloromethane. The organic layer was dried over sodium sulfate, evaporated *in vacuo,* and the residue gave beige crystals by stirring with hexane,
m.p. 151 - 153 °C

### Step D: Preparation of 6-Ethyl-7-[(2'-(tetrazol-5-yl)biphenyl-4-yl)methylamino]pyrazolo[1,5-a]pyrimidine

1.0 g (2.8 mmol) of the nitrile from the previous step were heated with an excess of *in situ* formed tributyltin azide according to Example 1, Step E. The tetrazol was purified by column chromatography (silica gel, dichloromethane and ascending polarity by addition of 5 % ethanol) and obtained in beige crystals after stirring with hexane / t-butylmethylether from the pure fractions,
m.p.101 - 103 °C (dec.)

### Example 18

### Ethyl 5-Ethyl-7-[(2'-(tetrazol-5-yl)biphenyl-4-yl)methylamino]pyrazolo[1,5-a]pyrimidine-3-carboxylate

### Step A: Preparation of Ethyl 5-Ethylpyrazolo[1,5-a]pyrimidine-7-ol-3-carboxylate

4.7 g (30.3 mmol) of commercially available ethyl 3(5)-aminopyrazole-4-carboxylate (Aldrich) and 3.9 g (30.0 mmol) methyl 3-oxo-n-valerate were heated with reflux in 9 ml acetic acid for 2.5 h. After standing over night at room temperature crystals precipitated. 10 ml of water were added, and the precipitate was filtered with suction, washed with water, and dried *in vacuo* to give the title compound as a white powder,
m.p. 173 - 174 °C

### Step B: Preparation of Ethyl 7-Chloro-5-ethylpyrazolo[1,5-a]pyrimidine-3-carboxylate

5.9 g (25.1 mmol) of the compound from the previous step were heated in 30 ml POCl₃ containing 2 ml N,N-diethylaniline according to Example 1, Step B. After the normal work-up procedure the title compound was purified by column chromatography (silica gel, dichloromethane). The pure fractions were evaporated to dryness *in vacuo*, and the chloride was obtained in orange crystals,
m.p. 115 - 117 °C

### Step C: Preparation of Ethyl 7-[(2'-Cyanobiphenyl-4-yl)methylamino]pyrazolo[1,5-a]pyrimidine-3-carboxylate

1.3 g (5.1 mmol) of the chloride from the previous step and 1.0 g (4.8 mmol) 4-aminomethyl-2'-cyanobiphenyl were dissolved in 30 ml dry ethanol and heated with reflux for 10 h. After work-up of the mixture as described in Example 3, Step B the title compound was purified by column chromatography (silica gel, dichloromethane) and obtained in pale yellow crystals by crystallization from dichloromethane / ether,
m.p. 148 - 150 °C

### Step D: Preparation of Ethyl 5-Ethyl-7-[(2'-(tetrazol-5-yl)biphenyl-4-yl)methylamino]pyrazolo[1,5-a]pyrimidine-3-carboxylate

1.0 g (2.35 mmol) of the nitrile from the previous step were heated with an excess of *in situ* formed tributyltin azide according to Example 1, Step E. After normal work-up the title compound was isolated from the crude mixture by column chromatography (silica gel, dichloromethane containing 8 % ethanol), and the remaining yellow oil of the pure fractions was treated with ethanol to give colorless crystals of the tetrazole,
m.p. 215 - 217 °C (dec.)

### Example 19

### 5-Ethyl-7-[(2'-(tetrazol-5-yl)biphenyl-4-yl)methylamino]pyrazolo[1,5-a]pyrimidine-3-carboxylic acid

320 mg (0.68 mmol) of the ester from the previous example were heated on a steam bath in 20 ml 2 N aqueous sodium hydroxide solution for 30 min. The clear solution was cooled to room temperature and adjusted to pH 6 by diluted hydrochloric acid. The title compound precipitated from the mixture, and it was filtered by suction, washed with ice cold water, and dried *in vacuo* to give a white powder,
m.p. 186 - 188 °C (dec.)

### Example 20

### 7-[(2'-(Tetrazol-5-yl)biphenyl-4-yl)methylamino]pyrazolo[1,5-a]pyrimidine

### Step A: Preparation of 5-Chloro-7-[(2'-cyanobiphenyl-4-yl)methylamino]pyrazolo[1,5-a]pyrimidine

5.7 g (30.3 mmol) 5,7-dichloropyrazolo[1,5-a]pyrimidine (prepared according to T. Novinson, B. Bhooshan, T. Okabe, G.R. Revankar, R.K. Robins, K. Senga, H.R. Wilson, J. Med. Chem. **1976**, 19, 512) and 6.3 g (30.25 mmol) 4-aminomethyl-2'-cyanobiphenyl were dissolved in 150 ml dry ethanol and heated with reflux for 10 h. The solvent was removed *in vacuo*, the residue dissolved in ethyl acetate, washed with 10 % aqueous sodium carbonate solution, and dried over sodium sulfate. After evaporation *in vacuo* the title compound crystallized from ethyl acetate / ether in colorless crystals,
m.p. 162 - 163 °C

### Step B: Preparation of 7-[(2'-Cyanobiphenyl-4-yl)methylamino]pyrazolo[1,5-a]pyrimidine

1.8 g (5.0 mmol) of the chloride from the previous step were dissolved in a mixture of 75 ml ethanol and 50 ml acetic acid. The solution was filled into a hydrogenation vessel and stirred over night under an atmosphere of hydrogen after addition of 300 mg of 10 % Pd on charcoal. The catalyst was removed by filtration, and the mixture was worked up as described in the preceding step. The title compound was separated from remaining starting material by column chromatography (silica gel, dichloromethane) and obtained as a colorless oil, which solidified upon standing,
m.p. 170 - 170 °C

### Step C: Preparation of 7-[(2'-(Tetrazol-5-yl)biphenyl-4-yl)methylamino]pyrazolo[1,5-a]pyrimidine

430 mg (1.32 mmol) of the nitrile from the previous step were heated with an excess of *in situ* formed tributyltin azide according to Example 1, Step E. The title compound was obtained in beige crystals after the normal work-up procedure and crystallization from THF, .
m.p. 150 - 155 °C

### Example 21

### 3-Chloro-5-ethyl-7-[N-methyl-N-((2'-(tetrazol-5-yl)biphenyl-4-yl)methyl)amino]pyrazolo[1,5-a]pyrimidine

### Step A: Preparation of 3-Chloro-7-[N-(2'-cyanobiphenyl-4-yl)methyl-N-methylamino]-5-ethylpyrazolo[1,5-a]pyrimidine

3.1 g (14.4 mmol) of the chloro compound (Example 3, Step A) and 3.2 g (14.4 mmol) of the amine (Example 1, Step C) were heated in 75 ml ethanol according to Example 4, Step C. The title compound was obtained as an oil after chromatography (silica gel; dichloromethane with ascending polarity by addition of up to 3 % ethanol).

### Step B: Preparation of 3-Chloro-5-ethyl-7-[N-methyl-N-((2'-(tetrazol-5-yl)biphenyl-4-yl)methyl)amino]pyrazolo[1,5-a]pyrimidine

2.8 g (7.0 mmol) of the nitrile from the previous step were converted to its tetrazole with an excess of *in situ* formed tributyltin azide by the method described in Example 1, Step E. The title compound was isolated by crystalization from THF / ether in colorless crystals,
m.p. 130 - 131 °C (dec.)

### Example 22

### 5-Ethyl-3-propyl-7-[(2'-(tetrazol-5-yl)biphenyl-4-yl)methylamino]pyrazolo[1,5-a]pyrimidine

### Step A: Preparation of 5-Ethyl-3-propylpyrazolo[1,5-a]pyrimidine-7-ol

12.5 g (100 mmol) 3(5)-amino-4-propylpyrazole (prepared from 2-formylvaleronitrile and hydrazine according to M.H. Elnagdi, M.R.H. Elmoghayar, G.E.H. Elgemeie, Synthesis **1984**, 1) and 13.0 g (100 mmol) methyl 3-oxovalerate were heated with reflux for 70 min in 35 ml acetic acid. The title compound precipitated from the mixture upon cooling. It was filtered with suction, washed with water, and dried *in vacuo* to give colorless crystals,
m.p. 279 - 281 °C

### Step B: Preparation of 7-Chloro-5-ethyl-3-propylpyrazolo[1,5-a]pyrimidine

8.9 g (43.3 mmol) of the hydroxy compound from the previous step were heated in 70 ml POCl₃ containing 4.6 ml N,N-diethylaniline according to Example 1, Step B. After the above described work-up procedure the crude title compound was obtained as a red oil, which solidified upon standing and which was used without further purification in the reaction of the next step.

### Step C: Preparation of 7-[(2'-Cyanobiphenyl-4-yl)methylamino]-5-ethyl-3-propylpyrazolo[1,5-a]pyrimidine

2.87 g (12.8 mmol) of the crude chloride from the previous step and 2.67 g (12.8 mmol) 4-aminomethyl-2'-cyanobiphenyl were dissolved in 30 ml dry ethanol and heated with reflux for 14 h. The solvent was removed *in vacuo*, and the residue was stirred with hexane and with t-butylmethylether to give crystals, which were filtered by suction and suspended in water. The mixture was neutralized by diluted aqueous sodium hydroxide solution, extracted with dichloromethane, dried over sodium sulfate, and evaporated *in vacuo*, and the title compound was obtained as a yellow oil after column chromatography (silica gel, dichloromethane).

### Step D: Preparation of 5-Ethyl-3-propyl-7-[(2'-(tetrazol-5-yl)biphenyl-4-yl)methylamino]pyrazolo[ 1,5-a]pyrimidine

1.0 g (2.5 mmol) of the nitrile from the previous step were converted to its tetrazole with an excess of *in situ* formed tributyltin azide by the method described in Example 1, Step E. After the normal work-up procedure the title compound was isolated from the residue by column chromatography (silica gel, dichloromethane and enhanced polarity by addition of 2.5 % ethanol) and obtained as a pale grey powder after stirring with hexane,
m.p. 125 °C (dec.)

### Example 23

### 2,5-Diethyl-3-methyl-7-[(2'-(tetrazol-5-yl)biphenyl-4-yl)methylamino]pyrazolo[1,5-a]pyrimidine

### Step A: Preparation of 2,5-Diethyl-3-methylpyrazolo[1,5-a]pyrimidine-7-ol

16.0 g (128 mmol) 3(5)-amino-5(3)-ethyl-4-methylpyrazole (prepared from 2-methyl-3-oxovaleronitrile and hydrazine according M.H. Elnagdi, M.R.H. Elmoghayar, G.E.H. Elgemeie, Synthesis **1984,** 1) and 16.7 g (128 mmol) methyl 3-oxovalerate were heated with reflux for 70 min in 40 ml acetic acid. The title compond was isolated from the mixture as described in Step A of the preceding example to give colorless crystals,
m.p. 287 °C

### Step B: Preparation of 7-Chloro-2,5-diethyl-3-methylpyrazolo[1,5-a]pyrimidine

5.5 g (26.8 mmol) of the hydroxy compound from the previous step were heated in 45 ml POCl₃ containing 2.8 ml N,N-diethylaniline according to Example 1, Step B. After the above described work-up procedure the crude title compound was obtained as a red oil, which solidified upon standing and which was used without further purification in the reaction of the next step.

### Step C: Preparation of 7-[2'-(Cyanobiphenyl-4-yl)methylamino]-2,5-diethyl-3-methyl-pyrazolo[1,5-a]pyrimidine

2.87 g (12.8 mmol) of the crude chloride from the previous step and 2.67 g (12.8 mmol) 4-aminomethyl-2'-cyanobiphenyl were dissolved in 30 ml dry ethanol and heated with reflux for 14 h. The title compound was obtained as a yellow oil after work-up as described in Step C of the preceding example.

### Step D: Preparation of 2,5-Diethyl-3-methyl-7-[(2'-(tetrazol-5-yl)biphenyl-4-yl)methylamino]pyrazolo[1,5-a]pyrimidine

1.7 g (4.3 mmol) of the nitrile from the previous step were converted to its tetrazole with an excess of *in situ* formed tributyltin azide by the method described in Example 1, Step E. The title compound was isolated from the mixture as described in Step D of the preceding example and obtained as a pale grey powder,
m.p. 125 °C (dec.)

### Example 24

### 5-Chloro-7-[(2'-(tetrazol-5-yl)biphenyl-4-yl)methylamino]pyrazolo[1,5-a]pyrimidine

1.8 g (5.0 mmol) of the nitrile from Example 20, Step A were heated with an excess of *in situ* formed tributyltin azide according to Example 1, Step E. After the normal work-up procedure the title compound was purified by column chromatography (silica gel, dichloromethane containing 3 % ethanol) and recrystallized from ethanol to give a pale yellow powder,
m.p. 210 - 211 °C (dec.)

### Example 25

### 2-[4-(N-(5-Ethylpyrazolo[1,5-a]pyrimidine-7-yl)amino)phenyl]-3-phenylpropionic acid

### Step A: Preparation of Diethyl 2-(4-Nitrophenyl)malonate

12.0 g (74.9 mmol) diethyl malonate were dissolved in 50 ml DMSO and 8.6 g (76.6 mmol) potassium t-butoxide were added. A solution of 7.9 g (50.1 mmol) 1-chloro-4-nitrobenzene in 50 ml DMSO was added via a dropping funnel. The mixture was heated at 100 °C for 4 h and poured on to crushed ice. It was extracted twice with ethyl acetate, the combined organic layers washed with brine, dried over sodium sulfate, and evaporated *in vacuo* to afford a brown oil, which contained the title compound in an amount of about 75 % and which was used in the reaction of the next step.

### Step B: Preparation of Diethyl 2-(4-Nitrophenyl)-2-(phenylmethyl)malonate

The remaining residue from the previous step was dissolved in 150 ml acetone, and 6.9 g (49.9 mmol) potassium carbonate and 8.55 g (50.0 mmol) benzyl bromide were added. The mixture was heated with reflux for 5 h, and after cooling to room temperature the inorganic material was filtered off. The filtrate was concentrated *in vacuo*, and the remaining oil was used in the next step without further purification.

### Step C: Preparation of 2-(4-Nitrophenyl)-3-phenylpropionic acid

The oil from the previous step was dissolved in a mixture of 100 ml acetic acid and 50 ml 5 N hydrochloric acid and heated with reflux for 40 h. The mixture was poured on to crushed ice and extracted with toluene, and the organic layer was extracted with 2 N sodium hydroxide solution. The alkaline aqueous extract was acidified with 12 % hydrochloric acid, while the title compound precipitated. The solid was filtered with suction, washed with water, and dried *in vacuo* to give the pure compound as a yellow powder,
m.p. 148 - 150 °C

### Step D: Preparation of 2-(4-Aminophenyl)-3-phenylpropionic acid

6.8 g (25.5 mmol) of the nitro compound from the previous step were dissolved in a mixture of 50 ml ethanol and 50 ml acetic acid. The solution was filled into a hydrogenation vessel, 500 mg of 10 % Pd on charcoal were added, and the mixture stirred over night under an atmosphere of hydrogen, while crystals of the title compound were formed. The precipitate was filtered through celite, and a first crop of the title amino acid was obtained by extraction of the remaining solids with dichloromethane and with hot ethanol and evaporation of the extracts *in vacuo.* Treatment of the rest of the solids with warm 2 N aqueous sodium hydroxide solution and acidification of the alkaline extract with diluted hydrochloric acid gave a second crop, which was filtered with suction and washed with water. The combined crops were dried in *vacuo* to give the pure title compound as a beige powder,
m.p. 196 - 198 °C

### Step E: Preparation of 2-[4-(N-(5-Ethylpyrazolo[1,5-a]pyrimidine-7-yl)amino)phenyl]-3-phenylpropionic acid

1.8 g (9.9 mmol) of 7-chloro-5-ethylpyrazolo[1,5-a]pyrimidine (Example 1, Step B) and 2.4 g (10.2 mmol) of the amine from the previous step were heated with reflux in 30 ml dry ethanol for 10 h. The solvent was removed *in vacuo*, the residue dissolved in dichloromethane, and washed with saturated sodium bicarbonate solution. The organic layer was dried over sodium sulfate, evaporated *in vacuo*, and the remaining solid recrystallized from ethanol to give the title compound as a pale yellow powder,
m.p. 195 - 197 °C

### Example 26

### 5-Ethyl-7-[(4--((phenyl)(tetrazol-5-yl)methyl)phenyl)amino]pyrazolo[1,5-a]pyrimidine

### Step A: Preparation of 7-[(4--((Cyano)(phenyl)methyl)phenyl)amino]-5-ethylpyrazolo[1,5-a]pyrimidine

2.1 g (10.1 mmol) 2-(4-Aminophenyl)-2-phenylacetonitrile (prepared by catalytic hydrogenation of phenylcyanomethylenequinone oxime according to the method of R.B. Davis, D.D. Carlos, G.S. Mattingly, J. Org. Chem. **1965,** 30, 2607) and 1.8 g (9.9 mmol) 7-Chloro-5-ethylpyrazolo[1,5-a]pyrimidine (Example 1, Step B) were dissolved in 50 ml dry ethanol and heated with reflux for 10 h. Solid materials, which precipitated after cooling to room temperature, were filtered, the filtrate evaporated *in vacuo*, the residue treated with 10 % aqueous sodium carbonate solution, and extracted with dichloromethane. The organic layer was dried over sodium sulfate, and the solvent was removed *in vacuo* to afford the crude title compound as an oil, which was pure enough for the next step as detected by its ¹H-NMR spectrum.

### Step B: Preparation of 5-Ethyl-7-[(4--((phenyl)(tetrazol-5-yl)methyl)phenyl)amino]pyrazolo[1,5-a]pyrimidine

2.3 g (6.5 mmol) of the nitrile from the previous step were converted to its tetrazole by heating with an excess of *in situ* formed tributyltin azide according to the method described in Example 1, Step E. The title compound was isolated by crystallization from THF / ether and obtained in grey crystals,
m.p. 220 - 222 °C (dec.)

### Example 27

### 7-[(3-Bromo-2-(2-(tetrazol-5-yl)phenyl)benzofuran-5-yl)methylamino]-5-ethylpyrazolo[1,5-a]pyrimidine

### Step A: Preparation of 7-[(3-Bromo-2-(2-cyanophenyl)benzofuran-5-yl)methylamino]-5-ethylpyrazolo[1,5-a]pyrimidine

540 mg (1.65 mmol) 5-(aminomethyl)-3-bromo-2-(2-cyanophenyl)benzofuran (prepared according to WO 92/09600) and 300 mg (1.65 mmol) 7-Chloro-5-ethylpyrazolo[1,5-a]pyrimidine (Example 1, Step B) were heated with reflux in 4 ml dry ethanol for 13 h. In order to complete the conversion of the amine 200 mg of the chloride and 0.24 ml triethylamine in 4 ml dry ethanol were added, and the mixture was heated for additional 21 h. The solvent was removed *in vacuo*, and the title compound isolated from the residue by chromatography (hexane / acetone 95:5 to 75:25) to give colorless crystals after evaporation to dryness,
m.p. 186 - 194 °C

### Step B: Preparation of 7-[(3-Bromo-2-(2-(tetrazol-5-yl)phenyl)benzofuran-5-yl)methylamino]-5-ethylpyrazolo[1,5-a]pyrimidine

380 mg (0.8 mmol) of the nitrile from the previous step were converted to its tetrazole by heating with an excess of *in situ* formed tributyltin azide according to the method described in Example 1, Step E. After the normal work-up procedure the tetrazole was purified by column chromatography (silica gel, dichloromethane and ascending polarity by addition of 5 % ethanol), and it was obtained as a beige powder after evaporation of the pure fractions to dryness,
m.p. 210 °C (dec.)

### Example 28

### 7-[N-((3-Bromo-2-(2-(tetrazol-5-yl)phenyl)benzofuran-5-yl)methyl)-N-methylamino]-5-ethylpyrazolo[1,5-a]pyrimidine

### Step A: Preparation of N-((3-Bromo-2-(2-cyanophenyl)benzofuran-5-yl)methyl)-N-methyl amine

3.1 g (7.9 mmol) 3-Bromo-5-(bromomethyl)-2-(2-cyanophenyl)benzofuran (prepared according to EP 434 249) were dissolved in 50 ml THF, and 25 ml of a 40 % aqueous solution of methylamine was added via a dropping funnel. The mixture was stirred at room temperature over night and warmed for 2 h at 40 °C. The solvent was removed *in vacuo*, the residue stirred with water, filtrated by suction, and washed with water. The title compound was purified by column chromatography (silica gel, dichloromethane / ethanol 99:1 to 90:10) and obtained as a colorless powder after evaporation to dryness,
m.p. 114 °C

### Step B: Preparation of 7-[N-((3-Bromo-2-(2-cyanophenyl)benzofuran-5-yl)methyl)-N-methylamino]-5-ethylpyrazolo[1,5-a]pyrimidine

800 mg (2.34 mmol) of the amine from the previous step and 410 mg (2.26 mmol) of 7-Chloro-5-ethylpyrazolo[1,5-a]pyrimidine (Example 1, Step B) were heated with reflux in 8 ml dry ethanol for 14 h. Additional 100 mg of the chloride were added, and the heating was continued for 3 h. The solvent was removed *in vacuo*, the residue stirred with acetone, and the remaining solid filtered with suction. It was dissolved in ethyl acetate, washed successively with saturated sodium bicarbonate solution and with water, the organic layer dried over sodium sulfate, and evaporated to dryness *in vacuo* to give the pure title compound as a beige powder,
m.p. 130 - 136 °C

### Step C: Preparation of 7-[N-((3-Bromo-2-(2-(tetrazol-5-yl)phenyl)benzofuran-5-yl)methyl)-N-methylamino]-5-ethylpyrazolo[1,5-a]pyrimidine

530 mg (1.09 mmol) of the nitrile from the previous step were converted to its tetrazole by heating with an excess of *in situ* formed tributyltin azide according to the method described in Example 1, Step E. After the normal work-up procedure the tetrazole was purified by column chromatography (silica gel, dichloromethane and ascending polarity by addition of 8 % ethanol). The title compound was obtained as a beige powder after recrystallization from acetone,
m.p. 244 °C (dec.)

### Example 29

### 5-Ethyl-7-[N-methyl-N-((2-(2-(tetrazol-5-yl)phenyl)benzofuran-5-yl)methyl)amino]pyrazolo[1,5-a]pyrimidine

### Step A: Preparation of N-((2-(2-Cyanophenyl)benzofuran-5-yl)methyl)-N-methyl amine

5.3 g (17.0 mmol) 5-(bromomethyl)-2-(2-cyanophenyl)benzofuran (prepared according to EP 434 249) were dissolved in 100 ml THF, and 25 ml of a 40 % aqueous solution of methylamine were added via a dropping funnel. The mixture was stirred at room temperature over night. 2 x 15 ml of the solution of methylamine were added, and the mixture was stirred for additional 5 h in each case. The work-up and the isolation of the title compound was carried out as described in Step A of the preceding example to give a colorless powder after crystallization of the remaining oil upon stirring with t-butylmethylether,
m.p. 180 °C

### Step B: Preparation of 7-[N-((2-(2-Cyanophenyl)benzofuran-5-yl)methyl)-N-methylamino]-5-ethylpyrazolo[1,5-a]pyrimidine

750 mg (2.86 mmol) of the amine from the previous step and 500 mg (2.75 mmol) 7-Chloro-5-ethylpyrazolo[1,5-a]pyrimidine (Example 1, Step B) were heated with reflux in 7 ml dry ethanol for 7 h. 100 mg of the chloride were added, and the mixture was heated for additional 10 h. The title compound was isolated as described in Step B of the preceding example to give a beige powder,
m.p. 143 °C

### Step C: Preparation of 5-Ethyl-7-[N-methyl-N-((2-(2-(tetrazol-5-yl)phenyl)benzofuran-5-yl)methyl)amino]pyrazolo[1,5-a]pyrimidine

410 mg (1.0 mmol) of the nitrile from the previous step were converted to its tetrazole by heating with an excess of *in situ* formed tributyltin azide according to the method described in Example 1, Step E. The title compound was obtained in colorless crystals after the normal work-up procedure, chromatographic purification (silica gel, dichloromethane with ascending polarity by addition of 0.5 % to 15 % ethanol), and recrystallization from acetone,
m.p. 168 °C (dec.)

### Example 30

### 5-Ethyl-7-[(4-(2-(tetrazol-5-yl)pyrrol-1-yl)phenyl)methylamino]pyrazolo[1,5-a]pyrimidine

### Step A: Preparation of 2-Cyano-1-[4-((1,3-dihydro-1,3-dioxo-2H-isoindol-2-yl)methyl)phenyl]pyrrole

17.0 g (65.1 mmol) 1-(4-(bromomethyl)phenyl)-2-cyanopyrrole (prepared according to P.R. Bovy et al., J. Med Chem. **1993**, 36, 101) and 22.0 g (119 mmol) potassium phthalimide were dissolved in 260 ml dry DMF and stirred at 70 °C for 10 h. It was cooled to room temperature, and 600 ml ice-cold water were added. After extraction with ethyl acetate, the organic layer was dried over sodium sulfate, and evaporated *in vacuo.* The remaining oil solidified upon standing, and it was stirred with t-butylmethylether to give pale yellow crystals of the title compond, which were filtered with suction and dried *in vacuo,*
m.p. 167 - 168 °C

### Step B: Preparation of 1-(4-(Aminomethyl)phenyl)-2-cyanopyrrole

11.2 g (34.2 mmol) of the compound from the previous step were dissolved in 420 ml dry methanol, and 1.1 ml of hydrazine hydrate were added. The mixture was heated with reflux, and for two times additional 0.5 ml hydrazine hydrate were added after 1 h and 3 h. The heating was continued for another 5 h. It was cooled with ice, and crystals precipitated, which were removed by filtration. The filtrate was evaporated *in vacuo*, treated with 2 N aqueous sodium hydroxide solution, and extracted with ethyl acetate. The organic layer was washed with water, dried over sodium sulfate, and the solvent was distilled off *in vacuo*. The residue was treated with t-butylmethylether to give colorless crystals, which were removed by filtration. The filtrate was concentrated, and the title compound was isolated by column chromatography (silica gel, acetone / hexane 1:2) to give a viscous yellow oil.

### Step C: Preparation of 7-[(4-(2-Cyanopyrrol-1-yl)phenyl)methylamino]-5-ethylpyrazolo[1,5-a]pyrimidine

2.3 g (11.7 mmol) of the amine from the previous step and 2.18 g (12.0 mmol) 7-Chloro-5-ethylpyrazolo[1,5-a]pyrimidine (Example 1, Step B) were heated with reflux in 27 ml dry ethanol for 4 h. The solvent was removed *in vacuo*, and the title compound isolated from the residue by column chromatography (silica gel, dichloromethane / ethanol 97:3) to give a pale yellow oil.

### Step D: Preparation of 5-Ethyl-7-[(4-(2-(tetrazol-5-yl)pyrrol-1-yl)phenyl)methylamino]pyrazolo[1,5-a]pyrimidine

900 mg (2.63 mmol) of the nitrile from the previous step were converted to its tetrazole by heating with an excess of *in situ* formed tributyltin azide according to the method described in Example 1, Step E. The title compound was isolated from the residue by column chromatography (silica gel, dichloromethane containing 3 % and 5 % ethanol). It was obtained in beige crystals from the pure fractions after evaporation to dryness *in vacuo*,
m.p. 129 - 130 °C

### Example 31

### 5-Ethyl-7-[N-methyl-N-((4-(2-(tetrazol-5-yl)pyrrol-1-yl)phenyl)methyl)amino]pyrazolo[1,5-a]pyrimidine

### Step A: Preparation of 2-Cyano-1-((4-(N-methylamino)methyl)phenyl)pyrrole

7.5 g (28.7 mmol) of 1-(4-(bromomethyl)phenyl)-2-cyanopyrrole (prepared according to P.R. Bovy et al., J. Med Chem. **1993**, 36, 101) were dissolved in 165 ml THF, and 90 ml of a 40 % aqueous solution of methylamine were added to this solution via a dropping funnel. The mixture was stirred over night at room temperature, and the THF removed *in vacuo.* The residue was treated with 100 ml water, extracted with ethyl acetate, the organic layer dried over sodium sulfate, and evaporated *in vacuo.* The title compound was purified by column chromatography (silica gel, acetone / hexane with ascending polarity from 1:16 to 1:1). An analytical sample was obtained from acetone in colorless crystals,
m.p. 198 - 199 °C

### Step B: Preparation of 7-[N-((4-(2-Cyanopyrrol-1-yl)phenyl)methyl)-N-methylamino]-5-ethylpyrazolo[1,5-a]pyrimidine

2.0 g (9.5 mmol) of the amine from the previous step and 1.72 g (9.5 mmol) of 7-Chloro-5-ethylpyrazolo[1,5-a]pyrimidine (Example 1, Step B) were dissolved in 25 ml dry ethanol and heated with reflux for 4 h. The solvent was removed *in vacuo*, the residue treated was saturated aqueous sodium bicarbonate solution, and extracted with ethyl acetate. The organic layer was dried over sodium sulfate and evaporated *in vacuo* to give the pure title compound as a yellow oil.

### Step C: Preparation of 5-Ethyl-7-[N-methyl-N-((4-(2-(tetrazol-5-yl)pyrrol-1-yl)phenyl)methyl)amino]pyrazolo[1,5-a]pyrimidine

900 mg (2.53 mmol) of the nitrile from the previous step were heated with an excess of *in situ* formed tributyltin azide according to Example 1, Step E. After the normal work-up procedure the title compound was purified by column chromatography (silica gel, dichloromethane containing 5 % ethanol) to give the title compound in beige crystals after evaporation to dryness,
m.p. 115 - 117 °C (dec.)

### Example 32

### 7-Ethyl-5-[(2'-(tetrazol-5-yl)biphenyl-4-yl)methylamino]imidazo[1,2-a]pyrimidine

### Step A: Preparation of 7-Ethylimidazo[1,2-a]pyrimidine-5-ol

20.0 g (151.4 mmol) commercially available (Aldrich) 2-aminoimidazole sulfate and 19.7 g (151.4 mmol) methyl 3-oxo-n-valerate were heated with reflux in 27 ml acetic acid for 7 h. After cooling to room temperature remaining solids were removed by filtration, and the filtrate was diluted with water, neutralized with sodium hydroxide, and extracted with dichloromethane. The organic layer was dried over sodium sulfate, evaporated *in vacuo*, while the title compound crystallized in colorless crystals, which were filtered with suction, washed with hexane, and dried *in vacuo*,
m.p. 93 - 94 °C

### Step B: Preparation of 5-[(2'-Cyanobiphenyl-4-yl)methylamino]-7-ethylimidazo[1,2-a]pyrimidine

1.68 g (10.3 mmol) of the compound from the previous step, 2.13 g (10.2 mmol) 4-aminomethyl-2'-cyanobiphenyl, and 2.13 g (10.3 mmol) dicyclohexyl carbodiimide (DCC) were dissolved in 80 ml dry dioxane and stirred over night at 50 °C. Another 1 g (4.8 mmol) of DCC was added, and the reaction was continued for 8 h at 50 °C and over night at 80 °C. The mixture was cooled to room temperature, solids were filtered, and the solvent was distilled off *in vacuo.* The title compound was isolated from the residue by column chromatography (silica gel, dichloromethane / ethanol 20:1) and obtained in colorless crystals after stirring with t-butylmethylether,
m.p. 99 - 100 °C

### Step C: Preparation of 7-Ethyl-5-[(2'-(tetrazol-5-yl)biphenyl-4-yl)methylamino]imidazo[1,2-a]pyrimidine

1.3 g (3.7 mmol) of the nitrile from the previous step were converted to its tetrazole by heating with an excess of *in situ* formed tributyltin azide according to the method described in Example 1, Step E. After work-up as described above the solution in THF was concentrated *in vacuo*, and the title compound crystallized upon treatment with hexane. It was purified by column chromatography (silica gel, dichloromethane with ascending polarity by addition of 1 % to 3 % ethanol), and obtained from the pure fractions in pale yellow crystals,
m.p. 92 - 93 °C

### Example 33

### (S)-2-[(4-(5-Ethylpyrazolo[1,5-a]pyrimidine-7-yl)amino)benzamido]-3-phenylpropanoic acid

### Step A: Preparation of Methyl (S)-2-(4-Nitrobenzamido)-3-phenylpropanoate

2.0 g (9.3 mmol) L-phenylalanine methylester hydrochloride and 1.73 g (9.3 mmol) 4-nitrobenzoyl chloride were dissolved in a mixture of 10 ml dichloromethane and 5 ml pyridine and stirred over night at room temperature. The mixture was evaporated *in vacuo*, while a colorless solid precipitated, which was filtered by suction, recrystallized from ethanol, washed with diisopropylether, and dried *in vacuo* to give the title compound in colorless needles,
m.p. 115 - 116 °C

### Step B: Preparation of Methyl (S)-2-(4-Aminobenzamido)-3-phenylpropanoate

2.4 g (7.3 mmol) of the nitro compound from the previous step were dissolved in 30 ml dry methanol, and 300 mg of 10 % Pd on charcoal were added. The mixture was filled into a hydrogenation vessel and stirred for 18 h at room temperature under an atmosphere of hydrogen. The catalyst was filtered off and washed with ethanol. After removal of the solvent *in vacuo* the remaining solid was recrystallized from ethanol to give the title compound in colorless crystals,
m.p. 145 - 146 °C

### Step C: Preparation of Methyl (S)-2-[(4-(5-Ethylpyrazolo[1,5-a]pyrimidine-7-yl)amino)benzamido]-3-phenylpropanoate

1.8 g (6.0 mmol) of the amine from the previous step and 1.1 g (6.0 mmol) of 7-chloro-5-ethylpyrazolo[1,5-a]pyrimidine (Example 1, Step B) were dissolved in 20 ml dry ethanol and heated with reflux for 5 h. The solvent was removed *in vacuo*, and the title compound obtained from the residue after chromatography (silica gel, ethyl acetate / hexane 4:1) and evaporation of the pure fractions to dryness in colorless crystals,
m.p. 207 - 209 °C

### Step D: Preparation of (S)-2-[(4-(5-Ethylpyrazolo[1,5-a]pyrimidine-7-yl)amino)benzamido]-3-phenylpropanoic acid

1.0 g (2.25 mmol) of the ester from the previous step was stirred at room temperature for 4 h in 20 ml aqueous 2 N sodium hydroxide solution. The mixture was acidified to pH 2 with diluted hydrochloric acid and concentrated to halve of the volume *in vacuo.* The formed precipitate was filtered by suction, recrystallized from methanol, washed with diisopropylether, and dried *in vacuo* to give the title compound in colorless crystals,
m.p. 227 - 232 °C

### Example 34

### Ethyl 2-[N-(5-Ethylpyrazolo[1,5-a]pyrimidine-7-yl)-N-((2'-(tetrazol-5-yl)biphenyl-4-yl)methyl)amino]acetate

### Step A: Preparation of Ethyl 2-[N-(5-Ethylpyrazolo[1,5-a]pyrimidine-7-yl)amino]acetate

1.8 g (9.9 mmol) 7-Chloro-5-ethylpyrazolo[1,5-a]pyrimidine (Example 1, Step B) and 1.4 g (10.0 mmol) ethyl 2-aminoacetate hydrochloride were dissolved in 50 ml dry ethanol. 500 mg (4.7 mmol) sodium carbonate were added, and the mixture was heated with reflux for 2 h. The inorganic material was filtered off, the residue treated with saturated aqueous sodium bicarbonate solution, and extracted with ethyl acetate. The organic layer was dried over sodium sulfate, and evaporated *in vacuo* to give the crude title compound as an oil, which was pure enough according to its ¹H-NMR spectrum and was used for the reaction of the next step without further purification.

### Step B: Preparation of Ethyl 2-[N-(5-Ethylpyrazolo[1,5-a]pyrimidine-7-yl)-N-((2'-(N-triphenylmethyl-tetrazol-5-yl)biphenyl-4-yl)methyl)amino]acetate

2.1 g (8.5 mmol) of the crude ester from the previous step were dissolved in 40 ml dry THF, and the solution was poured on to 200 mg (8.3 mmol) sodium hydride. The mixture was kept with stirring at 75 °C for 1 h, 4.7 g (8.4 mmol) 4-(bromomethyl)-2'-(N-triphenylmethyl-tetrazol-5-yl)biphenyl were added, and the heating was continued for 2 h. The mixture was poured on to crushed ice, extracted with ethyl acetate, the organic layer dried over sodium sulfate, and evaporated *in vacuo.* The title compound was isolated by column chromatography (silica gel, dichloromethane / ethanol 98:2) and obtained as an oil.

### Step C: Preparation of Ethyl 2-[N-(5-Ethylpyrazolo[1,5-a]pyrimidine-7-yl)-N-((2'-(tetrazol-5-yl)biphenyl-4-yl)methyl)amino]acetate

340 mg (0.47 mmol) of the protected tetrazole from the previous step were dissolved in 4 ml ethanol containing 0.25 ml concentrated aqueous hydrochloric acid, and the mixture was stirred at room temperature for 90 minutes. The alcohol was removed *in vacuo*, the residue treated with water, neutralized by addition of sodium acetate, and extracted with ethyl acetate. The organic layer was dried over sodium sulfate, evaporated *in vacuo*, and the title compound was obtained as a colorless oil after column chromatography (silica gel, dichlormethane / ethanol 96:4).

### Example 35

### 2-[N-(5-Ethylpyrazolo[1,5-a]pyrimidine-7-yl)-N-((2'-(tetrazol-5-yl)biphenyl-4-yl)methyl)amino]acetic acid

900 mg (12.4 mmol) of the protected tetrazole from Step B of the previous example were heated with a mixture of 25 ml aqueous 2 N sodium hydroxide solution and 10 ml ethanol on a steam bath for 2 h, while it became a clear solution. It was cooled to room temperature, brought to pH 4 with diluted aqueous hydrochloric acid, and extracted with ethyl acetate. The organic layer was dried over sodium sulfate, evaporated *in vacuo*, and the title acid was purified by column chromatography (silica gel, dichloromethane / ethanol 9:1). It was obtained in colorless crystals after evaporation to dryness,
m.p. 224 - 225 °C (dec.)

### Example 36

### 7-[(3-Bromo-2-(2-(tetrazol-5-yl)phenyl)benzofuran-5-yl)methylamino]-5-methylpyrazolo[1,5-a]pyrimidine

### Step A: Preparation of 7-[(3-Bromo-2-(2-cyanophenyl)benzofuran-5-yl)methylamino]-5-methylpyrazolo[1,5-a]pyrimidine

A solution of 1.7 g (10.1 mmol) 7-chloro-5-methylpyrazolo[1,5-a]pyrimidine (prepared according to Y. Makisumi, Chem. Pharm. Bull. **1962**, 10, 620) and 3.27 g (10.0 mmol) 5-(aminomethyl)-3-bromo-2-(2-cyanophenyl)benzofuran (prepared according to WO 92/09600) in 50 ml dry ethanol was heated with reflux for 5 h. The title compound precipitated upon cooling to room temperature, was filtered with suction, and recrystallized from ethanol to give colorless crystals.
yield: 3.5 g (76 %), m.p. 210 - 215 °C

### Step B: Preparation of 7-[(3-Bromo-2-(2-(tetrazol-5-yl)phenyl)benzofuran-5-yl)methylamino]-5-methylpyrazolo[1,5-a]pyrimidine

1.8 g (3.9 mmol) of the nitrile from the previous step were heated with an excess of *in situ* formed tributyltin azide according to Example 1, Step E. The title compound was obtained as a colorless powder after the normal work-up procedure, stirring with THF, and recrystallization from ethanol.
yield: 1.8 g (91 %), m.p. > 250 °C

## Claims

1. A compound of the formula 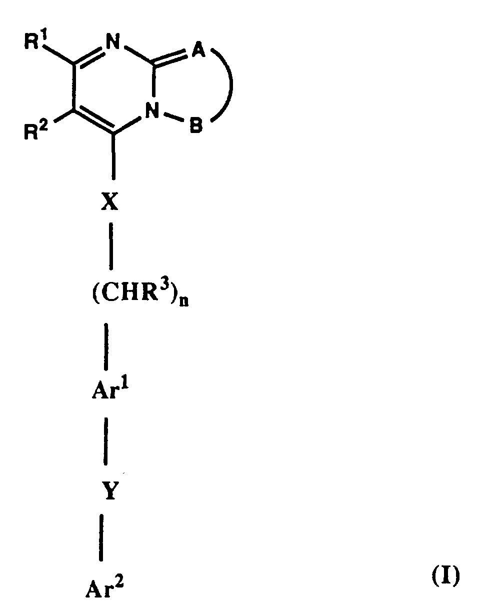 in which
R¹ is either R^{1a} selected from
a) hydrogen,
b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents, or by a single hydroxy, C₁₋₄-alkoxy, or C₁₋₄-alkylthio,
c) phenyl or phenyl-C₁₋₃-alkyl, in which the phenyl group optionally may be substituted,
d) C₂₋₈-alkenyl, C₃₋₈-cycloalkenyl, or C₂₋₈-alkynyl, which optionally may be substituted by phenyl, or
R^{1b} selected from
a) C₁₋₆-alkylthio, C₃₋₆-cycloalkylthio, C₁₋₆-alkoxy, C₃₋₆-cycloalkoxy, mono-C₁₋₆-alkylamino, or mono-C₃₋₆-cycloalkylamino, in which an alkyl group optionally may be substituted by phenyl or by one or more fluoro substituents,
b) phenylthio or phenoxy, in which the phenyl group optionally may be substituted,
c) di-C₁₋₄-alkylamino, in which the alkyl groups may be the same or different or together form a polymethylene ring with three, four, five, or six carbon atoms, which optionally may be interrupted by an oxygen atom and optionally may be substituted by one or more fluoro substituents,
d) mono-phenylamino or mono-C₁₋₄-alkyl-monophenylamino, in which the phenyl group optionally may be substituted and the alkyl groups optionally may be substituted by one or more fluoro substituents, or
e) halo,
R² is
a) hydrogen,
b) C₁₋₈-alkyl, which optionally may be substituted by one or more fluoro substituents,
c) optionally substituted phenyl,
R¹ and R² together form a polymethylene chain containing three, four or five carbon atoms, which optionally may be interrupted by an oxygen or sulfur atom,
R³ is hydrogen or C₁₋₄-alkyl, and n is 0 or 1,
X is O, S, or NR⁴, and
R⁴ is
a) hydrogen,
b) C₁₋₈-alkyl, C₃₋₈-cycloalkyl, C₄₋₈-cycloalkylalkyl, or C₄₋₈-alkylcycloalkyl, which optionally may be substituted by phenyl or by one or more fluoro substituents,
c) optionally substituted phenyl,
d) (CH₂)ₘCOOR²²,
e) (CH₂)ₘCONR²³R²⁴,
f) (CH₂)ₘCOOP¹, in which P¹ is a carboxy-protecting group,
g) (CH₂)ₘCN,
h) (CH₂)ₘ(5-tetrazolyl),
and m is 1 or 2 in groups d), e), f), g) or h)
= A―B- together with the pyrimidine ring forms
a) a pyrazolo[1,5-a]pyrimidine of formula (A), 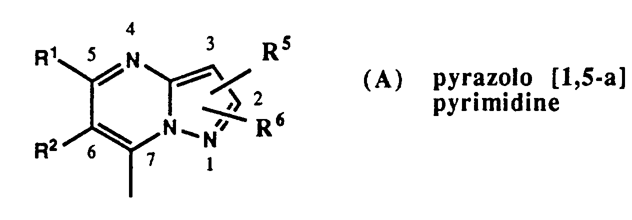
b) a [1,2,4]triazolo[1,5-a]pyrimidine of formula (B), 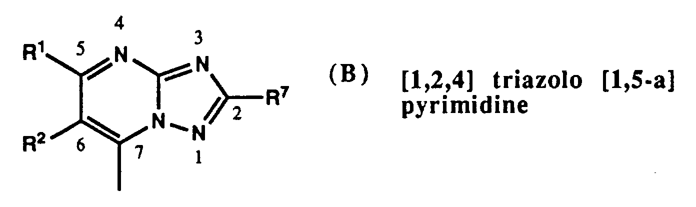
c) an imidazo[1,5-a]pyrimidine of formula (C), 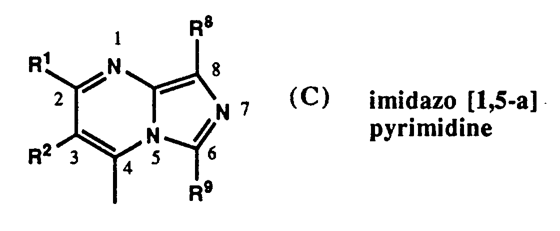
or d) an imidazo[1,2-a]pyrimidine of formula (D), 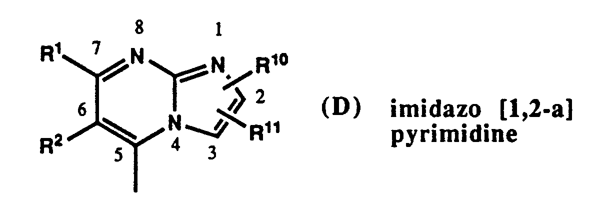 in which
R⁵ is
a) hydrogen,
b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents,
c) phenyl-C₁₋₃-alkyl,
d) hydroxy, C₁₋₆-alkoxy, C₃₋₆-cycloalkoxy, C₁₋₃-phenylalkoxy, or phenoxy, in which the phenyl groups are optionally substituted and the alkyl and cycloalkyl groups optionally are substituted by one or more fluorine atoms,
e) halo,
f) mercapto,
g) C₁₋₆-alkylthio, C₁₋₆-alkylsulfinyl, C₁₋₆-alkylsulfonyl, C₃₋₆-cycloalkylthio, C₃₋₆-cycloalkylsulfinyl, or C₃₋₆-cycloalkylsulfonyl, which optionally may be substituted by one or more fluorine atoms,
h) phenylthio, phenylsulfinyl, phenylsulfonyl, phenyl-C₁₋₃-alkylthio, phenyl-C₁₋₃-alkylsulfinyl, or phenyl-C₁₋₃-sulfonyl, in which the phenyl groups optionally may be substituted,
i) optionally substituted phenyl,
j) cyano,
k) COOR²²,
l) CONR²³R²⁴,
m) 5-tetrazolyl,
n) COOP¹, in which P¹ is a carboxy-protecting group,
o) SO₃H,
p) SO₂NR²³R²⁴,
q) nitro or nitroso, with the proviso that these groups are not connected to C-2 of the heterocycle,
r) NR²³R²⁴,
s) C₁₋₆-alkanoyl or 1-hydroxy-C₁₋₆-alkyl, which optionally may be substituted by one or more fluorine atoms,
t) benzoyl or phenylhydroxymethyl, in which the phenyl group optionally may be substituted,
u) NH(C₁₋₆-alkanoyl) or NH(C₁₋₆-alkylsulfonyl), in which the alkyl groups optionally may be substituted by one or more fluorine atoms,
v) NH(benzoyl) or NH(benzenesulfonyl), in which the phenyl group optionally may be substituted,
R⁶ is
a) hydrogen,
b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents,
c) halo,
d) optionally substituted phenyl,
e) C₁₋₆-alkylthio, C₁₋₆-alkylsulfinyl, C₁₋₆-alkylsulfonyl, C₃₋₆-cycloalkylthio, C₃₋₆-cycloalkylsulfinyl, or C₃₋₆-cycloalkylsulfonyl, which optionally may be substituted by one or more fluorine atoms,
R⁵ and R⁶ together may form a polymethylene chain containing three, four, or five carbon atoms,
R⁷ has the meaning as defined for R⁵ with the exception of nitro and nitroso,
R⁸ is
a) hydrogen,
b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents,
c) halo,
d) optionally substituted phenyl,
e) nitro,
f) cyano,
g) 5-tetrazolyl,
h) COOR²²,
i) CONR²³R²⁴,
j) COOP¹, in which P¹ is a carboxy-protecting group,
k) NR²³R²⁴,
l) NH(C₁₋₆-alkanoyl) or NH(C₁₋₆-alkylsulfonyl), in which the alkyl groups optionally may be substituted by one or more fluorine atoms,
m) NH(benzoyl) or NH(benzenesulfonyl), in which the phenyl group optionally may be substituted,
R⁹ is
a) hydrogen,
b) C₁₋₈-alkyl, C₃₋₈-cycloalkyl, C₄₋₈-cycloalkylalkyl, or C₄₋₈-alkylcycloalkyl, which optionally may be substituted by one or more fluoro or chloro substituents,
c) an optionally substituted phenyl group,
d) cyano,
ₑ) COOR²²,
f) CONR²³R²⁴,
g) 5-tetrazolyl,
h) COOP¹, in which P¹ is a carboxy-protecting group,
i) formyl,
j) hydroxymethyl,
R¹⁰ has independently the same meaning as R⁵,
R¹¹ has independently the same meaning as R⁶,
Ar¹ is a group selected from
a) 1,4-phenylene of formula (E), 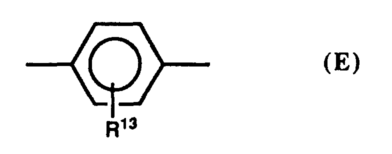
b) 1,4-substituted pyridine of formula (F) or formula (G), 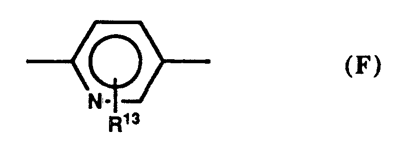 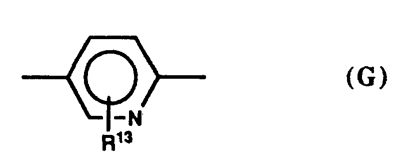
or c) benzofuran, benzothiophene, or indole of formula (H), 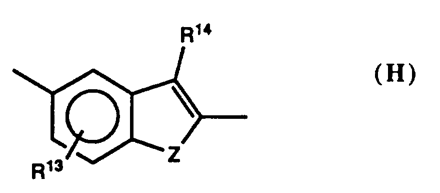 in which the group Z is O, S, or NR¹², and R¹² is hydrogen or C₁₋₄-alkyl,
and in each of the groups Ar¹ the substituent
R¹³ is
a) hydrogen,
b) halo,
c) C₁₋₄-alkyl,
d) C₁₋₄-alkoxy,
e) trifluoromethyl,
f) nitro,
R¹⁴ is
a) hydrogen,
b) C₁₋₆-alkyl, C₃₋₆-cycloalkyl, C₄₋₆-cycloalkylalkyl, or C₄₋₆-alkylcycloalkyl, which optionally may be substituted by one or more fluoro or chloro substituents,
c) C₂₋₆-alkenyl or C₃₋₆-cycloalkenyl,
d) halo,
e) cyano,
f) nitro,
g) C₁₋₆-alkanoyl, in which the alkyl group optionally may be substituted by one or more fluorine atoms,
h) C₁₋₆-alkoxy,
i) COOR²²,
j) CONR²³R²⁴,
Ar² is a group selected from
a) phenyl of formula (I), 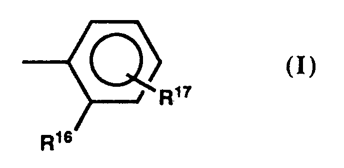
b) pyridine of formula (J), 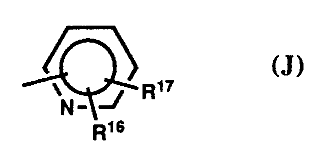
c) 1-pyrrolyl of formula (K), 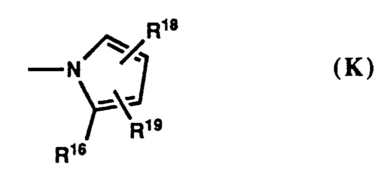
or d) a five-membered heterocycle of formula (L), 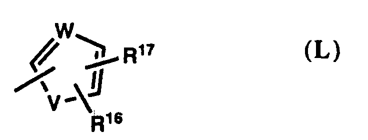 in which the group V is O, S, SO, SO₂, or NR¹⁵, the group W is CH or N, and R¹⁵ is hydrogen or C₁₋₄-alkyl,
with the proviso that in groups Ar² of formula (J) and (L) the substituent R¹⁶ and the group Y are in ortho positions, and in each of the groups Ar² the substituent
R¹⁶ is hydrogen, an acidic group,
COOP¹, in which P¹ is a carboxy-protecting group, or
a group selected from
a) cyano,
b) a protected 5-tetrazolyl of formula (M), 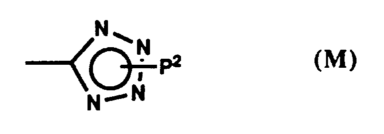 in which the group P² is a protecting group,
c) COO(C₁₋₄-alkyl),
d) nitro,
e) amino,
f) mercapto,
g) SO₂Cl,
h) SO₂(OC₁₋₄-alkyl),
i) PO(OC₁₋₄-alkyl)₂,
R¹⁷ has independently the same meaning as R¹³,
R¹⁸ and R¹⁹ are independently selected from
a) hydrogen,
b) C₁₋₆-alkyl, C₃₋₆-cycloalkyl, C₄₋₆-cycloalkylalkyl, or C₄₋₆-alkylcycloalkyl, which optionally may be substituted by one or more fluoro or chloro substituents,
c) C₂₋₆-alkenyl or C₃₋₆-cycloalkenyl,
d) halo,
e) nitro,
f) cyano,
g) C₁₋₄-alkylthio,
Y is a group selected from
a) C-C single bond, CHR²⁰, CHR²⁰CH₂, OCHR²⁰, OCHR²⁰CH₂, SCHR²⁰, SCHR²⁰CH₂, NR²¹CHR²⁰, NR²¹CHR²⁰CH₂, CH₂CHR²⁰, CH₂CHR²⁰CH₂,
b) O, S, SO₂, NR²¹, CO, CONH, NHCO, CH₂O, CH₂S, CH₂NR²¹, with the proviso that when Y is (b) Ar¹ is 1,4-phenylene of formula (E) and Ar² is phenyl of formula (I),
R²⁰ is hydrogen or
a) COOH,
b) COOP¹, in which P¹ is a carboxy-protecting group,
c) COO(C₁₋₄-alkyl),
d) 5-tetrazolyl,
e) cyano,
f) a protected 5-tetrazolyl of formula (M),
with the proviso that one of the substituents R¹⁶ and R²⁰ is hydrogen and the other is a substituent other than hydrogen,
R²¹ and R²² are independently selected from hydrogen or C₁₋₆-alkyl, and
R²³ and R²⁴ are independently selected from hydrogen or C₁₋₄-alkyl, or together may form a polymethylene chain containing three, four or five carbon atoms, which optionally may be interrupted by an oxygen atom;
whereby an optionally substituted phenyl is a phenyl group, which is unsubstituted or may be preferably substituted by one or more halo such as fluoro, chloro, bromo or iodo, C₁₋₄-alkyl, C₁₋₄-alkoxy, C₁₋₄-alkylthio, C₁₋₄-alkylsulfinyl, C₁₋₄-alkylsulfonyl, cyano, nitro, trifluoromethyl, or hydroxy, and includes for example, phenyl, 2-methylphenyl, 3-methylphenyl, 4-methylphenyl, 2,4-dimethylphenyl, 4-ethylphenyl, 2-methoxyphenyl, 3-methoxyphenyl, 4-methoxyphenyl, 2,4-dimethoxyphenyl, 2-chlorophenyl, 3-chlorophenyl, 4-chlorophenyl, 2,4-dichlorophenyl, 3,5-dichlorophenyl, 4-fluorophenyl, pentafluorophenyl, 4-bromophenyl, 4-methylthiophenyl, 4-trifluoromethylphenyl, 4-cyanophenyl, 4-nitrophenyl, 4-hydroxyphenyl;
or a salt thereof.

2. A compound according to claim 1, in which R¹⁶ is an acidic substituent or a group COOP¹, and R²⁰ is COOH, 5-tetrazolyl or a group COOP¹.

3. A compound according to claim 2 in which R¹ is hydrogen, C₁₋₆ alkyl or C₃₋ ₆ cycloalkyl.

4. A compound according to claim 3 in which R¹ is Me or Et.

5. A compound according to any of claims 2 to 4, in which R² is hydrogen.

6. A compound according to any of claims 2 to 5, in which -X-(CHR³)ₙ- is -NHCH₂-, -NR⁴CH₂-, -NH-, or -SCH₂-.

7. A compound according to claim 5 in which -X-(CHR³)ₙ is -NR⁴CH₂- and R⁴ is H, Me or CH₂COOH.

8. A compound according to any of claims 2 to 7, in which the group = A―B- together with the pyrimidine ring form a pyrazolo [1, 5 - a] pyrimidine, imidazo [1, 5 - a] pyrimidine or imidazo [1, 2 - a] pyrimidine.

9. A compound according to any of claims 2 to 8 in which Ar¹ is 1, 4-phenylene.

10. A compound according to any of claims 2 to 8 in which Ar¹ is 3-bromobenzofuran of formula (H).

11. A compound according to any of claims 2 to 10, in which Y is a C-C single bond.

12. A compound according to any of claims 2 to 11, in which Ar² is phenyl, pyrrol-1-yl or 3 - thienyl.

13. A pharmaceutical formulation comprising a compound according to any of claims 2 to 12, or a pharmaceutically-acceptable salt thereof, together with a pharmaceutically-acceptable diluent or carrier therefor.

14. A compound according to any of claims 2 to 12, or a pharmaceutically-acceptable salt thereof, for use as a pharmaceutical.

## Patentansprüche

1. Eine Verbindung der Formel 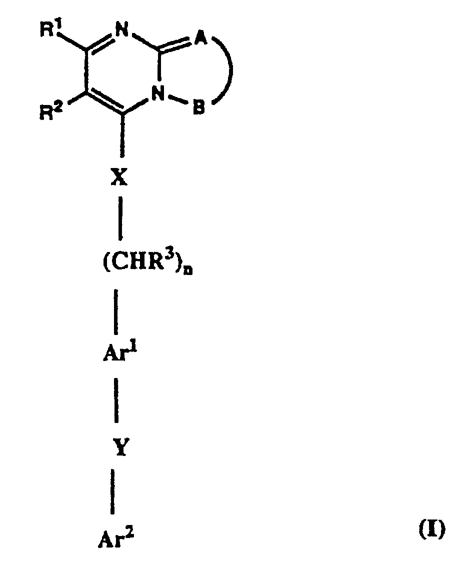 in welcher
R¹ entweder R^{1a} ist, ausgewählt aus
a) Wasserstoff,
b) C₁₋₈-Alkyl, C₃₋₈-Cycloalkyl, C₄₋₈-Cycloalkylalkyl, oder C₄₋₈-Alkylcycloalkyl, welche gegebenenfalls durch einen oder mehrere Fluor- oder Chlorsubstituenten substituiert sein können, oder durch ein einziges Hydroxy, C₁₋₄-Alkoxy, oder C₁₋₄-Alkylthio,
c) Phenyl oder Phenyl-C₁₋₃-alkyl, in welchen die Phenylgruppe gegebenenfalls substituiert sein kann
d) C₂₋₈-Alkenyl, C₃₋₈-Cycloalkenyl, oder C₂₋₈-Alkinyl, welche gegebenenfalls durch Phenyl substituiert sein können, oder
R^{1b} ausgewählt aus
a) C₁₋₆-Alkylthio, C₃₋₆-Cycloalkylthio, C₁₋₆-Alkoxy, C₃₋₆-Cycloalkoxy, mono-C₁₋₆ Alkylamino oder mono-C₃₋₆-Cycloalkylamino, in welchen eine Alkylgruppe gegebenenfalls durch Phenyl oder durch eine oder mehrere Fluorsubstituenten substituiert sein kann.
b) Phenylthio oder Phenoxy, in welchen die Phenylgruppe gegebenenfalls substituiert sein kann,
c) di-C₁₋₄-Alkylamino, in welchem die Alkylgruppen gleich oder verschieden sein können, oder zusammen einen Polymethylenring mit drei, vier, fünf oder sechs Kohlenstoffatomen ausbilden können, welcher gegebenenfalls durch einen Sauerstoffatom unterbrochen und gegebenenfalls durch einen oder mehrere Fluorsubstituenten substituiert sein kann,
d) mono-Phenylamino oder mono-C₁₋₄-Alkylmonophenylamino, in welchen die Phenylgruppe gegebenenfalls substituiert sein kann und die Alkylgruppen gegebenenfalls durch einen oder mehrere Fluorsubstituenten substituiert sein können, oder
e) Halogen,
R² ist
a) Wasserstoff,
b) C₁₋₈-Alkyl, welches gegebenenfalls durch einen oder mehrere Fluorsubstituenten substituiert sein kann,
c) gegebenenfalls substituiertes Phenyl,
R¹ und R² zusammen eine Polymethylenkette ausbilden, die drei, vier oder fünf Kohlenstoffatome enthält und gegebenenfalls durch ein Sauerstoff- oder Schwefelatom unterbrochen sein kann,
R³ ist Wasserstoff oder C₁₋₄-Alkyl und n ist 0 oder 1,
X ist O, S oder NR⁴, und
R⁴ ist
a) Wasserstoff,
b) C₁₋₈-Alkyl, C₃₋₈-Cycloalkyl, C₄₋₈-Cycloalkylalkyl oder C₄₋₈-Alkylcycloalkyl ist, welche gegebenenfalls durch Phenyl oder durch einen oder mehrere Fluorsubstituenten substituiert sein können,
c) gegebenenfalls substituiertes Phenyl,
d) (CH₂)ₘCOOR²²,
e) (CH₂)ₘCONR²³R²⁴,
f) (CH₂)ₘCOOP¹, in welchem P¹ eine Carboxyschutzgruppe ist,
g) (CH₂)ₘCN,
h) (CH₂)ₘ(5-tetrazolyl) ist,
und m in den Gruppen d), e), f), g) oder h) 1 oder 2 ist.
=A―B- zusammen mit dem Pyrimidinring bildet
a) ein Pyrazolo[1,5-a]pyrimidin der Formel (A), 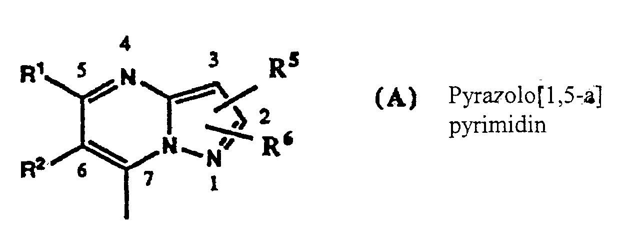
b) ein [1,2,4]Triazolo[1,5-a]pyrimidin der Formel (B), 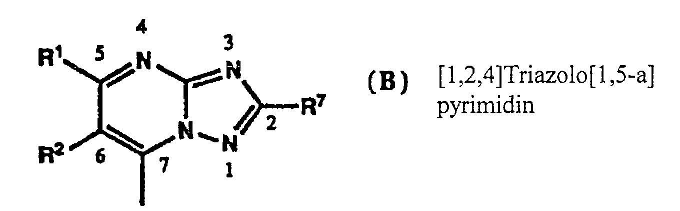
c) ein Imidazo[1,5-a]pyrimidin der Formel (C), 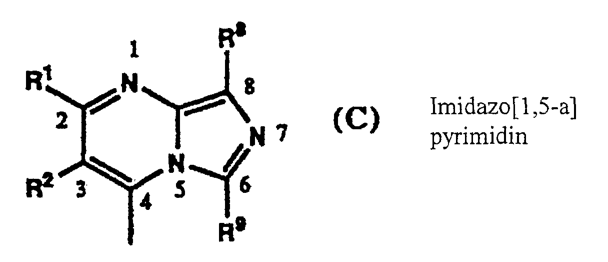
oder d) ein Imidazo[1,2-a]pyrimidin der Formel (D), 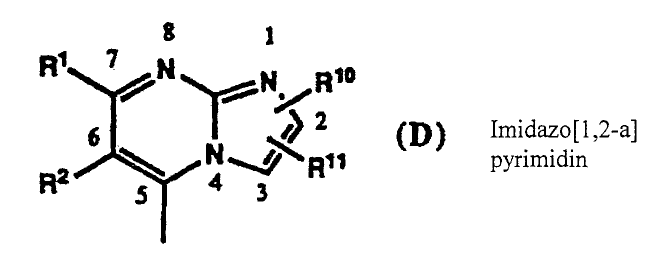
in welchen
R⁵ ist
a) Wasserstoff,
b) C₁₋₈-Alkyl, C₃₋₈-Cycloalkyl, C₄₋₈-Cycloalkylalkyl oder C₄₋₈-Alkylcycloalkyl, welche gegebenenfalls durch einen oder mehrere Fluor- oder Chlorsubstituenten substituiert sein können,
c) Phenyl-C₁₋₃-alkyl,
d) Hydroxy, C₁₋₆-Alkoxy, C₃₋₆-Cycloalkoxy, C₁₋₃-Phenylalkoxy oder Phenoxy, in welchen die Phenylgruppen gegebenenfalls substituiert und die Alkyl- und Cycloalkylgruppen gegebenenfalls durch ein oder mehrere Fluoratome substituiert sind,
e) Halogen,
f) Mercapto,
g) C₁₋₆-Alkylthio, C₁₋₆-Alkylsulfinyl, C₁₋₆-Alkylsulfonyl, C₃₋₆-Cycloalkylthio, C₃₋₆-Cycloalkylsulfinyl oder C₃₋₆-Cycloalkylsulfonyl, welche gegebenenfalls durch ein oder mehrere Fluoratome substituiert sein können,
h) Phenylthio, Phenylsulfinyl, Phenylsulfonyl, Phenyl-C₁₋₃-alkylthio, Phenyl-C₁₋₃-alkylsulfinyl oder Phenyl-C₁₋₃-sulfonyl, in welchen die Phenylgruppen gegebenenfalls substituiert sein können,
i) gegebenenfalls substituiertes Phenyl,
j) Cyano,
k) COOR²²,
l) CONR²³R²⁴,
m) 5-Tetrazolyl,
n) COOP¹, worin P¹ eine Carboxy- schützende Gruppe ist,
o) SO₃H,
p) SO₂NR²³R²⁴,
q) Nitro oder Nitroso, mit der Maßgabe, dass diese Gruppen nicht an C-2 des Heterocyclus gebunden sind,
r) NR²³R²⁴,
s) C₁₋₆-Alkanoyl oder 1-Hydroxy-C₁₋₆-alkyl, welche gegebenenfalls durch ein oder mehrere Fluoratome substituiert sein können,
t) Benzoyl oder Phenylhydroxymethyl, in welchen die Phenylgruppe gegebenenfalls substituiert sein kann,
u) NH(C₁₋₆-alkanoyl) oder NH(C₁₋₆-alkylsulfonyl), in welchen die Alkylgruppen gegebenenfalls durch ein oder mehrere Fluoratome substituiert sein können,
v) NH(benzoyl) oder NH(benzolsulfonyl), in welchen die Phenylgruppe gegebenenfalls substituiert sein kann,
R⁶ ist
a) Wasserstoff,
b) C₁₋₈-Alkyl, C₃₋₈-Cycloalkyl, C₄₋₈-Cycloalkylalkyl oder C₄₋₈-Alkylcycloalkyl, welche gegebenenfalls durch ein oder mehrere Fluor- oder Chlorsubstituenten substituiert sein können,
c) Halogen,
d) gegebenenfalls substituiertes Phenyl,
e) C₁₋₆-Alkylthio, C₁₋₆-Alkylsulfinyl, C₁₋₆-Alkylsulfonyl, C₃₋₆-Cycloalkylthio, C₃₋₆-Cycloalkylsulfinyl oder C₃₋₆-Cycloalkylsulfonyl, welche gegebenenfalls durch ein oder mehrere Fluoratome substituiert sein können,
R⁵ und R⁶ zusammen eine Polymethylenkette ausbilden können, die drei, vier oder fünf Kohlenstoffatome enthält,
R⁷ die für R⁵ definierte Bedeutung mit der Ausnahme von Nitro und Nitroso hat,
R⁸ ist
a) Wasserstoff,
b) C₁₋₈-Alkyl, C₃₋₈-Cycloalkyl, C₄₋₈-Cycloalkylalkyl oder C₄₋₈-Alkylcycloalkyl, welche gegebenenfalls durch einen oder mehrere Fluor- oder Chlorsubstituenten substituiert sein können,
c) Halogen,
d) gegebenenfalls substituiertes Phenyl,
e) Nitro,
f) Cyano,
g) 5-Tetrazolyl,
h) COOR²²,
i) CONR²³R²⁴,
j) COOP¹, worin P¹ eine Carboxy-Schutzgruppe ist,
k) NR²³R²⁴,
l) NH(C₁₋₆-alkanoyl) oder NH(C₁₋₆-alkylsulfonyl), in welchen die Alkylgruppen gegebenenfalls durch eine oder mehrere Fluoratome substituiert sein können,
m) NH(benzoyl) oder NH(benzolsulfonyl), in welchen die Phenylgruppe gegebenenfalls substituiert sein kann,
R⁹ ist
a) Wasserstoff,
b) C₁₋₈-Alkyl, C₃₋₈-Cycloalkyl, C₄₋₈-Cycloalkylalkyl oder C₄₋₈-Alkylcycloalkyl, welche gegebenenfalls durch einen oder mehrere Fluor- oder Chlorsubstituenten substituiert sein können,
c) eine gegebenenfalls substituierte Phenylgruppe,
d) Cyano,
e) COOR²²,
f) CONR²³R²⁴,
g) 5-Tetrazolyl,
h) COOP¹, worin P¹ eine Carboxy-Schutzgruppe ist,
i) Formyl,
j) Hydroxymethyl,
R¹⁰ unabhängig dieselbe Bedeutung wie R⁵ hat,
R¹¹ unabhängig dieselbe Bedeutung wie R⁶ hat,
Ar¹ ist eine Gruppe ausgewählt aus
a) 1,4-Phenylen der Formel (E), 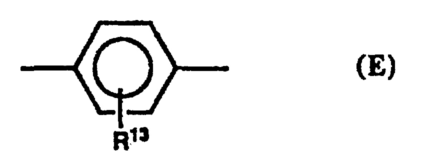
b) 1,4-substituiertes Pyridin der Formel (F) oder Formel (G), 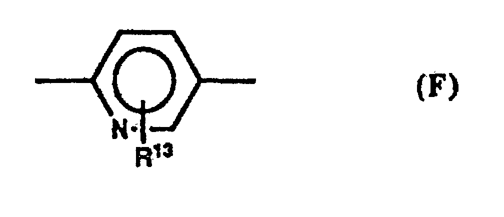 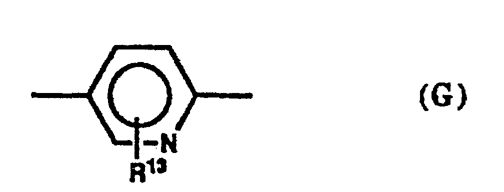
oder c) Benzofuran, Benzothiophen oder Indol der Formel (H), 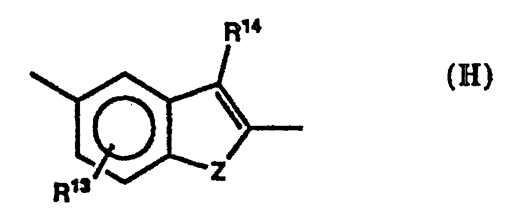 in welcher die Gruppe Z O, S, oder NR¹² ist, und R¹² Wasserstoff oder C₁₋₄-Alkyl ist, und jeder der Gruppen Ar¹ der Substituent
R¹³ ist
a) Wasserstoff,
b) Halogen,
c) C₁₋₄-Alkyl,
d) C₁₋₄-Alkoxy,
e) Trifluormethyl,
f) Nitro,
R¹⁴ ist
a) Wasserstoff,
b) C₁₋₆-Alkyl, C₃₋₆-Cycloalkyl, C₄₋₆-Cycloalkylalkyl oder C₄₋₆-Alkylcycloalkyl, welche gegebenenfalls durch eine oder mehrere Fluor- oder Chlorsubstituenten substituiert sein können,
c) C₂₋₆-Alkenyl oder C₃₋₆-Cycloalkenyl,
d) Halogen,
e) Cyano,
f) Nitro,
g) C₁₋₆-Alkanoyl, in welchem die Alkylgruppe gegebenenfalls durch ein oder mehrere Fluoratome substituiert sein kann,
h) C₁₋₆-Alkoxy,
i) COOR²²,
j) CONR²³R²⁴,
Ar² ist eine Gruppe ausgewählt aus
a) Phenyl der Formel (I), 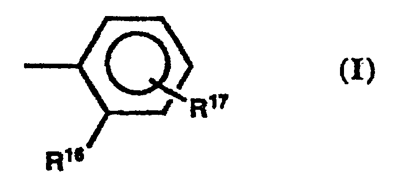
b) Pyridin der Formel (J), 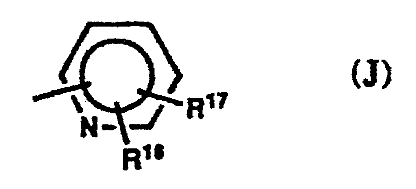
c) 1-Pyrrolyl der Formel (K), 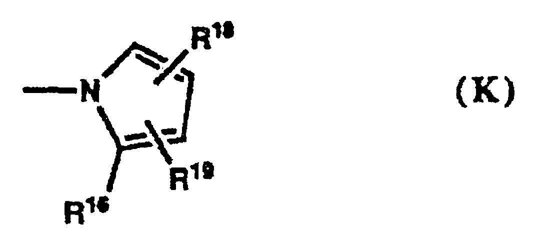
oder d) ein fünf-gliedriger Heterocyclus der Formel (L), 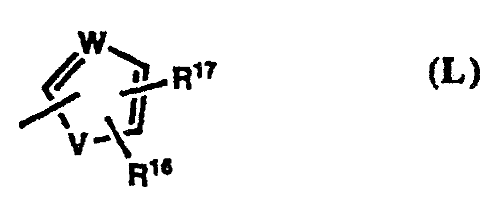
in welchen die Gruppe V ist O, S, SO, SO₂ oder NR¹⁵, die Gruppe W ist CH oder N und
R¹⁵ ist Wasserstoff oder C₁₋₄-Alkyl,
mit der Maßgabe, dass in den Gruppen Ar² der Formeln (J) und (L) die Substituenten R¹⁶ und die Gruppe Y in ortho-Stellungen sind, und in jeder der Gruppen Ar² der Substituent
R¹⁶ ist Wasserstoff, eine saure Gruppe,
COOP¹, in welcher P¹ eine Carboxy-Schutzgruppe ist, oder
eine Gruppe ausgewählt aus
a) Cyano,
b) ein geschütztes 5-Tetrazolyl der Formel (M), 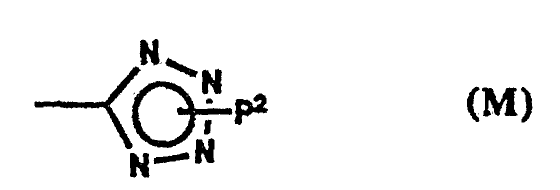 in welcher die Gruppe P² eine Schutzgruppe ist,
c) COO(C₁₋₄-alkyl),
d) Nitro,
e) Amino,
f) Mercapto,
g) SO₂Cl,
h) SO₂(OC₁₋₄-alkyl),
i) PO(OC₁₋₄-alkyl)₂,
R¹⁷ unabhängig dieselbe Bedeutung wie R¹³ hat,
R¹⁸ und R¹⁹ unabhängig ausgewählt sind aus
a) Wasserstoff,
b) C₁₋₆-Alkyl, C₃₋₆-Cycloalkyl, C₄₋₆-Cycloalkylalkyl oder C₄₋₆-Alkylcycloalkyl, welche gegebenenfalls durch einen oder mehrere Fluor- oder Chlorsubstituenten substituiert sein können,
c) C₂₋₆-Alkenyl oder C₃₋₆-Cycloalkenyl,
d) Halogen,
e) Nitro,
f) Cyano,
g) C₁₋₄-Alkylthio,
Y ist eine Gruppe die ausgewählt ist aus
a) C-C Einfachbindung, CHR²⁰, CHR²⁰CH₂, OCHR²⁰, OCHR²⁰CH₂, SCHR²⁰, SCHR²⁰CH₂, NR²¹CHR²⁰, NR²¹CHR²⁰CH₂, CH₂CHR²⁰, CH₂CHR²⁰CH₂,
b) O, S, SO₂, NR²¹, CO, CONH, NHCO, CH₂O, CH₂S, CH₂NR²¹, mit der Maßgabe, dass wenn Y (b) ist, Ar¹ 1,4-Phenylen von Formel (E) ist und Ar² Phenyl von Formel (I) ist,
R²⁰ ist Wasserstoff oder
a) COOH,
b) COOP¹, in welchem P¹ eine Carboxy-Schutzgruppe ist,
c) COO(C₁₋₄-Alkyl),
d) 5-Tetrazolyl,
e) Cyano,
f) ein geschütztes 5-Tetrazolyl der Formel (M), mit der Maßgabe, dass einer der Substituenten R¹⁶ und R²⁰ Wasserstoff ist und der andere ein anderer Substituent als Wasserstoff ist,
R²¹ und R²² unabhängig ausgewählt sind aus Wasserstoff oder C₁₋₆-Alkyl, und
R²³ und R²⁴ unabhängig ausgewählt sind aus Wasserstoff oder C₁₋₄-Alkyl oder zusammen eine Polymethylen-Kette bilden können, die drei, vier oder fünf Kohlenstoffatome enthält, welche gegebenenfalls durch ein Sauerstoffatom unterbrochen sein kann, wobei ein gegebenenfalls substituiertes Phenyl eine Phenylgruppe ist, welche unsubstituiert ist oder bevorzugt substituiert sein kann durch ein oder mehrere Halogene, wie Fluor, Chlor, Brom oder Jod, C₁₋₄-Alkyl, C₁₋₄-Alkoxy, C₁₋₄-Alkylthio, C₁₋₄-Alkylsulfinyl, C₁₋₄-Alkylsulfonyl, Cyano, Nitro, Trifluormethyl, oder Hydroxy, und schließt zum Beispiel ein Phenyl, 2-Methylphenyl, 3-Methylphenyl, 4-Methylphenyl, 2,4-Dimethylphenyl, 4-Ethylphenyl, 2-Methoxyphenyl, 3-Methoxyphenyl, 4-Methoxyphenyl, 2,4-Dimethoxyphenyl, 2-Chlorphenyl, 3-Chlorphenyl, 4-Chlorphenyl, 2,4-Dichlorphenyl, 3,5-Dichlorphenyl, 4-Fluorphenyl, Pentafluorphenyl, 4-Bromphenyl, 4-Methylthiophenyl, 4-Trifluormethylphenyl, 4-Cyanophenyl, 4-Nitrophenyl, 4-Hydroxyphenyl; oder deren Salze.

2. Eine Verbindung gemäß Anspruch 1, in welcher R¹⁶ ein saurer Substituent oder eine Gruppe COOP¹ ist, und R²⁰ COOH, 5-Tetrazolyl oder eine Gruppe COOP¹ ist.

3. Eine Verbindung gemäß Anspruch 2, in welcher R¹ Wasserstoff, C₁₋₆-Alkyl oder C₃₋₆-Cycloalkyl ist.

4. Eine Verbindung gemäß Anspruch 3, in welcher R¹ Me oder Et ist.

5. Eine Verbindung gemäß jedem der Ansprüche 2 bis 4, in welcher R² Wasserstoff ist.

6. Eine Verbindung gemäß jedem der Ansprüche 2 bis 5, in welcher -X-(CHR³)ₙ ist -NHCH₂-, -NR⁴CH₂-, -NH-, oder -SCH₂-.

7. Eine Verbindung gemäß Anspruch 5, in welcher -X-(CHR³)ₙ ist -NR⁴CH₂- und R⁴ ist H, Me oder CH₂COOH.

8. Eine Verbindung gemäß jedem der Ansprüche 2 bis 7, in welcher die Gruppe =A―B-zusammen mit dem Pyrimidinring ein Pyrazolo[1,5-a]pyrimidin, Imidazo[1,5-a]pyrimidin oder Imidazo[1,2-a]pyrimidin ausbildet.

9. Eine Verbindung gemäß jedem der Ansprüche 2 bis 8, in welcher Ar¹ 1,4-Phenylen ist.

10. Eine Verbindung gemäß jedem der Ansprüche 2 bis 8, in welcher Ar¹ 3-Brombenzofüran der Formel (H) ist.

11. Eine Verbindung gemäß jedem der Ansprüche 2 bis 10, in welcher Y eine C-C Einfachbindung ist.

12. Eine Verbindung gemäß jedem der Ansprüche 2 bis 11, in welcher Ar² Phenyl, Pyrrol-1-yl oder 3-Thienyl ist.

13. Eine Arzneimittel-Formulierung umfassend eine Verbindung gemäß einem der Ansprüche 2 bis 12 oder deren pharmazeutisch verträgliches Salz zusammen mit einem pharmazeutisch verträglichen Verdünnungsmittel oder Trägerstoff dafür.

14. Eine Verbindung gemäß jedem der Ansprüche 2 bis 12 oder deren pharmazeutisch verträgliches Salz zur Verwendung als Arzneimittel.

## Revendications

1. Composé de formule 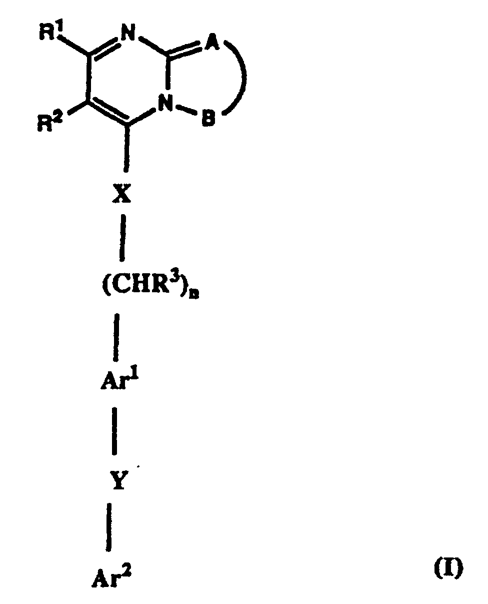 dans laquelle
R¹ est soit R^{1a} choisi parmi
a) un atome d'hydrogène,
b) un groupe alkyle en C₁₋₈, un groupe cycloalkyle en C₃₋₈, un groupe (cycloalkyle en C₄₋₈)alkyle ou un groupe (alkyle en C₄₋₈)cycloalkyle, qui peuvent éventuellement être substitués par un ou plusieurs substituants fluoro ou chloro, ou par un seul groupe hydroxy, un seul groupe alcoxy en C₁₋₄ ou un seul groupe (alkyle en C₁₋₄)thio,
c) un groupe phényle ou un groupe phényl-(alkyle en C₁₋₃), dans lesquels le groupe phényle peut éventuellement être substitué,
d) un groupe alcényle en C₂₋₈, un groupe cycloalcényle en C₃₋₈ ou un groupe alcynyle en C₂₋₈, qui peuvent éventuellement être substitués par un groupe phényle
soit R^{1b} choisi parmi
a) un groupe (alkyle en C₁₋₆)thio, un groupe (cycloalkyle en C₃₋₆)thio, un groupe alcoxy en C₁₋₆, un groupe cycloalcoxy en C₃₋₆, un groupe mono(alkyle en C₁₋₆)amino ou un groupe mono(cycloalkyle en C₃₋₆)amino, dans lesquels un groupe alkyle peut éventuellement être substitué par un groupe phényle ou par un ou plusieurs substituants fluoro
b) un groupe phénylthio ou phénoxy, dans lesquels le groupe phényle peut éventuellement être substitué,
c) un groupe di-(alkyle en C₁₋₄)amino, dans lequel les groupes alkyle peuvent être identiques ou différents ou former ensemble un cycle polyméthylène comprenant trois, quatre, cinq ou six atomes de carbone, qui peut éventuellement être interrompu par un atome d'oxygène et qui peut éventuellement être substitué par un ou plusieurs substituants fluoro
d) un groupe monophénylamino ou mono(alkyle en C₁₋₄)monophénylamino, dans lesquels le groupe phényle peut éventuellement être substitué et les groupes alkyle peuvent éventuellement être substitués par un ou plusieurs substituants fluoro ou
e) un groupe halo,
R² est
a) un atome d'hydrogène,
b) un groupe alkyle en C₁₋₈, qui peut éventuellement être substitué par un ou plusieurs substituants fluoro
c) un groupe phényle éventuellement substitué,
R¹ et R² forment ensemble une chaîne polyméthylène contenant trois, quatre ou cinq atomes de carbone, qui peut éventuellement être interrompue par un atome d'oxygène ou de soufre,
R³ est un atome d'hydrogène ou un groupe alkyle en C₁₋₄, et n représente 0 ou 1,
X représente O, S, ou un groupe NR⁴, et
R₄ représente
a) un atome d'hydrogène,
b) un groupe alkyle en C₁₋₈, un groupe cycloalkyle en C₃₋₈, un groupe (cycloalkyle en C₄₋₈)alkyle ou un groupe (alkyle en C₄₋₈)cycloalkyle qui peuvent éventuellement être substitués par un groupe phényle ou par un ou plusieurs substituants fluoro,
c) un groupe phényle éventuellement substitué,
d) un groupe (CH₂)ₘCOOR²²,
e) un groupe (CH₂)ₘCONR²³R²⁴,
f) un groupe (CH₂)ₘCOOP¹, dans lequel P¹ est un groupe de protection du groupe carboxy,
g) un groupe (CH₂)ₘCN,
h) un groupe (CH₂)ₘ(5-tétrazolyle),
et m représente 1 ou 2 dans les groupes d), e), f), g) ou h)
=A-B- forme, avec le cycle pyrimidine
a) une pyrazolo[1,5-a]pyrimidine de formule (A) 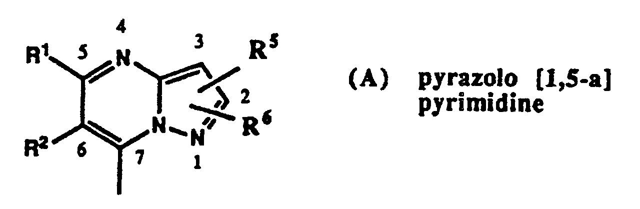
b) une [1,2,4]triazolo[1,5-a]pyrimidine de formule (B) 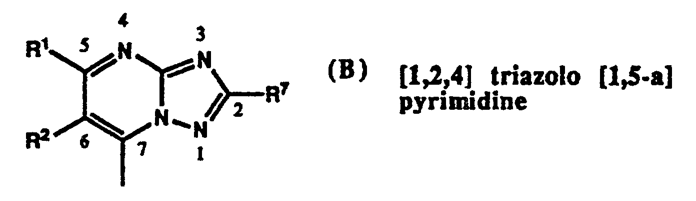
c) une imidazo[1,5-a]pyrimidine de formule (C) 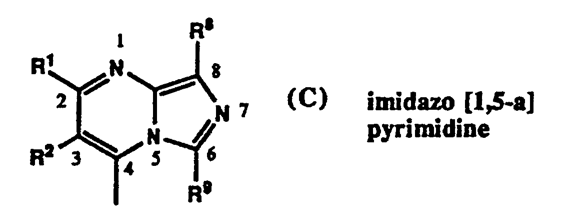
ou d) une imidazo[1,2-a]pyrimidine de formule (D) 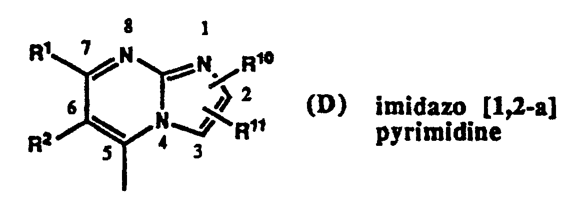 dans lesquelles
R⁵ représente
a) un atome d'hydrogène,
b) un groupe alkyle en C₁₋₈, un groupe cycloalkyle en C₃₋₈, un groupe (cycloalkyle en C₄₋₈)alkyle ou un groupe (alkyle en C₄₋₈)cycloalkyle, qui peuvent éventuellement être substitués par un ou plusieurs substituants fluoro ou chloro,
c) un groupe phényl-(alkyle en C₁₋₃),
d) un groupe hydroxy, un groupe alcoxy en C₁₋₆, un groupe cycloalcoxy en C₃₋₆, un groupe phénylalcoxy en C₁₋₃, ou un groupe phénoxy, dans lequel les groupes phényle sont éventuellement substitués et les groupes alkyle et cycloalkyle sont éventuellement substitués par un ou plusieurs atomes de fluor,
e) un groupe halo,
f) un groupe mercapto,
g) un groupe (alkyle en C₁₋₆)thio, un groupe (alkyle en C₁₋₆)sulfinyle, un groupe (alkyle en C₁₋₆)sulfonyle, un groupe (cycloalkyle en C₃₋₆)thio, un groupe (cycloalkyle en C₃₋₆)sulfinyle, un groupe (cycloalkyle en C₃₋₆)sulfonyle, qui peuvent éventuellement être substitués par un ou plusieurs atomes de fluor,
h) un groupe phénylthio, un groupe phénylsulfinyle, un groupe phénylsulfonyle, un groupe phényl(alkyle en C₁₋₃)thio, un groupe phényl(alkyle en C₁₋₃)sulfinyle ou un groupe phényl(alkyle en C₁₋₃)sulfonyle, dans lesquels les groupes phényle peuvent éventuellement être substitués,
i) un groupe phényle éventuellement substitué,
j) un groupe cyano
k) un groupe COOR²²
I) un groupe CONR²³R²⁴,
m) un groupe 5-tétrazolyle,
n) un groupe COOP¹, dans lequel P¹ est un groupe de protection du groupe carboxy
o) un groupe SO₃H
p) un groupe SO₂NR²³R²⁴,
q) un groupe nitro ou nitroso à condition que ces groupes ne soient pas fixés sur l'atome C-2 de l'hétérocycle
r) un groupe NR²³R²⁴
s) un groupe alcanoyle en C₁₋₆ ou un groupe 1-hydroxy(alkyle en C₁₋₆), qui peuvent éventuellement être substitués par un ou plusieurs atomes de fluor,
t) un groupe benzoyle ou phénylhydroxyméthyle, dans lesquels le groupe phényle peut éventuellement être substitué,
u) un groupe NH(alcanoyle en C₁₋₆) ou un groupe NH((alkyle en C₁₋₆)sulfonyle), dans lesquels les groupes alkyle peuvent éventuellement être substitués par un ou plusieurs atomes de fluor,
v) un groupe NH(benzoyle) ou un groupe NH(benzènesulfonyle), dans lesquels le groupe phényle peut éventuellement être substitué,
R⁶ représente
a) un atome d'hydrogène,
b) un groupe alkyle en C₁₋₈, un groupe cycloalkyle en C₃₋₈, un groupe (cycloalkyle en C₄₋₈)alkyle ou un groupe (alkyle en C₄₋₈)cycloalkyle, qui peuvent éventuellement être substitués par un ou plusieurs substituants fluoro ou chloro,
c) un groupe halo
d) un groupe phényle éventuellement substitué
e) un groupe (alkyle en C₁₋₆)thio, un groupe (alkyle en C₁₋₆)sulfinyle, un groupe (alkyle en C₁₋₆)sulfonyle, un groupe (cycloalkyle en C₃₋₆)thio, un groupe (cycloalkyle en C₃₋₆)sulfinyle, un groupe (cycloalkyle en C₃₋₆)sulfonyle, qui peuvent éventuellement être substitués par un ou plusieurs atomes de fluor,
R⁵ et R⁶ peuvent former ensemble une chaîne polyméthylène contenant trois, quatre ou cinq atomes de carbone,
R⁷ a la signification telle que définie pour R⁵ à l'exception du groupe nitro et du groupe nitroso,
R⁸ représente
a) un atome d'hydrogène
b) un groupe alkyle en C₁₋₈, un groupe cycloalkyle en C₃₋₈, un groupe (cycloalkyle en C₄₋₈)alkyle ou un groupe (alkyle en C₄₋₈)cycloalkyle, qui peuvent éventuellement être substitués par un ou plusieurs substituants fluoro ou chloro,
c) un groupe halo
d) un groupe phényle éventuellement substitué
e) un groupe nitro
f) un groupe cyano
g) un groupe 5-tétrazolyle
h) un groupe COOR²²,
i) un groupe CONR²³R²⁴,
j) un groupe COOP¹, dans lequel P¹ est un groupe de protection du groupe carboxy,
k) un groupe NR²³R²⁴,
l) un groupe NH(alcanoyle en C₁₋₆) ou un groupe NH((alkyle en C₁₋₆) sulfonyle), dans lesquels les groupes alkyle peuvent éventuellement être substitués par un ou plusieurs atomes de fluor,
m) un groupe NH(benzoyle) ou un groupe NH(benzènesulfonyle), dans lesquels le groupe phényle peut éventuellement être substitué,
R⁹ représente
a) un atome d'hydrogène
b) un groupe alkyle en C₁₋₈, un groupe cycloalkyle en C₃₋₈, un groupe (cycloalkyle en C₄₋₈)alkyle ou un groupe (alkyle en C₄₋₈)cycloalkyle, qui peuvent éventuellement être substitués par un ou plusieurs substituants fluoro ou chloro,
c) un groupe phényle éventuellement substitué
d) un groupe cyano
e) un groupe COOR²²,
f) un groupe CONR²³R²⁴,
g) un groupe 5-tétrazolyle,
h) un groupe COOP¹, dans lequel P¹ est un groupe de protection du groupe carboxy,
i) un groupe formyle
j) un groupe hydroxyméthyle
R¹⁰ présente indépendamment la même signification que R⁵,
R¹¹ présente indépendamment la même signification que R⁶,
Ar¹ est un groupe choisi parmi
a) un 1,4-phénylène de formule (E) 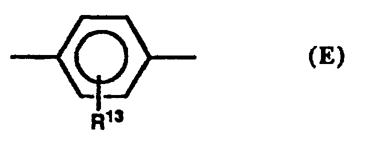
b) une pyridine substituée en positions 1,4 de formule (F) ou de formule (G) 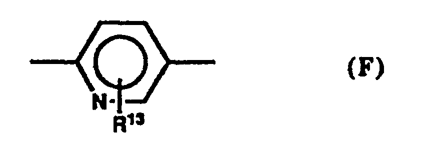 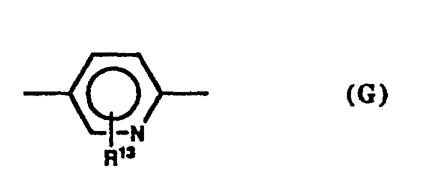
ou c) un benzofuranne, un benzothiophène ou un indole de formule (H) 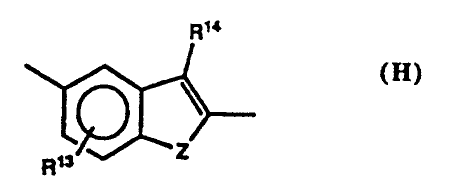 dans laquelle le groupe Z est O, S ou un groupe NR¹² et R¹² est un atome d'hydrogène ou un groupe alkyle en C₁₋₄
et dans chacun des groupes Ar¹, le substituant
R¹³ représente
a) un atome d'hydrogène
b) un groupe halo
c) un groupe alkyle en C₁₋₄,
d) un groupe alcoxy en C₁₋₄,
e) un groupe trifluorométhyle,
f) un groupe nitro,
R¹⁴ représente
a) un atome d'hydrogène
b) un groupe alkyle en C₁₋₆, un groupe cycloalkyle en C₃₋₆, un groupe (cycloalkyle en C₄₋₆)alkyle ou un groupe (alkyle en C₄₋₆)cycloalkyle, qui peuvent éventuellement être substitués par un ou plusieurs substituants fluoro ou chloro,
c) un groupe alcényle en C₂₋₆ ou un groupe cycloalcényle en C₃₋₆,
d) un groupe halo
e) un groupe cyano
f) un groupe nitro
g) un groupe alcanoyle en C₁₋₆ dans lequel le groupe alkyle peut éventuellement être substitué par un ou plusieurs atomes de fluor
h) un groupe alcoxy en C₁₋₆
i) un groupe COOR²²,
j) un groupe CONR²³R²⁴
Ar² est un groupe choisi parmi
a) un groupe phényle de formule (I) 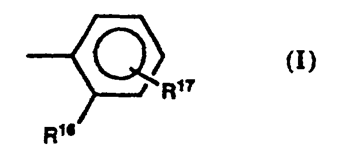
b) une pyridine de formule (J) 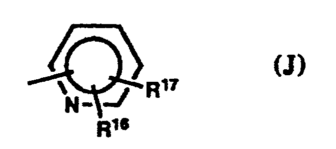
c) un groupe 1-pyrrolyle de formule (K) 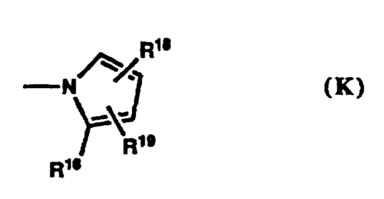
ou d) un hétérocycle à cinq membres de formule (L) 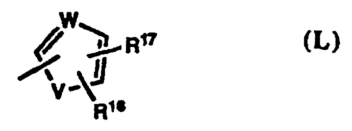 dans laquelle le groupe V représente O, S, un groupe SO, un groupe SO₂ ou un groupe NR¹⁵, le groupe W représente un groupe CH ou N et R¹⁵ représente un atome d'hydrogène ou un groupe alkyle en C₁₋₄
à condition que dans les groupes Ar² de formule (J) et (L), le substituant R¹⁶ et le groupe Y soient en position ortho et dans chacun des groupes Ar², le substituant
R¹⁶ est un atome d'hydrogène, un groupe acide
un groupe COOP¹, dans lequel P¹ est un groupe de protection du groupe carboxy ou
un groupe choisi parmi
a) un groupe cyano
b) un groupe 5-tétrazolyle protégé de formule (M) 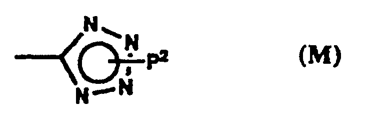 dans lequel le groupe P² est un groupe de protection
c) un groupe COO(alkyle en C₁₋₄)
d) un groupe nitro
e) un groupe amino
f) un groupe mercapto
g) un groupe SO₂Cl
h) un groupe SO₂(O-alkyle en C₁₋₄)
i) un groupe PO(O-alkyle en C₁₋₄)₂ ;
R¹⁷ présente indépendamment la même signification que R¹³
R¹⁸ et R¹⁹ sont choisis indépendamment parmi
a) un atome d'hydrogène
b) un groupe alkyle en C₁₋₆, un groupe cycloalkyle en C₃₋₆, un groupe (cycloalkyle en C₄₋₆)alkyle ou un groupe (alkyle en C₄₋₈)cycloalkyle, qui peuvent éventuellement être substitués par un ou plusieurs substituants fluoro ou chloro,
c) un groupe alcényle en C₂₋₆ ou un groupe cycloalcényle en C₃₋₆,
d) un groupe halo
e) un groupe nitro
f) un groupe cyano
g) un groupe (alkyle en C₁₋₄)thio
Y est un groupe choisi parmi
a) une simple liaison C-C, un groupe CHR²⁰, un groupe CHR²⁰CH₂, un groupe OCHR²⁰, un groupe OCHR²⁰CH₂, un groupe SCHR²⁰, un groupe SCHR²⁰CH₂, un groupe NR²¹CHR²⁰, un groupe NR²¹CHR²⁰CH₂, un groupe CH₂CHR²⁰, un groupe CH₂CHR²⁰CH₂,
b) O, S, un groupe SO₂, un groupe NR²¹, un groupe CO, un groupe CONH, un groupe NHCO, un groupe CH₂O, un groupe CH₂S, un groupe CH₂NR²¹, à condition que lorsque Y représente (b), Ar¹ soit un groupe 1,4-phénylène de formule (E) et Ar² soit un groupe phényle de formule (I)
R²⁰ représente un atome d'hydrogène ou
a) un groupe COOH
b) un groupe COOP¹ dans lequel P¹ est un groupe de protection du groupe carboxy,
c) un groupe COO(alkyle en C₁₋₄)
d) un groupe 5-tétrazolyle
e) un groupe cyano
f) un groupe 5-tétrazolyle protégé de formule (M)
à condition qu'un des substituants R¹⁶ et R²⁰ soient un atome d'hydrogène et que l'autre soit un substituant autre que de l'hydrogène,
R²¹ et R²² sont choisis indépendamment parmi un atome d'hydrogène ou un groupe alkyle C₁₋₆ et
R²³ et R²⁴ sont choisis indépendamment parmi un atome d'hydrogène ou un groupe alkyle en C₁₋₄ ou peuvent former ensemble une chaîne polyméthylène contenant trois, quatre ou cinq atomes de carbone, qui peut éventuellement être interrompue par un atome d'oxygène
un « groupe phényle éventuellement substitué » étant un groupe phényle, qui n'est pas substitué ou qui peut de préférence être substitué par un ou plusieurs groupes halo, tels qu'un groupe fluoro, un groupe chloro, un groupe bromo, un groupe iodo, un groupe alkyle en C₁₋₄, un groupe alcoxy en C₁₋₄, un groupe (alkyle en C₁₋₄)thio, un groupe (alkyle en C₁₋₄)sulfinyle, un groupe (alkyle en C₁₋₄)sulfonyle, un groupe cyano, un groupe nitro, un groupe trifluorométhyle, ou un groupe hydroxy et qui comprend par exemple un groupe phényle, un groupe 2-méthylphényle, un groupe 3-méthylphényle, un groupe 4-méthylphényle, un groupe 2,4-diméthylphényle, un groupe 4-éthylphényle, un groupe 2-méthoxyphényle, un groupe 3-méthoxyphényle, un groupe 4-méthoxyphényle, un groupe 2,4-diméthoxyphényle, un groupe 2-chlorophényle, un groupe 3-chlorophényle, un groupe 4-chlorophényle, un groupe 2,4-dichlorophényle, un groupe 3,5-dichlorophényle, un groupe 4-fluorophényle, un groupe pentafluorophényle, un groupe 4-bromophényle, un groupe 4-méthylthiophényle, un groupe 4-trifluorométhylphényle, un groupe 4-cyanophényle, un groupe 4-nitrophényle, un groupe 4-hydroxyphényle,
ou un sel de celui-ci.

2. Composé selon la revendication 1, dans lequel R¹⁶ est un substituant acide ou un groupe COOP¹, et R²⁰ est un groupe COOH, un groupe 5-tétrazolyle ou un groupe COOP¹.

3. Composé selon la revendication 2, dans lequel R¹ est un atome d'hydrogène, un groupe alkyle en C₁₋₆ ou un groupe cycloalkyle en C₃₋₆.

4. Composé selon la revendication 3, dans lequel R¹ est un groupe Me ou un groupe Et.

5. Composé selon l'une quelconque des revendications 2 à 4, dans lequel R² est un atome d'hydrogène.

6. Composé selon l'une quelconque des revendications 2 à 5, dans lequel le groupe -X-(CHR³)ₙ- est un groupe -NHCH₂-, un groupe -NR⁴CH₂-, un groupe -NH- ou un groupe -SCH₂-.

7. Composé selon la revendication 5, dans lequel le groupe -X-(CHR³)ₙ- est un groupe -NR⁴CH₂-, et R⁴ est un atome d'hydrogène, un groupe Me ou un groupe CH₂COOH.

8. Composé selon l'une quelconque des revendications 2 à 7, dans lequel le groupe =A-B- forme avec le cycle pyrimidine une pyrazolo[1,5-a]pyrimidine, une imidazo[1,5-a]pyrimidine ou une imidazo[1,2-a]pyrimidine.

9. Composé selon l'une quelconque des revendications 2 à 8, dans lequel Ar¹ est un 1,4-phénylène.

10. Composé selon l'une quelconque des revendications 2 à 8, dans lequel Ar¹ est un 3-bromobenzofuranne de formule (H).

11. Composé selon l'une quelconque des revendications 2 à 10, dans lequel Y est une simple liaison C-C.

12. Composé selon l'une quelconque des revendications 2 à 11, dans lequel Ar² est un groupe phényle, un groupe pyrrol-1-yle ou un groupe 3-thiényle.

13. Formulation pharmaceutique comprenant un composé selon l'une quelconque des revendications 2 à 12, ou un sel pharmaceutiquement acceptable de celui-ci, avec un diluant ou un support pharmaceutiquement acceptable pour celui-ci.

14. Composé selon l'une quelconque des revendications 2 à 12 ou un sel pharmaceutiquement acceptable de celui-ci pour une utilisation comme produit pharmaceutique.
